# EUROPEAN PATENT SPECIFICATION

(11) **EP 0 862 560 B1**
(45) Date of publication and mention of the grant of the patent: **02.04.2003**
(21) Application number: 96939171.3
(22) Date of filing: 20.11.1996
(51) Int. Cl.: C07D 239/42, C07D 239/48, C07D 401/04, C07D 417/04, C07D 409/04, A61K 31/505

(54) **SUBSTITUTED 2-ANILINOPYRIMIDINES USEFUL AS PROTEIN KINASE INHIBITORS**
SUBSTITUIERTE 2-ANILINO-PYRIMIDINES VERWENDBAR ALS PROTEIN KINASE INHIBITOREN
2-ANILINOPYRIMIDINES SUBSTITUEES UTILES EN TANT QU'INHIBITEURS DE PROTEINE KINASE

(30) Priority: 20.11.1995 GB 9523675
(43) Date of publication of application: 09.09.1998
(73) Proprietor: CELLTECH THERAPEUTICS LIMITED, Slough, Berkshire SL1 3WE (GB)
(72) Inventor: DAVIS, Peter David, Aston Rowant, Oxford OX9 5SW (GB); MOFFAT, David Festus Charles, Maidenhead, Berkshire SL6 2YX (GB); DAVIS, Jeremy Martin, Wokingham, Berkshire RG40 1TA (GB); HUTCHINGS, Martin Clive, Wokingham, Berkshire RG40 4NH (GB)
(74) Representative: Hallybone, Huw George
(86) International application number: PCT/GB96/02854
(87) International publication number: WO 97/019065

(56) References cited:
- EP-A- 0 295 210
- FR-A- 2 545 356

## Description

This invention relates to substituted 2-anilinopyrimidines and to pharmaceutical compositions containing them.

Protein kinases participate in the signalling events which control the activation, growth and differentiation of cells in response to extracellular mediators and to changes in the environment. In general, these kinases fall into two groups; those which preferentially phosphorylate serine and/or threonine residues and those which preferentially phosphorylate tyrosine residues [Hanks, S K, Hunter T, FASEB. J. 9, 576-596 (1995)]. The serine/threonine kinases include for example, protein kinase C isoforms [Newton A C, J. Biol. Chem. 270, 28495-28498 (1995)] and a group of cyclin-dependent kinases such as cdc2 [Pines J, Trends in Biochemical Sciences 18, 195-197 (1995)]. The tyrosine kinases include membrane-spanning growth factor receptors such as the epidermal growth factor receptor [Iwashita S and Kobayashi M. Cellular Signalling 4, 123-132 (1992)], and cytosolic non-receptor kinases such as p56^{lck} p59^{fyn} ZAP-70 and csk kinases [Chan C *et al* Ann. Rev. Immunol. 12, 555-592 (1994)].

Inappropriately high protein kinase activity has been implicated in many diseases resulting from abnormal cellular function. This might arise either directly or indirectly, for example by failure of the proper control mechanisms for the kinase, related for example to mutation, overexpression or inappropriate activation of the enzyme; or by over- or underproduction of cytokines or growth factors also participating in the transduction of signal upstream or downstream of the kinase. In all of these instances, selective inhibition of the action of the kinase might be expected to have a beneficial effect.

We have now found a series of substituted 2-anilinopyrimidines which are potent and selective inhibitors of protein kinases, especially the kinases p56^{lck}, p59^{fyn}, ZAP-70 and protein kinase C. The compounds are thus of use in the prophylaxis and treatment of immune diseases, hyperproliferative disorders and other diseases in which inappropriate protein kinase action is believed to have a role.

Thus, according to one aspect of the invention, we provide a compound of formula (1): 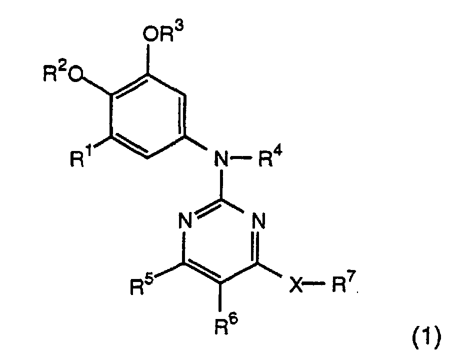 wherein R¹ R², R³, R⁴, R⁵, R⁶, X and R⁷ are as defined in claim 1, and the salts, solvates, hydrates and N-oxides thereof.

Halogen atoms represented by the group R⁶ in compounds of formula (1) include for example fluorine, chlorine, bromine or iodine atoms.

When R², R³, R⁵ and/or R^{6a} is an optionally substituted straight or branched chain alkyl, alkenyl or alkynyl group each of said groups may independently be an optionally substituted straight or branched chain C₁₋₆ alkyl, e.g. C₁₋₃ alkyl, C₂₋₆ alkenyl, e.g. C₂₋₄ alkenyl, or C₂₋₆ alkynyl, e.g. C₂₋₄ alkynyl group. Particular examples of such groups include optionally substituted -CH₃, -CH₂CH₃, -(CH₂)₂CH₃, -CH(CH₃)₂, -(CH₂)₃CH₃, -CH(CH₃)CH₂CH₃, -CH₂CH(CH₃)₂, -C(CH₃)₃, -(CH₂)₄CH₃, -(CH₂)₅CH₃, -CHCH₂, -CHCHCH₃, -CH₂CHCH₂, -CHCHCH₂CH₃, -CH₂CHCHCH₃, -(CH₂)₂CHCH₂, -CCH, -CCCH₃, -CH₂CCH, -CCCH₂CH₃, -CH₂CCCH₃, or -(CH₂)₂CCH groups. The optional substituents which may be present on these groups include one, two, three or more substituents selected from halogen atoms, e.g. fluorine, chlorine, bromine or iodine atoms, or hydroxyl, C₁₋₆ alkoxy, e.g. methoxy or ethoxy, thiol, C₁₋₆ alkylthio e.g. methylthio or ethylthio, amino, C₁₋₆ alkylamino, e.g. methylamino or ethylamino, or C₁₋₆ dialkylamino, e.g. dimethylamino or diethylamino groups.

R⁸ is an optionally substituted straight or branched C₁₋₆ alkyl, e.g. methyl or ethyl, C₂₋₆ alkenyl, e.g. allyl, or C₂₋₆ alkynyl, e.g. ethynyl group. The optional substituents which may be present on these groups include a phenyl group and/or one, two, three or more of the atoms or groups described above in relation to substituents present on alkyl groups.

When R⁶ is a substituted amino group it may be for example a -NHR⁹ or -NR⁹R¹⁰ group where R⁹ and R¹⁰, which may be the same or different, is each a group -R⁸ or -COR⁸ where R⁸ is as just defined.

Esterified carboxyl groups represented by the group R⁶ include groups of formula -CO₂Alk¹ wherein -CO₂Alk¹ is as defined hereinafter in connection with esterified carboxyl groups represented by the group R¹³.

Linker atoms represented by X or X¹ in compounds of formula (1) include -O- or -S- atoms. When X or X¹ is a linker group it may be for example a - C(O)-, -C(S)-, -S(O)-, -S(O)₂-_{,} -N(R¹¹)- [where R¹¹ is a hydrogen atom or a C₁₋₆ alkyl, e.g. methyl or ethyl, group], -CON(R¹¹)-, -OC(O)N(R¹¹)-, -CSN(R¹¹)-, -N(R¹¹)CO-, -N(R¹¹)C(O)O-, -N(R¹¹)CS-, -SON(R¹¹), -SO₂N(R¹¹), -N(R¹¹)SO₂-, -N(R¹¹)CON(R¹¹)-, -N(R¹¹)CSN(R¹¹)-, -N(R¹¹)SON(R¹¹)- or -N(R¹¹)SO₂N(R¹¹) group.

When R⁷ in compounds of formula (1) is an optionally substituted aliphatic or cycloaliphatic group it may be an optionally substituted C₁₋₁₀ aliphatic or C₃₋₁₀ cycloaliphatic group. Particular examples include optionally substituted straight or branched chain C₁₋₆ alkyl, C₂₋₆ alkenyl, or C₂₋₆ alkynyl groups or optionally substituted C₃₋₁₀cycloalkyl, C₃₋₁₀cycloalkenyl or C₃₋₁₀cycloalkynyl groups.

Heteroaliphatic or heterocycloaliphatic groups represented by R⁷ include the aliphatic or cycloaliphatic groups just described but with each group additionally containing one, two, three or four heteroatoms or heteroatom-containing groups. Particular heteroatoms or groups include atoms or groups -X²- where X² is as defined above for X when X is a linker atom or group.

Aromatic groups represented by R⁷ in compounds of formula (1) include for example optionally substituted monocyclic or bicyclic fused ring C₆₋₁₂ aromatic groups, such as optionally substituted phenyl, 1- or 2-naphthyl, 1-or 2-tetrahydronaphthyl, indanyl or indenyl groups.

Heteroaromatic groups represented by R⁷ include optionally substituted C₁₋₉ heteroaromatic groups containing for example one, two, three or four heteroatoms selected from oxygen, sulphur or nitrogen atoms. In general, the heteroaromatic groups may be for example monocyclic or bicyclic fused ring heteroaromatic groups. Monocyclic heteroaromatic groups include for example five- or six-membered heteroaromatic groups containing one, two, three or four heteroatoms selected from oxygen, sulphur or nitrogen atoms. Bicyclic heteroaromatic groups include for example nine- to thirteen-membered fused-ring heteroaromatic groups containing one, two or more heteroatoms selected from oxygen, sulphur or nitrogen atoms.

In general, when R⁷ is a heteroaliphatic, heterocycloaliphatic or heteroaromatic group it is attached to the remainder of the molecule of formula (1) through any available heteroatom or group or, preferably, carbon atom.

Particular examples of R⁷ aliphatic groups include those alkyl, alkenyl or alkynyl groups specifically described above in relation to the groups R², R³, R⁵ and R⁶. Each of these groups may be optionally substituted, and/or optionally interrupted by one or two heteroatoms or heteratom-containing groups represented by -X²- [where X² is as previously defined], to yield particular examples of R⁷ optionally substituted aliphatic or heteroaliphatic groups.

Particular examples of R⁷ cycloaliphatic and heterocycloaliphatic groups include optionally substitued cyclopropyl, cyclobutyl, cyclopentyl, cyclohexyl, cycloheptyl, 2-cyclobuten-1-yl, 2-cyclopenten-1-yl, 3-cyclopenten-1-yl, 2,4-cyclopentadien-1-yl, 3,5,-cyclohexadien-1-yl, pyrroline, e.g. 2- or 3-pyrrolinyl, pyrrolidinyl, dioxolanyl, e.g. 1,3-dioxolanyl, imidazolinyl, e.g. 2-imidazolinyl, imidazolidinyl, pyrazolinyl, e.g. 2-pyrazolinyl, pyrazolidinyl, pyranyl, e.g. 2- or 4-pyranyl, piperidinyl, 1,4-dioxanyl, morpholinyl, 1,4-dithianyl, thiomorpholinyl, piperazinyl, 1,3,5-trithianyl, oxazinyl, e.g. 2H-1,3-, 6H-1,3-, 6H-1,2-, 2H-1,2- or 4H-1,4-oxazinyl, 1,2,5-oxathiazinyl, isoxazinyl, e.g. o- or p-isoxazinyl, oxathiazinyl, e.g. 1,2,5 or 1,2,6-oxathiazinyl, or 1,3,5,2-oxadiazinyl groups.

Examples of heteroaromatic groups represented by R⁷ include optionally substituted pyrrolyl, furyl, thienyl, imidazolyl, N-methylimidazolyl, N-ethylimidazolyl, oxazolyl, isoxazolyl, thiazolyl, isothiazolyl, pyrazolyl, 1,2,3-triazolyl, 1,2,4-triazolyl, 1,2,3-oxadiazolyl, 1,2,4-oxadiazolyl, 1,2,5-oxadiazolyl, 1,3,4-oxadiazolyl, 1,3,4-thiadiazole, pyridyl, pyrimidinyl, pyridazinyl, pyrazinyl, 1,3,5-triazinyl, 1,2,4-triazinyl, 1,2,3-triazinyl, benzofuryl, [2,3-dihydro]benzofuryl, isobenzofuryl, benzothienyl, benzotriazolyl, isobenzothienyl, indolyl, isoindolyl, benzimidazolyl, imidazo[1,2-a]pyridyl, benzothiazolyl, benzoxazolyl, benzopyranyl, [3,4-dihydro]benzopyranyl, quinazolinyl, naphthyridinyl, pyrido[3,4-b]pyridyl, pyrido[3,2-b]pyridyl, pyrido[4,3-b]pyridyl, quinolinyl, isoquinolinyl, tetrazolyl, 5,6,7,8-tetrahydroquinolinyl, 5,6,7,8-tetrahydroisoquinolinyl, and imidyl, e.g. succinimidyl, phthalimidyl, or naphthalimidyl such as 1,8-naphthalimidyl.

Optional substituents which may be present on any of the above R⁷ groups in compounds of formula (1) include one, two, three or more substituents, each represented by the group R¹². The substituent R¹² may be selected from an atom or group R¹³ or -Alk(R¹³)ₘ, where R¹³ is a halogen atom, or an amino (-NH₂), -NHR¹⁴ [where R¹⁴ is an -Alk(R¹³)ₘ, heterocycloalkyl, -Alk-heterocycloalkyl, aryl or heteroaryl group], -N(R¹⁴)₂ [where each R¹⁴ group is the same or different], nitro, cyano, hydroxyl (-OH), -OR¹⁴, formyl, carboxyl (-CO₂H), esterified carboxyl, thiol (-SH), -SR¹⁴, -COR¹⁴, -CSR¹⁴, -SO₃H, -SO₂R¹⁴ -SO₂NH₂, -SO₂NHR¹⁴, SO₂N[R¹⁴]₂, -CONH₂, -CSNH₂, -CONHR¹⁴, -CSNHR¹⁴, -CON[R¹⁴]₂, -CSN[R¹⁴]₂, -NHSO₂H, -NHSO₂R¹⁴, -N[SO₂R¹⁴]₂, -NHSO₂NH₂, -NHSO₂NHR¹⁴, -NHSO₂N[R¹⁴]₂, -NHCOR¹⁴, -NHCONH₂, -NHCONHR¹⁴, -NHCON[R¹⁴]₂, -NHCSR¹⁴, -NHCSNH₂, -NHCSNHR¹⁴, -NHCSN[R¹⁴]₂, -NHC(O)OR¹⁴, or an optionally substituted cycloalkyl, aryl or heteroaryl group; or R¹² may be an optionally substituted heterocycloalkyl or -Alk-heterocycloalkyl group provided that when R¹² is a heterocycloalkyl group X in compounds of the invention is either a linker atom or group, or X is a bond and R⁷ is an optionally substituted aliphatic, cycloaliphatic, heteroaliphatic, heterocycloaliphatic, aromatic or heteroaromatic group other than a pyridyl group; Alk is a straight or branched C₁₋₆ alkylene, C₂₋₆ alkenylene or C₂₋₆ alkynylene chain, optionally interrupted by one, two or three -O- or -S- atoms or S-(O)-, -S(O)₂- or -N(R¹¹)- groups; and m is zero or an integer 1, 2 or 3.

When in the group -Alk(R¹³)ₘ m is an integer 1, 2 or 3, it is to be understood that the substituent or substituents R¹³ may be present on any suitable carbon atom in -Alk. Where more than one R¹³ substituent is present these may be the same or different and may be present on the same or different atom in -Alk or in R⁷ as appropriate. Thus for example, R⁷ may represent a -CH(R¹³)₂ group, such as a -CH(OH)Ar group where Ar is an aryl or heteroaryl group as defined below. Clearly, when m is zero and no substituent R¹³ is present the alkylene, alkenylene or alkynylene chain represented by Alk becomes an alkyl, alkenyl or alkynyl group.

When R¹³ is a halogen atom it may be for example a fluorine, chlorine, bromine, or iodine atom.

Esterified carboxyl groups represented by the group R¹³ include groups of formula -CO₂Alk¹ wherein Alk¹ is a straight or branched, optionally substituted C₁₋₈ alkyl group such as a methyl, ethyl, n-propyl, i-propyl, n-butyl, i-butyl, s-butyl or t-butyl group; a C₆₋₁₂arylC₁₋₈alkyl group such as an optionally substituted benzyl, phenylethyl, phenylpropyl, 1-naphthylmethyl or 2-naphthylmethyl group; a C₆₋₁₂aryl group such as an optionally substituted phenyl, 1-naphthyl or 2-naphthyl group; a C₆₋₁₂aryloxyC₁₋₈alkyl group such as an optionally substituted phenyloxymethyl, phenyloxyethyl, 1-naphthyloxymethyl, or 2-naphthyloxymethyl group; an optionally substituted C₁₋₈alkanoyloxyC₁₋₈alkyl group, such as a pivaloyloxymethyl, propionyloxyethyl or propionyloxypropyl group; or a C₆₋₁₂aroyloxyC₁₋₈alkyl group such as an optionally substituted benzoyloxyethyl or benzoyloxypropyl group. Optional substituents present on the Alk¹ group include R¹³ substituents described above.

When Alk is present in or as a substituent R¹² it may be for example a methylene, ethylene, n-propylene, i-propylene, n-butylene, i-butylene, s-butylene, t-butylene, ethenylene, 2-propenylene, 2-butenylene, 3-butenylene, ethynylene, 2-propynylene, 2-butynylene or 3-butynylene chain, optionally interrupted by one, two, or three -O- or -S-, atoms or -S(O)-, -S(O)₂- or -N(R¹¹)- groups.

Optionally substituted cycloalkyl groups represented by the group R¹³ include optionally substituted C₅₋₇ cycloalkyl groups such as optionally substituted cyclopentyl or cyclohexyl groups.

Heterocycloalkyl groups represented by the group R¹² or R¹⁴ include optionally substituted heteroC₃₋₆cycloalkyl groups containing one or two oxygen, sulphur or nitrogen atoms. Particular examples of such groups include optionally substituted azetidinyl pyrrolidinyl, piperidinyl, piperazinyl, homopiperazinyl, morpholinyl or thiomorpholinyl groups. The heterocycloalkyl group may be attached to the remainder of the molecule through any of its ring carbon atoms, or where present, ring nitrogen atom. Where the group R¹² is an -Alk-heterocycloalkyl group, Alk may be as defined above and the heterocycloalkyl portion may be as just defined, attached to Alk through any of its ring carbon atoms, or where present, ring nitrogen atom.

Optional subsituents which may be present on R¹², R¹³ or R¹⁴ cycloalkyl or heterocycloalkyl groups include one or two C₁₋₆ alkyl, e.g. methyl or ethyl, hydroxyl (-OH) hydroxyC₁₋₆alkyl, e.g. hydroxymethyl or hydroxyethyl, or C₁₋₆ alkoxy, e.g. methoxy or ethoxy groups. The substituent(s) may be present on any available ring carbon or nitrogen atom as appropriate.

Aryl and heteroaryl groups represented by the groups R¹³ or R¹⁴ include for example optionally substituted monocyclic or bicyclic C₆₋₁₂ aromatic groups, or C₁₋₉ heteroaromatic groups such as those described above in relation to the group R⁷.

Particularly useful atoms or groups represented by R¹² include fluorine, chlorine, bromine or iodine atoms, or C₁₋₆alkyl, C₁₋₆ alkylamino, C₁₋₆hydroxyalkyl, C₁₋₆alkylthiol, C₁₋₆alkoxy, hydroxyC₁₋₆alkoxy, aminoC₁₋₆alkoxy, C₁₋₆alkylaminoC₁₋₆alkoxy, C₁₋₆dialkylaminoC₁₋₆alkoxy, optionally substituted C₅₋₇cyclo-alkoxy, optionally substituted C₅₋₇cycloalkyl, optionally substituted C₅₋₇cycloalkylamino, haloC₁₋₆alkyl, haloC₁₋₆alkoxy, C₁₋₆alkylamino, amino (-NH₂), aminoC₁₋₆alkyl, C₁₋₆dialkylamino, hydroxyC₁₋₆ alkylamino, aminoC₁₋₆alkylamino, C₁₋₆alkylaminoC₁₋₆alkylamino, C₁₋₆dialkylaminoC₁₋₆alkylamino, C₁₋₆alkylaminoC₁₋₆ dialkylamino, C₁₋₆dialkylaminoC₁₋₆dialkylamino, nitro, cyano, hydroxyl (-OH), formyl [HC(O)-], carboxyl (-CO₂H), -CH₂CO₂H, -OCH₂CO₂H, -CO₂Alk¹ [where Alk¹ is as defined above], -CH₂CC₂Alk¹, C₁₋₆alkoxycarbonylC₁₋₆alkoxy, C₁₋₆ alkanoyl, optionally substituted phenyl C₁₋₆alkanoyl, thiol (-SH), thioC₁₋₆alkyl, -SC(NH)NH₂, sulphonyl (-SO₃H), C₁₋₆alkylsulphonyl, optionally substituted phenylsulphonyl, aminosulphonyl (-SO₂NH₂), C₁₋₆alkylaminosulphonyl, C₁₋₆dialkylaminosulphonyl, optionally substituted phenylamino-sulphonyl, carboxamido (-CONH₂), C₁₋₆alkylaminocarbonyl, C₁₋₆dialkylaminocarbonyl, optionally substituted phenylaminocarbonyl, aminocarbonylmethyl, C₁₋₆alkylaminocarbonylmethyl, optionally substituted benzylaminocarbonylmethyl, -NHC(S)NH₂, sulphonylamino (-NHSO₂H), C₁₋₆alkylsulphonylamino, C₁₋₆dialkylsulphonylamino, optionally substituted phenylsulphonylamino, aminosulphonylamino (-NHSO₂NH₂), C₁₋₆alkylaminosulphonylamino, C₁₋₆dialkylaminosulphonylamino, optionally substituted phenylaminosulphonylamino, aminocarbonylamino, C₁₋₆alkylaminocarbonylamino C₁₋₆dialkylaminocarbonylamino, phenylaminocarbonylamino, C₁₋₆alkanoylamino, aminoC₁₋₆ alkanoylamino, optionally substituted pyridylcarboxyamino, C₁₋₆alkanoylaminoC₁₋₆alkyl, C₁₋₆ alkoxycarbonylamino, optionally substituted heteroC₃₋₆cycloalkyl, piperidinyl, piperazinyl, 4-methyl-piperazinyl, homopipeprazinyl, or morpholinyl, optionally substituted heteroC₃₋₆cycloalkylC₁₋₆alkyl, piperidinylC₁₋₆alkyl, piperazinylC₁₋₆alkyl or morpholinylC₁₋₆alkyl, optionally substituted heteroC₃₋₆alkylC₁₋₆alkylamino, optionally substituted heteroC₃₋₆cycloalkylamino, tetrazolyl, optionally substituted phenylamino, optionally substituted benzylamino, optionally substituted benzyloxy, or optionally substituted pyridiylmethylamino group.

Where desired, two R¹² substituents may be linked together to form a cyclic group such as a cyclic ether, e.g. a C₁₋₆alkylenedioxy group such as a methylenedioxy or ethylenedioxy group.

Especially useful R¹² substituents include for example fluorine, chlorine, bromine or iodine atoms, or a methylamino, ethylamino, hydroxymethyl, hydroxyethyl, methylthiol, ethyithiol, methoxy, ethoxy, n-propoxy, 2-hydroxyethoxy, 3-hydroxypropoxy, 4-hydroxybutoxy, 2-aminoethoxy, 3-aminopropoxy, 2-(methylamino)ethoxy, 2-(dimethylamino)ethoxy, 3-(dimethylamino)propoxy, cyclopentyloxy, cyclohexyl, cyclohexylamino, 2-hydroxycyclohexylamino, trifluoromethyl, trifluoromethoxy, methylamino, ethylamino, amino (-NH)₂, aminomethyl, aminoethyl, dimethylamino, diethylamino, ethyl(methyl)amino, propyl(methyl)amino, 2-hydroxyethylamino, 3-hydroxypropylamino, 4-hydroxybutylamino, 2-aminoethylamino, 3-aminopropylamino, 4-aminobutylamino, 2-(methylamino)ethylamino, 2-(ethylamino)ethylamino, 2-(i-propylamino)ethylamino, 3-(i-propylamino)-propylamino, 2-(dimethylamino)ethylamino, 3-(dimethylamino)propylamino, 2-(diethylamino)ethylamino, 3-(diethyamino)propylamino, 2-(methyfamino)-ethyl(methyl)amino, 3-(methylamino)propyl(methyl)amino, 2-(dimethylamino)ethyl(methyl)amino, 2-(dimethylamino)ethyl(ethyl)amino, nitro, cyano, hydroxyl (-OH), formyl [HC(O)-], carboxyl (-CO₂H), -CH₂CO₂H, --OCH₂CO₂H, -CO₂CH₃, -CO₂CH₂CH₃, -CH₂CO₂CH₃, -CH₂CO₂CH₂CH₃, -CH₂CO₂CH₂phenyl, t-butoxycarbonylmethoxy, acetyl, phenacetyl, thio (-SH), thiomethyl, thioethyl, -SC(NH)NH₂, sulphonyl (-SO₂H), methylsulphonyl, methylaminosulphonyl, ethylaminosulphonyl, dimethylaminosulphonyl, diethylaminosulphonyl, carboxamido (-CONH₂), methylaminocarbonyl, ethylaminocarbonyl, dimethylaminocarbonyl, diethylaminocarbonyl, methylaminocarbonylmethyl, -NHC(S)NH₂, sulphonylamino (-NHSO₂H), methylsulphonylamino ethylsulphonylamino, dimethylsulphonylamino, diethylsulphonytamino, sulphonylamino (-HHSO₂NH₂), methylaminosulphonylamino, ethylaminosulphonylamino, dimethylaminosulphonylamino, diethylaminosulphonylamino, methylaminocarbonylamino, ethylaminocarbonylamino, dimethylaminocarbonylamino diethylaminocarbonylamino, acetylamino, aminomethylcarbonylamino, acetylaminomethyl, methoxycarbonylamino, ethoxycarbonylamino, t-butoxycarbonylamino, pyrrolidinyl, piperidinyl, piperazinyl, 4-methylpiperazinyl, homopipeprazinyl, morpholinyl, pyrrolidinylC₁₋₆alkyl, piperidinylC₁₋₆alkyl, piperazinylC₁₋₆alkyl, morpholinylC₁₋₆alkyl, 2-pyrrolidinylethylamino, 2-(1-methylpyrrolidinyl)ethylamino, 1-ethylpyrrolidinylmethylamino, piperidinylamino, 1-benzylpiperidinylamino, 4-(methoxy)phenylamino, 4-(3-hydroxypropyl)phenylamino, benzylamino, benzyloxy, pyridiylmethylamino group.

It will be appreciated that where two or more R¹² substituents are present, these need not necessarily be the same atoms and/or groups.

The presence of certain substituents in the compounds of formula (1) may enable salts of the compounds to be formed. Suitable salts include pharmaceutically acceptable salts, for example acid addition salts derived from inorganic or organic acids, and salts derived from inorganic and organic bases.

Acid addition salts include hydrochlorides, hydrobromides, hydroiodides, alkylsulphonates, e.g. methanesulphonates, ethanesulphonates, or isethionates, arylsulphonates, e.g. p-toluenesulphonates, besylates or napsylates, phosphates, sulphates, hydrogen sulphates, acetates, trifluoroacetates, propionates, citrates, maleates, fumarates, malonates, succinates, lactates, oxalates, tartrates and benzoates.

Salts derived from inorganic or organic bases include alkali metal salts such as sodium or potassium salts, alkaline earth metal salts such as magnesium or calcium salts, and organic amine salts such as morpholine, piperidine, dimethylamine or diethylamine salts.

Particularly useful salts of compounds according to the invention include pharmaceutically acceptable salts, especially acid addition pharmaceutically acceptable salts.

It will be appreciated that depending on the nature of the substituents R¹-R³ and R⁵ - R⁷ the compounds of formula (1) may exist as geometrical isomers and/or may have one or more chiral centres so that enantiomers or diasteromers may exist. It is to be understood that the invention extends to all such isomers of the compounds of formula (1), and to mixtures thereof, including racemates.

In one class of compounds of formula (1) the groups R¹, R², R³, R⁴, R⁵, R⁷ and X are as defined for formula (1), as is the group R⁶ except it is not an amino, substituted amino, nitro, carboxyl or esterified carboxyl group.

One particular class of compounds according to the invention has the formula (1) wherein R² and R³ is each an optionally substituted methyl or ethyl group. Particular examples of groups of these types include methyl, ethyl, halomethyl, e.g. -CH₂F, -CH₂Cl, -CHF₂, -CHCl₂, -CF₃, -CCl₃, or haloethyl groups. In general however, R² and R³ is each preferably a methyl group. In compounds of this class and in general in compounds of formula (1) R¹ may in particular be a methoxy group.

In a further preferred class of compounds of formula (1) R⁴ is preferably a hydrogen atom.

The groups R⁵ and R⁶ in compounds of formula (1) are each preferably a hydrogen atom.

In yet another preference, X in compounds of formula (1) is a direct bond, an oxygen or sulphur atom or a -N(R¹¹)- group. Especially useful compounds of this type are those whereinX is a direct bond, a sulphur atom or a -N(R¹¹)-, particularly a -NH-, group.

R⁷ in compounds of formula (1) is preferably an optionally substituted aromatic or heteroaromatic group.

A further class of compounds according to the invention has the formula 1(a): 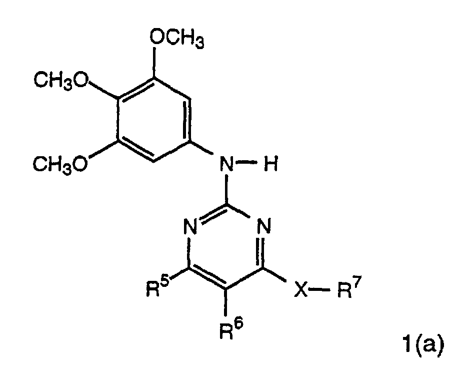 wherein R⁵ and R⁶ are as defined for formula (1), X is a direct bond, an oxygen or sulphur atom, or a group -N(R¹¹)- and R⁷ is an optionally substituted aromatic or heteroaromatic group, and the salts, solvates, hydrates and N-oxides thereof.

In these compounds, R⁵ is preferably a hydrogen atom. R⁶ is preferably a group -X¹R^{6a} where X¹ is as defined for formula (1) and R^{6a} is an optionally substituted straight or branched chain alkyl group, or R⁶ is especially a hydrogen atom.

X in compounds of formula (1a) is preferably a direct bond, a sulphur atom, or a -N(R¹¹)- group, particularly a -NH-group.

The aromatic or heteroaromatic R⁷ group in compounds of formulae (1) or 1(a) in general may be as defined previously for compounds of formula (1). In one preference, however, R⁷ is an optionally substituted phenyl, 1-or 2-naphthyl or heteroaromatic group containing one or two oxygen, sulphur and/or nitrogen atoms. Thus in particular R⁷ may be an optionally substituted phenyl, 1- or 2-naphthyl, pyrrolyl, furyl, thienyl, indolyl, pyrazolyl, thiazolyl, [2,3-dihydro]benzofuryl, benzothiazolyl, 2-pyridyl, 3-pyridyl or 4-pyridyl group. Particularly useful groups include optionally substituted phenyl, 2-pyridyl, 3-pyridyl or 4-pyridyl groups. The aromatic or heteroaromatic group may in particular be attached to the remainder of the compound of formula (1) through any available ring carbon atom.

In general, the optional substituents which may be present on aromatic or heteroaromatic R⁷ groups in compounds of formulae (1) or (1a) include one, two, or three R¹² substituents as generally and particularly described above and hereinafter in the Examples. Particularly useful R¹² substituents include -NHR¹⁴, -AlkNH₂, -AlkNHR¹⁴, -OR¹⁴, -AlkCO₂H or -AlkCO₂Alk¹ groups where R¹⁴, Alk and Alk¹ are as generally and particularly defined above. Useful members of these substituents include those wherein R¹⁴ is an -Alk, -AlkNH₂ or -Alk-heterocycloalkyl group. In t hese, and the other preferred substituents just mentioned, Alk and Alk¹ when present is each preferably a C₁₋₆alkyl group.

Particularly useful compounds according to the invention include:
N,N'-Bis(3,4,5-Trimethoxyphenyl)-2,4-pyrimidinediamine
4-(2-(2-Dimethylaminoethylamino)pyridin-5-yl)-N-(3,4,5-trimethoxyphenyl)-2-pyrimidineamine;
4-(2-(4-Hydroxybutylamino)pyridin-5-yl)-N-(3,4,5-trimethoxyphenyl)-2-pyrimidineamine;
4-(3-(3-Aminopropoxy)phenyl)-N-(3,4,5-trimethoxyphenyl)-2-pyrimidineamine;
N4-(3,4-Dimethoxyphenyl)-N2-(3,4,5-trimethoxyphenyl)-2,4-pyrimidinediamine;
4-(4-(2-Hydroxyethoxy)phenyl)-N-(3,4,5-trimethoxyphenyl)-2-pyrimidineamine;
4-(2-(3-(Morpholino)propylamino)pyridin-5-yl)-N-(3,4,5-trimethoxyphenyl)-2-pyrimidineamine;
4-(2-(2-(1-Methylpyrrolidin-2-yl)ethylamino)pyridin-5-yl)-N-(3,4,5-trimethoxyphenyl)-2-pyrimidineamine;
N4-(4-(Ethoxycarbonylmethyl)phenyl)-N2-(3,4,5-trimethoxyphenyl)-2,4-pyrimidinediamine;
N4-(4-Hydroxyphenyl)-N2-(3,4,5-trimethoxyphenyl)-2,4-pyrimidinediamine and
N4-(4-(3-Aminopropoxy)phenyl)-N2-(3,4,5-trimethoxyphenyl)-2,4-pyrimidinediamine;
and the salts, solvates, hydrates and N-oxides thereof

Compounds according to the invention are potent and selective inhibitors of protein kinases as demonstrated by differential inhibition of enzymes such as EGFr kinase, p56^{lck} kinase, ZAP-70 kinase, Csk kinase and p59^{fyn} kinase. The ability of the compounds to act in this way may be simply determined by employing tests such as those described in the Examples hereinafter.

The compounds according to the invention are thus of particular use in the prophylaxis and treatment of diseases in which inappropriate protein tyrosine kinase action plays a role, for example in autoimmune diseases such as rheumatoid arthritis, multiple sclerosis, and systemic lupus erythematosus, in transplant rejection, in graft v host disease, in hyperproliferative disorders such as tumours, psoriasis, in pannus formation in rheumatoid arthritis, restenosis following angioplasty and atherosclerosis, and in diseases in which cells receive pro-inflammatory signals such as asthma, inflammatory bowel disease and pancreatitis.

For the prophylaxis or treatment of disease the compounds according to the invention may be administered as pharmaceutical compositions, and according to a further aspect of the invention we provide a pharmaceutical composition which comprises a compound of formula (1) together with one or more pharmaceutically acceptable carriers, excipients or diluents.

Pharmaceutical compositions according to the invention may take a form suitable for oral, buccal, parenteral, nasal, topical or rectal administration, or a form suitable for administration by inhalation or insufflation.

For oral administration, the pharmaceutical compositions may take the form of, for example, tablets, lozenges or capsules prepared by conventional means with pharmaceutically acceptable excipients such as binding agents (e.g. pregelatinised maize starch, polyvinylpyrrolidone or hydroxypropyl methylcellulose); fillers (e.g. lactose, microcrystalline cellulose or calcium hydrogen phosphate); lubricants (e.g. magnesium stearate, talc or silica); disintegrants (e.g. potato starch or sodium glycollate); or wetting agents (e.g. sodium lauryl sulphate). The tablets may be coated by methods well known in the art. Liquid preparations for oral administration may take the form of, for example, solutions, syrups or suspensions, or they may be presented as a dry product for constitution with water or other suitable vehicle before use. Such liquid preparations may be prepared by conventional means with pharmaceutically acceptable additives such as suspending agents, emulsifying agents, non-aqueous vehicles and preservatives. The preparations may also contain buffer salts, flavouring, colouring and sweetening agents as appropriate.

Preparations for oral administration may be suitably formulated to give controlled release of the active compound.

For buccal administration the compositions may take the form of tablets or lozenges formulated in conventional manner.

The compounds for formula (1) may be formulated for parenteral administration by injection e.g. by bolus injection or infusion. Formulations for injection may be presented in unit dosage form, e.g. in glass ampoule or multi dose containers, e.g. glass vials. The compositions for injection may take such forms as suspensions, solutions or emulsions in oily or aqueous vehicles, and may contain formulatory agents such as suspending, stabilising, preserving and/or dispersing agents. Alternatively, the active ingredient may be in powder form for constitution with a suitable vehicle, e.g. sterile pyrogen-free water, before use.

In addition to the formulations described above, the compounds of formula (1) may also be formulated as a depot preparation. Such long acting formulations may be administered by implantation or by intramuscular injection.

For nasal administration or administration by inhalation, the compounds for use according to the present invention are conveniently delivered in the form of an aerosol spray presentation for pressurised packs or a nebuliser, with the use of suitable propellant, e.g. dichlorodifluoromethane, trichlorofluoromethane, dichlorotetrafluoroethane, carbon dioxide or other suitable gas or mixture of gases.

The compositions may, if desired, be presented in a pack or dispenser device which may contain one or more unit dosage forms containing the active ingredient. The pack or dispensing device may be accompanied by instructions for administration.

The quantity of a compound of the invention required for the prophylaxis or treatment of a particular condition will vary depending on the compound chosen, and the condition of the patient to be treated. In general, however, daily dosages may range from around 100ng/kg to 100mg/kg e.g. around 0.01 mg/kg to 40mg/kg body weight for oral or buccal administration, from around 10ng/kg to 50mg/kg body weight for parenteral administration and around 0.05mg to around 1000mg e.g. around 0.5mg to around 1000mg for nasal administration or administration by inhalation or insufflation.

The compounds of the invention may be prepared by a number of processes as generally described below and more specifically in the Examples hereinafter. In the following process description, the symbols R¹-R⁷ and X when used in the formulae depicted are to be understood to represent those groups described above in relation to formula (1) unless otherwise indicated. In the reactions described below, it may be necessary to protect reactive functional groups, for example hydroxy, amino, thio or carboxy groups, where these are desired in the final product, to avoid their unwanted participation in the reactions. Conventional protecting groups may be used in accordance with standard practice [see, for example, Green, T. W. in "Protective Groups in Organic Synthesis", John Wiley and Sons, 1981]. In some instances, deprotection may be the final step in the synthesis of a compound of formula (1) and the processes described hereinafter are to be understood to extend to such removal of protecting groups.

Thus a compound of formula (1) wherein X is a direct bond may be prepared by reaction of a guanidine of formula (2): 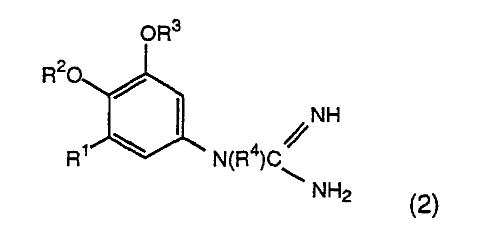 or a salt thereof
with an enaminone of formula (3):

R⁷COC(R⁶)C(R⁵)N(R¹⁵)(R¹⁶) (3)

where R¹⁵ and R¹⁶, which may be the same or different is each a C₁₋₆ alkyl group.

The reaction may be performed in a solvent, for example a protic solvent such as an alcohol, e.g. ethanol, methoxyethanol or propanol, optionally in the presence of a base e.g. an alkali metal base, such as sodium hydroxide or potassium carbonate, at an elevated temperature, e.g. the reflux temperature.

Salts of the compounds of formula (2) include acid salts such as inorganic acid salts e.g. hydrochlorides or nitrates.

intermediate guanidines of formula (2) may be prepared by reaction of the corresponding aniline of formula (4): 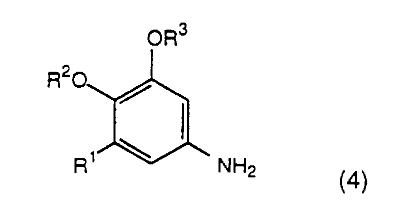 with cyanamide at an elevated temperature.

The reaction may be performed in a solvent such as ethanol at an elevated temperature, e.g. up to the reflux temperature. Where it is desired to obtain a salt of a guanidine of formula (2), the reaction may be performed in the presence of a concentrated acid, e.g. hydrochloric or nitric acid.

The anilines of formula (4) are either known compounds or may be obtained by conventional procedures, for example by hydrogenation of the corresponding nitro derivatives using for example hydrogen in the presence of a metal catalyst in a suitable solvent, for example as more particularly described in the interconversion reactions discussed below or by use of the corresponding nitro derivative and a reducing agent such as a sodium hydrosulphite in a solvent such as ethanol at an elevated temperature such as the reflux temperature. The nitrobenzenes for this particular reaction are either known compounds or may be prepared using similar methods to those used for the preparation of the known compounds, for example by treatment of the corresponding benzene with nitric acid in the prsence of an acid such as acetic acid at around ambient to the reflux temperature.

Intermediate enaminones of formula (3) may be prepared by reaction of an acetyl derivative R7COCH₂R⁶ with an acetal (R¹⁶)(R¹⁵)NCR⁵(OCH₃)₂ at an elevated temperature. The starting materials for this reaction are either known compounds of may be prepared by methods analogous to those used for the preparation of the known compounds.

In another process, a compound of formula (1) where X is a linker atom or group may be prepared by reaction of an intermediate of formula (5) 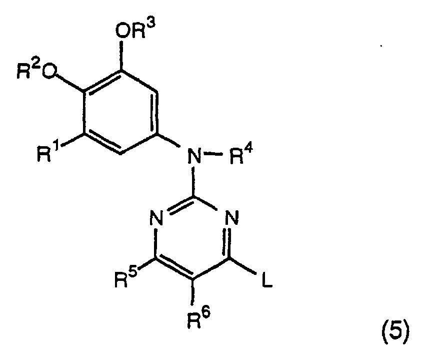 where L is a leaving atom or group, with a reagent R⁷X²H where X² is a linking atom or group as defined above.

Particular leaving atoms or groups represented by L include for example halogen atoms, e.g. bromine, iodine or chlorine atoms, and sulphonyloxy groups, e.g. alkylsulphonyloxy groups, such as trifluoromethylsulphonyloxy, and arylsulphonyloxy groups, such as p-toluenesulphonyloxy.

The reaction may be performed in the presence of a base, for example an organic base such as an organic amine, e.g. triethylamine or an inorganic base, for example a hydride such as sodium hydride, an alkoxide such as potassium t-butoxide, or a carbonate such as caesium or potassium carbonate, where necessary in the presence of a dipolar aprotic solvent such as an ether, e.g. a cyclic ether such as tetrahydrofuran, or an amide, e.g. a substituted amide such as dimethylformamide, at a suitable temperature e.g. from around room temperature to around 90°C.

Intermediates of formula (5) may be prepared by reaction of the corresponding pyrimidinones (where -N=C(L)- in formula (5) is -NH-C(O)-) with a halide using standard procedures, for example by reaction with a reagent PO(Hal)₃ (where Hal is a halogen atom), e.g. POCl₃, or RSO₂Hal (where R is an optionally substituted alkyl or aryl group) where necessary at an elevated temperature.

The starting pyrimidinones may be prepared by reaction of a guanidine of formula (2) or a salt thereof with a β-ketoester [for example a compound CH₃CH₂C (O)OCH(R⁶)C(O)R⁵ (where R⁵ is an optionally substituted alkyl, alkenyl or alkynyl group)] or a functional analogue thereof where it is desired to obtain compounds wherein R⁵ is a hydrogen atom, in the presence of a base, for example an alkoxide such as sodium methoxide in a solvent, for example a protic solvent, such as an alcohol, e.g. methanol at an elevated temperature, e.g. up to around the reflux temperature.

In a further process, a compound of formula (1) may be prepared by displacement of a leaving atom or group in a pyrimidine of formula (6): 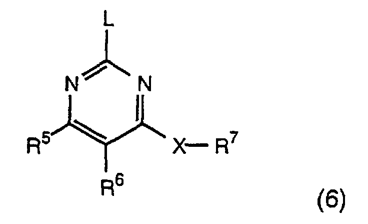 [where L is a leaving atom or group as defined above] with an aniline of formula (4).

The reaction may be performed at an elevated temperature, for example the reflux temperature, where necessary in the presence of a solvent, for example a ketone such as acetone, an alcohol such as ethanol or 2-ethoxyethanol or an aromatic hydrocarbon such as toluene, optionally in the presence of a base, for example an organic amine such as triethylamide or pyridine, or an acid, for example an inorganic acid such as hydrochloric acid.

Intermediate pyrimidines of formula (6) may be prepared by displacement of a leaving group from a pyrimidine of formula (7): 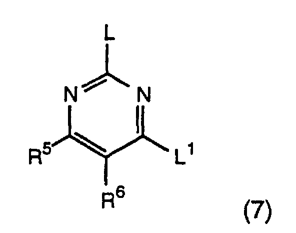 [where L¹ is a leaving atom or group as described above for the group L] using a nucleophilic reagent R⁷XH. The reaction may be performed as just described in relation to the preparation of compounds of formula (1) from the intermediate pyrimidines of formula (6).

The pyrimidines of formula (7) and the nucleophilic reagents R⁷XH are either known compounds or may be prepared using methods analogous to those used for the preparation of the known compounds.

In another process, a compound of formula (1) wherein X is a -C(O)- group may be prepared by treating a carboxamide of formula (8): 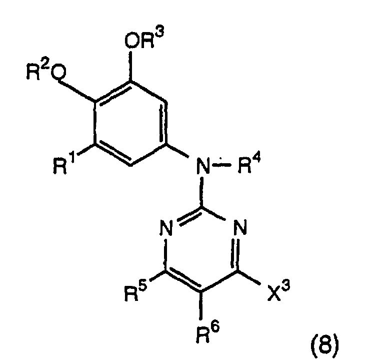 [where X³ is a carboxamide group] with a reagent R⁷MHal¹ (where M is a metal atom such as an zinc atom and Hal¹ is a halogen atom such as a bromine atom).

The group X³ in intermediates of formula (8) may be for example a group -CONR¹⁵R₁₆ or -CON(R¹⁵)(OR¹⁶).

The reaction may be performed in a solvent such as an ether, e.g. a cyclic ether such as a tetrahydrofuran, at a low temperature, e.g. around -78°C.

Intermediate carboxamides of formula (8) may be prepared by reaction of the corresponding acid of formula (9): 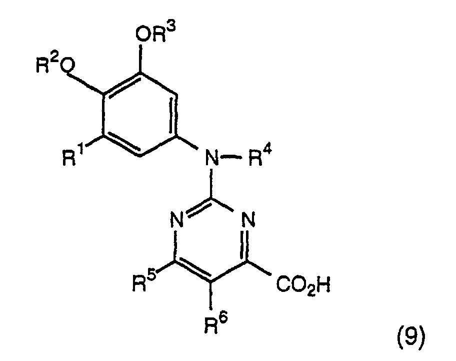 or a reactive derivative thereof with a reagent R¹⁵R¹⁶NH or R¹⁵R¹⁶ONH in the presence of a base e.g. an organic amine such as triethylamine in a solvent such as dichloromethane at a low temperature, e.g. around -20° to 0°C.

Intermediate acids of formula (9) may be prepared by heating the corresponding nitrile in the presnce of a base such as sodium hydroxide in a solvent such as ethanol. The nitrile starting material may be prepared by heating 2-chloro-4-cyano-pyrimidine with the appropriate aniline in the presence of a base such as triethylamine in a solvent such as ethanol at the reflux temperature.

Acids of formula (9) may also be used to generate compounds of formula (1) wherein X is a -NHC(O)O- group by reaction with an azide, for example diphenylphosphorylazide, and an alcohol R⁷OH in the presence of a base such as triethylamine at an elevated temperature, e.g. the reflux temperature.

Compounds of formula (1) may also be prepared by interconversion of other compounds of formula (1). Thus, for example, standard substitution approaches employing for example alkylation, arylation, acylation, thioacylation, sulphonylation, formylation or coupling reactions may be used to add new substitutents to and/or extend existing substituents in compounds of formula (1). Alternatively existing substituents in compounds of formula (1) may be modified by for example oxidation, reduction or cleavage reactions to yield other compounds of formula (1).

The following describes in general terms a number of approaches which can be employed to modify existing R¹, R², R³, R⁴, R⁵ and/or R⁶ groups in compounds of formula (1). It will be appreciated that each of these reactions may only be possible where an appropriate functional group exists in a compound of formula (1). Equally, any of the following reactions may be used to generate appropriately substituted intermediates of formulae (2), (4), (5), (6) and (7) for use in the preparation of compounds of formula (1).

Thus, for example alkylation or arylation of a compound of formula (1) may be achieved by reaction of the compound with a reagent R⁴L, AlkL or ArL, where R⁴, Alk, Ar and L are as previously defined.

The alkylation or arylation reaction may be carried out in the presence of a base, e.g. an inorganic base such as a carbonate, e.g. caesium or potassium carbonate, an alkoxide, e.g. potassium t-butoxide, or a hydride, e.g. sodium hydride, in a dipolar aprotic solvent such as an amide, e.g. a substituted amide such as dimethylformamide or an ether, e.g. a cyclic ether such as tetrahydrofuran, at around 0°C to around 40°C.

In a variation of this process the leaving group L may be alternatively part of the compound of formula (1) and the reaction performed with an appropriate nucleophilic reagent in a solvent such as an alcohol, e.g. ethanol, at an elevated temperature, e.g. the reflux temperature.

In another general example of an interconversion process, a compound of formula (1) may be acylated or thioacylated. The reaction may be performed for example with an acyl halide or anhydride in the presence of a base, such as a tertiary amine e.g. triethylamine in a solvent such as a halogenated hydrocarbon, e.g. dichloromethane or carbon tetrachloride, or an alcohol, e.g. methanol at for example ambient temperature, or by reaction with a thioester in an inert solvent such as tetrahydrofuran at a low temperature such as around 0°C. The reaction is particularly suitable for use with compounds of formula (1) containing primary or secondary amino groups.

In a further general example of an interconversion process, a compound of formula (1) may be formylated, for example by reaction of the compound with a mixed anhydride HCOOCOCH₃ or with a mixture of formic acid and acetic anhydride.

Compounds of formula (1) may be prepared in another general interconversion reaction by sulphonylation, for example by reaction of the compound with a reagent AlkS(O)₂L, or ArS(O)₂L in the presence of a base, for example an inorganic base such as sodium hydride in a solvent such as an amide, e.g. a substituted amide such as dimethylformamide at for example ambient temperature. The reaction may in particular be performed with compounds of formula (1) possessing a primary or secondary amino group.

In further examples of interconversion reactions compounds of formula (1) may be prepared from other compounds of formula (1) by modification of existing functional groups in the latter.

Thus in one example, ester groups -CO₂Alk¹ in compounds of formula (1) may be converted to the corresponding acid [-CO₂H] by acid- or base-catalysed hydrolysis or by catalytic hydrogenation depending on the nature of the group Alk¹. Acid- or base-catalysed hydrolysis may be achieved for example by treatment with an organic or inorganic acid, e.g. trifluoroacetic acid in an aqueous solvent or a mineral acid such as hydrochloric acid in a solvent such as dioxan or an alkali metal hydroxide, e.g. lithium hydroxide in an aqueous alcohol, e.g. aqueous methanol. Catalytic hydrogenation may be carried out using for example hydrogen in the presence of a metal catalyst, for example palladium on a support such as carbon in a solvent such as an ether, e.g. tetrahydrofuran or an alcohol, e.g. methanol. Similarly, base-catalysed hydrolysis with for example an alkali metal hydroxide such as sodium hydroxide in a solvent such as an alcohol e.g. ethanol may be used to convert a >NSO₂Alk or >NSO₂Ar group to a >N-H group.

In a second example, -OAlk² [where Alk² represents an alkyl group such as a methyl group] groups in compounds of formula (1) may be cleaved to the corresponding alcohol [-OH] by reaction with boron tribromide in a solvent such as a halogenated hydrocarbon, e.g. dichloromethane at a low temperature, e.g. around -78°C.

Alcohol [-OH] groups may also be obtained by hydrogenation of the corresponding -OCH₂Ar group using for example hydrogen in the presence of a metal catalyst, for example palladium on a support such as carbon in a solvent such as ethanol in the presence of ammonium formate. In another example, -OH groups may be generated from the corresponding ester [-CO₂Alk] by reduction using for example a complex metal hydride such as lithium aluminium hydride.

In a further example, alcohol -OH groups in compounds of formula (1) may be converted to a corresponding -OAlk or -OAr group by coupling with a reagent AlkOH or ArOH in a solvent such as tetrahydrofuran in the presence of a phosphine, e.g. triphenylphosphine and an activator such as diethyl-, diisopropyl-, or dimethylazodicarboxylate.

In another example of an interconversion reaction, amines of formula (1) may be alkylated using a reductive alkylation process employing an aldehyde and a borohydride, for example sodium triacetoxyborohydride, in a solvent such as dichloromethane, in the presence of an acid such as acetic acid at around ambient temperature.

Aminosulphonylamino [-NHSO₂NH₂] groups in compounds of formula (1) may be obtained, in another example, by reaction of a corresponding amine [-NH₂] with sulphamide in the presence of an organic base such as pyridine at an elevated temperature, e.g. the reflux temperature.

In a further example, amine [-NH₂] groups in compounds of formula (1) may be obtained by hydrolysis from a corresponding imide by reaction with hydrazine in a solvent such as an alcohol, e.g. ethanol at ambient temperature.

In another example, a nitro [-NO₂] group may be reduced to an amine [-NH₂], for example by catalytic hydrogenation as just described, or by chemical reduction using for example a metal, e.g. tin or iron, in the presence of an acid such as hydrochloric acid, optionally in a solvent such as an alcohol, e.g. methanol.

In a further example of an interconversion process, a tetrazole substituent may be obtained from the corresponding nitrile by treatment of the latter with an azide, e.g. sodium azide, in a solvent such as a substituted amine, e.g. dimethylformamide at an elevated temperature.

N-oxides of compounds of formula (1) may be prepared for example by oxidation of the corresponding nitrogen base using an oxidising agent such as hydrogen peroxide in the presence of an acid such as acetic acid, at an elevated temperature, for example around 70°C to 80°C, or alternatively by reaction with a peracid such as peracetic acid or 3-chloroperoxybenzoic acid in a solvent, e.g. dichloromethane, at ambient temperature.

Where salts of compounds of formula (1) are desired, these may be prepared by conventional means, for example by reaction of a compound of formula (1) with an appropriate acid or base in a suitable solvent or mixture of solvents, e.g. an organic solvent such as an ether, e.g. diethylether, or an alcohol, e.g. ethanol.

The following Examples illustrate the invention. In the Examples all ¹Hnmr were run at 300MHz unless specified otherwise. All temperatures are in °C. The following abbrevioations are used: DMSO - dimethylsulphoxide; DMF - dimethylformamide; THF - tetrahydrofuran.

### EXAMPLE 1

### 4-Phenyl-N-(3,4,5-trimethoxyphenyl)-2-pyrimidineamine

To a solution of 3,4,5-(trimethoxyphenyl)guanidine nitrate (1.89g, 6.57mmol) and 3-dimethylamino-1-phenyl-2-propen-1-one (1.15g, 6.57mmol) in propan-2-ol (20ml) was added powdered sodium hydroxide (289mg, 7.23mmol) and the mixture refluxed for 18h. On cooling (0°) the resulting precipitate was collected and washed with propan-2-ol and water, and then subjected to column chromatography [silica 1% methanol-CH₂CH₂] to afford the title compound (730mg) after recrystallisation from ethyl acetate as a yellow solid m.p. 146°. δ_{H} (CDCl₃) 3.85 (3H, s), 3.91 (6H, s), 7.07 (2H, s), 7.17 (1H, d, J 5.2Hz), 7.18 (1H, s, NH), 7.46-7.50 (3H, m), 8.10 (2H, m), and 8.46 (1H, d, J 5.2Hz).

The guanidine starting material was prepared by heating a mixture of 3,4,5-trimethoxyaniline (5.49g, 30.0mmol), cyanamide [Aldrich, 50% solution in water w/v] (3.50ml, 45.0mmol) and concentrated nitric acid (2.10ml, 300mmol) in ethanol (30ml). The solid which formed on cooling to room temperature was collected by filtration, washed with ethanol and dried *in vacuo* to give the desired product (4.60g) as a grey solid m.p. 187°. δ_{H} (d⁶DMSO) 3.65 (3H, s), 3.77 (6H, s), 6.54 (2H, s), 7.27 (4H, br s), and 9.46 (1H, s).

The following compounds of Examples 2-35 were prepared in a similar manner using the above guanidine starting material and the appropriate enaminone:

### EXAMPLE 2

### 4-(3-Pyridyl)-N-(3,4,5-trimethoxyphenyl)-2-pyrimidineamine

from 3,4,5-(trimethoxyphenyl)guanidine nitrate (1.33g, 4.60mmol), 3-dimethylamino-1-(3-pyridyl)-2-propen-1-one (812mg, 4.60mmol) and sodium hydroxide (203mg, 5.07mmol) to give the title compound (290mg) as a green solid m.p. 155°. δ_{H} (CDCl₃) 3.63 (3H, s), 3.78 (6H, s), 7.28 (2H, s), 7.47 (1H, d, J 5.1Hz), 7.56 (1H, m), 8.49 (1H, m), 8.58 (1H, d, J 5.1 Hz), 8.71 (1H, dd, J 4.8, 1.5Hz), 9.35 (1 H, d, J 2.0Hz) and 9.61 (1H, s).

### EXAMPLE 3

### 4-(4-Pyridyl)-N-(3,4,5-trimethoxyphenyl)-2-pyrimidineamine

from 3,4,5-(trimethoxyphenyl)guanidine nitrate (1.44g, 5.0mmol), 3-dimethylamino-1-(4-pyridyl)-2-propene-1-one (880mg, 5.0mmol) and sodium hydroxide (220mg, 5.5mmol) to give the title compound (765mg) as a green solid m.p. 205°. δ_{H} (d⁶DMSO) 3.63 (3H, s), 3.79 (6H, s), 7.27 (2H, s), 7.49 (1H, d, J 5.1Hz), 8.08 (2H, dd, J 4.5, 1.6Hz), 8.64 (1H, d, J 5.1Hz), 8.76 (2H, dd, J 4.5, 1.6Hz), and 9.66 (1H, br s).

### EXAMPLE 4

### 4-(2-Furyl)-N-3,4,5-(trimethoxyphenyl)-2-pyrimidineamine

from 3,4,5-(trimethoxyphenyl)guanidine nitrate (1.66g, 5.75mmol), 3-dimethylamino-1-(2-furyl)-2-propen-1-one) (950mg, 5.75mmol) and sodium hydroxide (253mg, 6.33 mmol) to give the title compound (350mg) as a yellow solid m.p. 139°. δ_{H} (CDCl₃) 3.83 (3H, s), 3.90 (6H, s), 6.56 (1H, m), 7.01 (2H, s), 7.06 (1H, d, J 5.1Hz), 7.18 (1H, m), 7.25 (1H, s), 7.58 (1 H, d, J 2.3 Hz), and 8.42 (1 H, d, J 5.1 Hz).

### EXAMPLE 5

### 4-(3,4,5-Trimethoxyphenyl)-N-(3,4,5-trimethoxyphenyl)-2-pyrimidineamine

from 3,4,5-(trimethoxyphenyl)guanidine nitrate (1.08g, 3.77mmol), 3-dimethylamino-1-(3,4,5-trimethoxyphenyl)-2-propen-1-one (1.0g, 3.77mmol) and sodium hydroxide (166mg) to give the title compound (67mg) as a colourless solid m.p. 187°. δ_{H} (CDCl₃) 3.83 (3H, s), 3.88 (6H, s), 3.92 (3H, s), 3.94 (6H, s), 6.99 (2H, s), 7.09 (1H, d, J 5.2Hz), 7.23 (1H, br s), 7.32 (2H, s) and 8.45 (1H, d, J 5.2Hz).

### EXAMPLE 6

### 4-(5-(2,3-Dihydrobenzofuryl))-N-(3,4,5-trimethoxyphenyl)-2-pyrimidineamine

from 3,4,5-(trimethoxyphenyl)guanidine nitrate (1.19g, 4.14mmol), 1-(5-(2,3-dihydrobenzofuryl)-3-dimethylamino-2-propen-1-one (900mg, 4.14mmol) and sodium hydroxide (182mg, 4.56mmol) to give the title compound (450mg) as a yellow solid m.p. 160°. δ_{H} (CDCl₃) 3.26 (2H, t, J 8.7Hz), 3.83 (3H, s), 3.90 (6H, s), 4.66 (2H, t, J 8,7Hz), 6.86 (1H, d, J 8.3Hz), 7.06 (3H, m), 7.17 (1H, s), 7.88 (1H, d, J 8.3Hz), 8.02 (1H, s), and 8.38 (1H, d, J 5.3Hz).

### EXAMPLE 7

### 4-(1-Phenylsulphonylindol-3-yl)-N-(3,4,5-trimethoxyphenyl)-2-pyrimidineamine

from 3,4,5-(trimethoxyphenyl)guanidine nitrate (820mg, 2.84mmol), 3-dimethylamino-1-(1-phenylsulphonylindol-3-yl)-2-propen-1-one (1.0g, 2.84mmol), and sodium hydroxide (125mg, 3.12mmol) to give the title compound (380mg) as a yellow solid m.p. 202°. δ_{H} (d⁶DMSO) 3.30 (3H, s), 3.63 (6H, s), 7.19 (2H, s), 7.33 (1H, t, J 7.3Hz), 7.44 (2H, m), 7.61 (2H, m), 7.71 (1H, m), 7.99 (1H, d, J 8.3Hz), 8.09 (2H, d, J 7.4Hz), 8.49 (1H, d, J 5.2Hz), 8.60 (1H, d, J 7.9Hz), 8.74 (1H, s) and 9.46 (1H, s).

### EXAMPLE 8

### 4-(2-Thiazolyl)-N-(3,4,5-trimethoxyphenyl)-2-pyrimidineamine

from 3,4,5-(trimethoxyphenyi)guanidine nitrate (1.27g, 4.40mmol), 3-dimethylamino-1-(2-thiazolyl)-2-propen-1-one (802mg, 4.40mmol), and sodium hydroxide (194mg, 4.84mmol) to give the title compound (520mg) as a green solid m.p. 159°, δ_{H} (d⁶DMSO) 3.63 (3H, s), 3.82 (6H, s), 7.24 (2H, s), 7.45 (1H, d, J 4.9Hz), 8.07 (1H, d, J 3.0Hz), 8.62 (1H, d, J 4.8Hz) and 9.70 (1H, s).

### EXAMPLE 9

### 4-(3-Thienyl)-N-(3,4,5-Trimethoxyphenyl)-2-pyrimidineamine

from 3,4,5-(trimethoxyphenyl)guanidine nitrate (1.27g, 4.41mmol), 3-dimethylamino-1-(3-thienyl)-2-propen-1-one (0.80g, 4.41mmol) to give the title compound (365mg) as a yellow solid m.p. 163°. δ_{H} (d⁶DMSO) 3.62 (3H, s), 3.78 (6H, s), 7.26-7.27 (3H, m), 7.68-7.71 (1H, m), 7.78 (1H, d, J 4.9Hz), 8.34-8.36 (1H, m), 8.47 (1 H, d, J 5.0Hz) and 9.44 (1H, s).

### EXAMPLE 10

### 4-(2-Naphthyl)-N-(3,4,5-trimethoxyphenyl)-2-pyrimidineamine

from 3,4,5-(trimethoxypheny)guanidine nitrate (1.02g, 3.55mmol), 3-dimethylamino-1-(2-naphthyl)-2-propen-1-one (800mg, 3.55mmol) and sodium hydroxide (156mg, 3.90mmol) to give the title compound (310mg) as a yellow solid m.p. 156°. δ_{H} (d⁶DMSO) 3.64 (3H, s), 3.82 (6H, s), 7.36 (2H, s), 7.53 (1H, d, J 6.2Hz), 7.58-7.61 (2H, m), 7.97-8.07 (3H, m), 8.29 (1H, d, J 8.5Hz), 8.58 (1H, d, J 5.1Hz), 8.76 (1H, s), and 9.58 (1H, br s).

### EXAMPLE 11

### 4-(3-Nitrophenyl)-N-(3,4,5-trimethoxyphenyl)-2-pyrimidineamine

from 3,4,5-(trimethoxyphenyl)guanidine nitrate (3.93g, 13.63mmol), 3-dimethylamino-1-(3-nitrophenyl)-2-propen-1-one (3.0g, 13.63mmol) and sodium hydroxide (545mg, 13.63mmol) to give the title compound (250mg) as a yellow solid. m.p. 184-185°. MS m/z 383 (M+H)⁺.

### EXAMPLE 12

### 4-(4-Nitrophenyl)-N-(3,4,5-trimethoxyphenyl)-2-pyrimidineamine

from 3,4,5-(trimethoxyphenyl)guanidine nitrate (1.3g, 4.5mmol), 3-dimethylamino-1-(4-nitrophenyl)-2-propen-1-one (1.0g, 4.5mmol) and sodium hydroxide (200mg) to give the title compound (550mg) as an orange solid m.p. 196°. MS m/z 383 (M+H)⁺.

### EXAMPLE 13

### 4-(3-Bromophenyl)-N-(3,4,5-trimethoxyphenyl)-2-pyrimidineamine

from 3,4,5-(trimethoxyphenyl)guanidine nitrate (1.63g, 5.66mmol), 1-(3-bromophenyl)-3-dimethylamino-2-propen-1-one (1.5g, 5.66mmol) and sodium hydroxide (250mg, 6.23mmol) to give the title compound (785mg) as a yellow solid m.p. 145°. MS m/z 418 (M+H)⁺.

### EXAMPLE 14

### 4-(Pyrrol-2-yl)-N-(3,4,5-trimethoxyphenyl)-2-pyrimidineamine

from 3,4,5-(trimethoxyphenyl)guanidine nitrate (1.15g, 4.0mmol), 3-dimethylamino-1-(pyrrol-2-yl)-2-propen-1-one (656g, 4.0mmol) and sodium hydroxide (176mg, 4.4mmol) to give the title compound (10mg) as a yellow solid m.p. 150°. MS m/z 327 (M+H)⁺.

### EXAMPLE 15

### 4-(3,4,-Methylenedioxyphenyl)-N-(3,4,5-trimethoxyphenyl)-2-pyrimidineamine

from 3,4,5-(trimethoxyphenyl)guanidine nitrate (1.15g, 4.0mmol), 3-dimethylamino-1-(3,4-methylenedioxyphenyl)-2-propen-1-one (8.76g, 4.0mmol) and sodium hydroxide (176mg) to give the title compound (160mg) as a yellow solid m.p. 180°. MS m/z 382 (M+H)⁺.

### EXAMPLE 16

### 4-(Pyrazin-2-yl)-N-(3,4,5-trimethoxyphenyl)-2-pyrimidineamine

from 3,4,5-(trimethoxyphenyl)guanidine nitrate (1.15g, 4.0mmol), 3-dimethylamino-1-(pyrazin-2-yl)-2-propen-1-one (708mg, 4.0mmol) and sodium hydroxide (176mg) to give the title compound (254mg) as a yellow solid. m.p. 181-182°. MS m/z 340 (M+H)⁺.

### EXAMPLE 17

### 4-(tert-Butyl)-N-(3,4,5-trimethoxyphenyl)-2-pyrimidineamine

from 3,4,5-(trimethoxyphenyl)guanidine nitrate (1.2g, 4.2mmol), 1-*tert*-butyl-3-dimethylamino-2-propen-1-one (650mg, 4.2mmol) and sodium hydroxide (185mg) to give the title compound (90mg) as a white solid m.p. 122°. MS m/z 318 (M+H)⁺.

### EXAMPLE 18

### 4-(1,2,3,4-Tetrahydronaphthalen-6-yl)-N-(3,4,5-trimethoxyphenyl)-2-pyrimidineamine

from 3,4,5-(trimethoxyphenyl)guanidine nitrate (1.0g, 435mmol), 3-dimethylamino-1-(1,2,3,4-tetrahydronaphthalen-6-yl)-2-propen-1-one (801mg, 3.5mmol) and sodium hydroxide (155mg, 3.85mmol) to give the title compound (120mg) as a yellow solid m.p. 150-151°. MS m/z 392 (M+H)⁺.

### EXAMPLE 19

### 4-(2,2-Dimethyl-3,4-dihydro-2H-benzo[b]oxin-6-yl)-N-(3,4,5-trimethoxyphenyl)-2-pyrimidineamine

from 3,4,5-(trimethoxyphenyl)guanidine nitrate (1.15g, 4.0mmol), 3-dimethylamino-1-(2,2-dimethyl-3,4-dihydro-2H-benzo[b]oxin-6-yl)-2-propen-1-one (1.04g, 4.0mmol) and sodium hydroxide (180mg, 4.4mmol) to give the title compound (120mg) as a pale yellow solid m.p. 149-150°. MS m/z 422.3 (M+H)⁺.

### EXAMPLE 20

### 4-(Benzothiazol-2-yl)-N-(3,4,5-trimethoxyphenyl)-2-pyrimidineamine

from 3,4,5-(trimethoxyphenyl)guanidine nitrate (1.72g, 6.0mmol), 1-(benzothiazol-2-yl)-3-dimethylamino-2-propen-1-one (1.39g, 6.0mmol) and sodium hydroxide (270mg, 6.6mmol) to give the title compound (420mg) as a yellow solid m.p. 182-183°. MS m/z 395 (M+H)⁺.

### EXAMPLE 21

### 4-(2-Nitrothien-5-yl)-N-(3,4,5-trimethoxyphenyl)-2-pyrimidineamine

from 3,4,5-(trimethoxyphenyl)guanidine nitrate (1.27g, 4.4mmol), 3-dimethylamino-1-(2-nitrothien-5-yl)-2-propen-1-one (1.0g, 4.4mmol) and sodium hydroxide (186mg, 4.9mmol) to give the title compound (540mg) as a dark red solid m.p. 182-183°. MS m/z 389 (M+H)⁺.

### EXAMPLE 22

### 4-(3-Methoxyphenyl)-N-(3,4,5-trimethoxyphenyl)-2-pyrimidineamine

from 3,4,5-(trimethoxyphenyl)guanidine nitrate (1.41g, 4,88mmol), 3-dimethylamino-1-(3-methoxyphenyl)-2-propen-1-one (1.0g, 4.88mmol) and sodium hydroxide (216mg, 5.4mmol) to give the title compound (275mg) as a yellow solid m.p. 155-156°. MS m/z 368 (M+H)⁺.

### EXAMPLE 23

### 4-(2-Pyridyl)-N-(3,4,5-trimethoxyphenyl)-2-pyrimidineamine

from 3,4,5-(trimethoxyphenyl)guanidine nitrate (1.99g, 6.91mmol), 3-dimethylamino-1-(2-pyridyl)-2-propen-1-one (1.22g, 6.91 mmol) and sodium hydroxide (276mg, 6.9mmol) to give the title compound (158mg) as a yellow solid m.p. 185°. MS m/z 339 (M+H)⁺.

### EXAMPLE 24

### 4-(4-tert-Butoxycarbonylaminophenyl)-N-(3,4,5-trimethoxyphenyl)-2-pyrimidineamine

from 3,4,5-(trimethoxyphenyl)guanidine nitrate (10.9g, 37.8mmol), 1-(4-*tert*-butoxycarbbonylaminophenyl)-3-dimethylamino-2-propen-1-one (10.0g, 34.4mmol) and sodium hydroxide (1.52g, 37.8mmol) to give the title compound (5.4g) as a yellow solid m.p. 158-159°. MS m/z 453 (M+H)⁺.

### EXAMPLE 25

### 4-(3-Hydroxyphenyl)-N-(3,4,5-trimethoxyphenyl)-2-pyrimidineamine

from 3,4,5-(trimethoxyphenyl)guanidine nitrate (4.72g, 16.4mmol), 1-(3-tert-butyldimethylsilyloxyphenyl)-3-dimethylamino-2-propen-1-one (5.0g, 16.4mmol) and sodium hydroxide (655mg, 16.4mmol) to give the title compound (2.0g) as a light yellow solid m.p. 191-192°. MS m/z 354 (M+H)⁺.

### EXAMPLE 26

### 4-(5-Thiazolyl)-N-(3,4,5-trimethoxyphenyl)-2-pyrimidineamine

from 3,4,5-(trimethoxyphenyl)guanidine nitrate (634mg, 2.2mmol), 3-dimethylamino-1-(5-thiazolyl)-2-propen-1-one (330g, 1.81mmol) and sodium hydroxide (88mg, 2.2mmol) to give the title compound (42mg) as a yellow solid m.p. 171°. MS m/z 345 (M+H)⁺.

### EXAMPLE 27

### 4-(4-Cyanophenyl)-N-(3,4,5-trimethoxyphenyl)-2-pyrimidineamine

from 3,4,5-(trimethoxyphenyl)guanidine nitrate (17.0g, 59.0mmol), 1-(4-cyanophenyl)-3-dimethylamino-2-propen-1-one (11.82g, 58.0mmol) and sodium hydroxide (2.36mg, 59.0mmol) to give the title compound (11.66g) as a green solid m.p. 183°, MS m/z 363 (M+H)⁺.

### EXAMPLE 28

### 4-(4-Ethoxycarbonylphenyl)-N-(3,4,5-trimethoxyphenyl)-2-pyrimidineamine

from 3,4,5-(trimethoxyphenyl)guanidine nitrate (11.66g, 40.5mmol), 3-dimethylamino-1-(4-ethoxycarbonylphenyl)-2-propen-1-one (10.0g, 40.5mmol) and sodium hydroxide (1.62g, 40.5mmol) to give the title compound (3.21g) as a yellow solid m.p. 181-182°. MS m/z 410 (M+H)⁺.

### EXAMPLE 29

### 4-(1-Naphthyl)-N-(3,4,5-trimethoxyphenyl)-2-pyrimidineamine

from 3,4,5-(trimethoxyphenyl)guanidine nitrate (2,94g, 10.22mmol), 3-dimethylamino-1-(1-naphthyl)-2-propen-1-one (2.30g, 10.22mmol) and sodium hydroxide (409mg, 10.22mmol) to give the title compound (750mg) as a yellow solid m.p. 130-133°. MS m/z 444 (M+H)⁺.

### EXAMPLE 30

### 4(4,5-Dimethylthiazol-2-yl)-N-(3,4,5-trimethoxyphenyl)-2-pyrimidineamine

from 3,4,5-(trimethoxyphenyl)guanidine nitrate (4.99g, 6.91 mmol), 3-dimethylamino-1-(4,5-dimethylthiazol-2-yl)-2-propen-1-one (3.64g, 17.3mmol) and sodium hydroxide (720mg, 18mmol) to give the title compound (490mg) as a yellow solid m.p. 207°. MS m/z 373 (M+H)⁺.

### EXAMPLE 31

### 4-(4-Methoxyphenyl)-N-(3,4,5-trimethoxyphenyl)-2-pyrimidineamine

from 3,4,5-(trimethoxyphenyl)guanidine nitrate (8.16g, 28.3mmol), 3-dimethylamino-1-(4-methoxyphenyl)-2-propen-1-one (5.5g, 28.3mmol) and sodium hydroxide (1.13g, 28.3mmol) to give the title compound (3.4g) as an orange solid m.p. 148-150°. MS m/z 368 (M+H).⁺

### EXAMPLE 32

### 4-(4-Benzyloxyphenyl)-N-(3,4,5-trimethoxyphenyl)-2-pyrimidineamine

from 3,4,5-(trimethoxyphenyl)guanidine nitrate (9.0g, 31.25mmol), 1-(4-benzyloxyphenyl)-3-dimethylamino-2-propen-1-one (8.88g, 31.25mmol) and sodium hydroxide (1.25g, 31.25mmol) to give the title compound (11.0g) as a yellow solid m.p. 171-172°. MS m/z 444(M+H)⁺.

### EXAMPLE 33

### 4-(5-(2-Hydroxyethyl)-4-methylthiazol-2-yl)-N-(3,4,5-trimethoxyphenyl)-2-pyrimidineamine

from 3,4,5-(trimethoxyphenyl)guanidine nitrate (1.22g, 4.23mmol), 1-(5-(2-*tert*-butyldimethylsilyloxyethyl)-4-methylthiazol-2-yl)-3-dimethylamino-2-propen-1-one (1.50g, 4.23mmol) and sodium hydroxide (200mg, 5.0mmol) to give the title compound (210mg) as an orange solid m.p. >190° (decomp). MS m/z 403 (M+H)⁺.

### EXAMPLE 34

### 4-(4-Bromophenyl)-N-(3,4,5-trimethoxyphenyl)-2-pyrimidineamine

from 3,4,5-(trimethoxyphenyl)guanidine nitrate (2.26g, 7.80mmol), 1-(4-bromophenyl)-3-dimethylamino-2-propen-1-one (2.0g, 7.80mmol) and sodium hydroxide (346mg, 8.60mmol) to give the title compound (1.0g) as a green solid m.p. 179°. MS m/z 416 (M+H)⁺.

### EXAMPLE 35

### 4-(2-Chloropyridin-5-yl)-N-(3,4,5-trimethoxyphenyl)-2-pyrimidineamine

from 3,4,5-(trimethoxyphenyl)guanidine nitrate (16.42g, 22.3mmol), 1-(2-chloropyridin-5-yl)-3-dimethylamino-2-propen-1-one (4.70g, 22.3mmol) and sodium hydroxide (895mg) to give the title compound (1.43g) as a yellow solid m.p. 191-192°. MS m/z 373.2 (M+H)⁺.

### EXAMPLE 36

### 4-(1-H-Indol-3-yl)-N-(3,4,5-trimethoxyphenyl)-2-pyrimidineamine

The compound of Example 7 (400mg, 0.78mmol) was suspended in ethanol (5ml) and 4M sodium hydroxide (5ml) and heated at reflux for 4h. On cooling a precipitate formed which was collected by filtration and washed with water to give the title compound (79mg) as a yellow solid m.p. 197°. MS m/z 377 (M+H)⁺.

### EXAMPLE 37

### N-Methyl-4-(4-pyridyl)-N-(3,4,5-trimethoxyphenyl)-2-pyrimidineamine

A solution of the compound of Example 3 (650mg, 1.92mmol) in DMF (5ml) was added dropwise to a stirred suspension of 60% NaH (76mg, 1.92mmol) in DMF (5ml), cooled to 0°C. After 0.5h iodomethane (0.13ml, 2.11 mmol) was added dropwise and the resulting mixture was left to warm to room temperature over 20h. The reaction was concentrated under reduced pressure and the resulting residue subjected to column chromatography to give the title compound (159mg) as a pale green solid m.p. 139°. MS m/z 353 (M+H)⁺.

### EXAMPLE 38

### 4-(2-Aminothien-5-yl)-N-(3,4,5-trimethoxyphenyl)-2-pyrimidineamine

A solution of the compound of Example 21 (380mg, 0.98mmol) was treated with ammonium formate (200mg, 6.34mmol) and 10% palladium on carbon (200mg) at 65° for 24h. On cooling, the catalyst was filtered off on a pad of celite and the filtrate concentrated under reduced pressure to give the title compound (100mg) as a green solid m.p. 161-162°. MS m/z 389 (M+H)⁺.

The compounds of Examples 39 and 40 were prepared in a similar manner to the compound of Example 38:

### EXAMPLE 39

### 4-(4-Aminophenyl)-N-(3,4,5-trimethoxyphenyl)-2-pyrimidineamine

from the compound of Example 12 (800mg, 2.09mmol), ammonium formate (660mg, 10.5mmol) and 10% palladium on carbon (80mg) to give the title compound (253mg) as a yellow solid m.p. 195°. MS m/z 353 (M+H)⁺.

### EXAMPLE 40

### 4-(3-Aminophenyl)-N-(3,4,5-trimethoxyphenyl)-2-pyrimidineamine

from the compound of Example 11 (150mg, 0.39mmol), ammonium formate (150mg, 2.38mmol) and 10% palladium on carbon (100mg) to give the title compound (120mg) as a yellow solid m.p. 166-167°. MS m/z 353 (M+H)⁺.

### EXAMPLE 41

### 4-(3-Acetamidophenyl)-N-(3,4,5-trimethoxyphenyl)-2-pyrimidineamine

To a solution of the compound of Example 40 (0.25g, 0,71 mmol) in dry CH₂Cl₂ (40ml) and triethylamine (0.11ml) was added acetyl chloride (0.06ml, 0.78mmol) and the resulting mixture stirred at room temperature for 4h. After washing with water (100ml) and saturated aqueous NaHCO₃ (100ml), the organic layer was dried (MgSO₄) and concentrated under reduced pressure. The residue was subjected to column chromatography [silica-ethyl acetate] to give after recrystallisation from ethyl acetate-hexane the title compound (163mg) as a yellow solid. m.p. 162-164°. MS m/z 395(M+H)⁺.

The compounds of Examples 42-44 were prepared in a similar manner to the compound of Example 41:

### EXAMPLE 42

### 4-(4-Acetamidophenyl)-N-(3,4,5-trimethoxyphenyl)-2-pyrimidineamine

from the compound of Example 39 (300mg, 0.85mmol), triethylamine (0.13ml, 0.94mmol) and acetyl chloride (0.07ml, 0.94mmol) to give the title compound (178mg) as a yellow solid m.p. 239°. MS m/z 395 (M+H)⁺.

### EXAMPLE 43

### N-(3-(2-(3,4,5-Trimethoxyphenylamino)pyrimidin-4-yl)phenyl)-5-methylisoxazole-3-carboxamide

from the compound of Example 40 (150mg, 0.43mmol), 5-methylisoxazole-3-carbonyl chloride (68mg, 0.47mmol) and triethylaminie (0.1ml, 0.7mmol) to give the title compound (7mg) as a yellow solid m.p. 173-175°. MS m/z 462 (M+H)⁺.

### EXAMPLE 44

### N-(3-(2-(3,4,5-Trimethoxyphenylamino)pyrimidin-4-yl)phenyl)pyridine-3-carboxamide

from the compound of Example 40 (250mg, 0.71 mmol), nicotinoyl chloride hydrochloride (139mg, 0.78mmol), and triethylamine (0.2ml, 2.3mmol) to give the title compound (155mg) as a yellow solid m.p. 140-143°. MS m/z 458 (M+H)⁺.

### EXAMPLE 45

### 4-(3-(3-Phthalimidopropoxy)phenyl)-N-(3,4,5-trimethoxyphenyl)-2-pyrimidineamine

To a suspension of caesium carbonate (462mg, 1.4mmol) in dry DMF (10ml) under a nitrogen atmosphere were added the compound of Example 25 (500mg, 1.4mmol) and 3-bromopropylphthalimide (380mg, 1.4mmol). The resulting mixture was stirred at room temperature for 4.5h, followed by the addition of water (50ml). The resulting precipitate was collected, washed with diethyl ether and recrystallised from ethyl acetate-hexane to give the title compound (620mg) as an off-white solid m.p. 209°. MS m/z 541 (M+H)⁺.

The following compound was prepared in a similar manner:

### EXAMPLE 46

### 4-(3-Propoxyphenyl)-N-(3,4,5-trimethoxyphenyl)-2-pyrimidineamine

from the compound of Example 25 (350mg, 0.99mmol), iodopropane (168mg, 0.99mmol) and caesium carbonate (322mg, 0.99mmol) to give the title compound (187mg) as an off-white solid m.p. 107°. MS m/z 396 (M+H)⁺.

### EXAMPLE 47

### 4-(3-(2-Hydroxyethoxy)phenyl)-N-(3,4,5-trimethoxyphenyl)-2-pyrimidineamine

To a suspension of 60% NaH (dispersion in oil) (62mg, 1.6mmol) in DMF (15ml) under a nitrogen atmosphere were added the compound of Example 25 (500mg, 1.4mmol) and ethylene carbonate (137mg, 1.6mmol). The resulting mixture was heated at 130° for 6h. On cooling the reaction was diluted with water (30ml), extracted with CH₂Cl₂ (3x25ml), dried (MgSO₄) and concentrated under reduced pressure. The resulting residue was subjected to column chromatography [silica-ethyl acetate] to give the title compound (260mg) as a yellow solid m.p. 70°. MS m/z 398 (M+H)⁺.

### EXAMPLE 48

### 4-(3-(3-Aminopropoxy)phenyl)-N-(3,4,5-trimethoxyphenyl)-2-pyrimidineamine dihydrochloride

The compound of Example 45 (500mg, 0.9mmol) was suspended in ethanol (30ml) containing hydrazine monohydrate (0.14ml, 2.8mmol) and the resulting mixture heated at reflux for 18h. On cooling the resulting precipitate was filtered off and the filtrate concentrated under reduced pressure. The residue was suspended in CH₂Cl₂ (30ml), extracted with 2N hydrochloric acid (2x30ml) Combined acid layers were taken to pH 11 with 6N NaOH and extracted with CH₂Cl₂ (3x30ml), the organic layers were dried (MgSO₄), and concentrated under reduced pressure. The residue was dissolved in ethanol (10ml) and the solution saturated with HCI (g). The resulting precipitate was collected to give the title compound (137mg) as an orange solid m.p. 236°. δH (d⁶-DMS)O 9.62 (1H, s), 8.54 (1H, d, J 5.2Hz), 8.05 (2H, br s), 7.75 (2H, m), 7.45 (2H, m), 7.29 (2H, s), 7.14 (1H, d, J 7.3Hz), 4.14 (2H, t, J 6.0Hz), 3.79 (6H, s), 3.62 (3H, s), 2.95 (2H, m) and 2.06 (2H, m). MS m/z 411 (M+H)⁺.

### EXAMPLE 49

### 4-(4-Hydroxyphenyl)-N-(3,4,5-trimethoxyphenyl)-2-pyrimidineamine

A solution of the compound of Example 32 (1.06g, 2.37mmol) in ethanol (100ml) was treated with ammonium formate (750mg, 12.0mmol) and 10% palladium on carbon (100mg) and stirred at room temperature for 18h, followed by heating at reflux for 18h. On cooling the catalyst was removed by filtration through Celite® and the filtrate concentrated under reduced pressure. The resulting residue was recrystallised from ethyl acetate to give the title compound (760mg) as a cream solid m.p. 200-202°. MS m/z 354 (M+H)⁺.

### EXAMPLE 50

### 4-(4-(3-Phthalimidopropoxy)phenyl)-N-(3,4,5-trimethoxyphenyl)-2-pyrimidineamine

To a supension of 60% NaH (dispersion in oil) (55mg, 1.36mmol) in DMF (10ml) was added the compound of Example 49 (480mg, 1.36mmol) and 3-bromopropylphthalimide (365mg, 1.36mmol). The resulting mixture was stirred at room temperature for 6h. After this time the reaction was concentrated under reduced pressure, the residue dissolved in ethyl acetate (50ml), washed with water (50ml), and dried (MgSO₄). The solvent was removed under reduced pressued to give the title compound (700mg) as a buff solid. MS m/z 541 (M+H)⁺.

### EXAMPLE 51

### 4-(4-(3-Aminopropoxy)phenyl)-N-(3,4,5-trimethoxyphenyl)-2-pyrimidineamine

The compound was prepared in a manner analogous to the preparation of the compound of Example 48 from the compound of Example 50 (830mg, 1.54mmol) and hydrazine monohydrate (0.23ml, 4.62mmol) to give the title compound (63mg) as a white solid m.p. 161-162°. MS m/z 411(M+H)⁺.

### EXAMPLE 52

### 4-(4-(2-Hydroxyethoxy)phenyl)-N-(3,4,5-trimethoxyphenyl)-2-pyrimidineamine

The compound was prepared in a manner analogous to the preparation of the compound of Example 47 from the compound of Example 49 (250mg, 0.71 mmol), ethylene carbonate (70mg, 6.78mmol) and 60% sodium hydride (dispersion in oil) (30mg, 0.78mmol) to give the title compound (140mg) as a yellow solid m.p 145-146°. MS m/z 398 (M+H)⁺.

### EXAMPLE 53

### 4-(4-(2-N,N-Dimethylaminoethoxy)phenyl)-N-(3,4,5-trimethoxyphenyl)-2-pyrimidineamine

The compound of Example 49 (250mg, 0.71mmol), N,N-dimethylaminoethanol (69mg, 0.78mmol) and triphenylphosphine (205mg, 0.78mmol) were dissolved in dry THF (20ml) under a nitrogen atmosphere and the mixture stirred for 0.5h. Diethylazodicarboxylate (136mg, 0.78mmol) in THF (10ml) was added dropwise and the reaction stirred for 48h, after which time the solvent was removed under reduced pressure. The residue was dissolved in ethyl acetate (40ml), washed with water (2x100ml), dried (MgSO₄) and the solvent evaporated. The resulting solid was subjected to column chromatography [silica-ethyl acetate] to give the title compound (75mg) as white crystals m.p. 140-147°. MS m/z 425 (M+H)⁺.

The following compound was prepared in a similar manner:

### EXAMPLE 54

### 4-(4-(3-N,N-Dimethylaminopropoxy)phenyl)-N-(3,4,5-trimethoxyphenyl)-2-pyrimidineamine

from the compound of Example 49 (250mg, 0.71mmol), N,N-dimethylaminopropanol (88mg, 0.85mmol), triphenylphosphine (223mg, 0.85mmol) and diethylazodicarboxylate (148mg, 0.85mmol) to give the title compound (20mg) as a white solid m.p. 161-164°. MS m/z 439 (M+H)⁺.

### EXAMPLE 55

### 4-(3-(3-Hydroxypropylamino)phenyl)-N-(3,4,5-trimethoxyphenyl)-2-pyrimidineamine

The compound of Example 40 (250mg, 0.71mmol) and 3-bromopropanol were heated at 85° in DMF for 72h. The solvent was removed under reduced pressure and the residue subjected to column chromatography [silica-ethyl acetate] to give the title compound (33mg) as a yellow solid m.p. 92-95°. MS m/z 411 (M+H)⁺.

The following compound was prepared in a similar manner:

### EXAMPLE 56

### 4-(4-(3-Hydroxypropylamino)phenyl)-N-(3,4,5-trimethoxyphenyl)-2-pyrimidineamine

from the compound of Example 39 (750mg, 2.13mmol) and 3-bromopropanol (3.55mg, 2.56mmol) to give the title compound (24mg) as a yellow solid m.p. 86-90°. MS m/z 411 (M+H)⁺.

### EXAMPLE 57

### 4-(3-(3-Pyridylmethyl)aminophenyl)-N-(3,4,5-trimethoxyphenyl)-2-pyrimidineamine

To a solution of the compound of Example 40 (250mg, 0.71mmol) in CH₂Cl₂ (20ml) was added 3-pyridinecarboxaldehyde (69mg, 0.65mmol), sodium triacetoxyborohydride (226mg, 1.07mmol) and acetic acid (0.1 ml), and the resulting mixture stirred at ambient temperature for 48h. The reaction was washed with water (2x100ml), dried (MgSO₄), concentrated under reduced pressure, and the residue columned [silica-ethyl acetate] to give the title compound (100mg) as a yellow solid m.p. 78-80°. MS m/z 444 (M+H)⁺.

The following compound was prepared in a similar manner:

### EXAMPLE 58

### 4-(4-(3-Pyridylmethyl)aminophenyl)-N-(3,4,5-trimethoxphenyl)-2-pyrimidineamine

from the compound of Example 39 (250mg, 0.71mmol), 3-pyridinecarboxaldehyde (69mg, 0.65mmol), sodium triacetoxyborohydride (226mg, 1.67mmol) and acetic acid (0.1ml) to give the title compound (134mg) as a yellow solid m.p. 189-190°. MS m/z 444 (M+H)⁺.

### EXAMPLE 59

### 4-(4-(Aminomethyl)phenyl)-N-(3,4,5-trimethoxyphenyl)-2-pyrimidineamine

To a suspension of LiAlH₄ (628mg, 16.56mmol) in dry THF (100ml) at 0° was added dropwise a solution of the compound of Example 27 in THF (10ml) and when addition was complete the reaction was heated at reflux for 3h. On cooling a saturated solution of aqueous ammonium chloride (25ml) was added and the organic solvent removed under reduced pressure. The aqueous solution was extracted with ethyl acetate (2x200ml) and the combined organic layers dried (MgSO₄) followed by evaporation under reduced pressure. The residue was subjected to column chromatography [silica-ethyl acetate] to give the title compound (330mg) as a yellow solid m.p. 143-144°. MS m/z 367 (M+H)⁺.

### EXAMPLE 60

### 4-(4-(1H-1,2,3,4-Tetrazol-5-yl)phenyl)-N-(3,4,5-trimethoxyphenyl)-2-pyrimidineamine

To a solution of the compound of Example 27 (500mg, 1.38mmol) in DMF (15ml) was added sodium azide (897mg, 13.8mmol) and ammonium chloride (738mg, 13.8mmol) and the mixture heated at 130° for 18h. On cooling, water (45ml) was added, the resulting precipitate collected and recrystallised from ethyl acetate to give the title compound (333mg) as a yellow solid m.p. 246-247°. δH 9.60 (1 H, br s), 8.59 (1 H, d, J 5.0Hz), 8.39 (2H, d, J 8.4), 8.19 (2H, d, J 8.4Hz), 7.47 (1H, d, J 5.3Hz), 7.31 (2H, s), 3.80 (6H, s) and 3.62 (3H, s). MS m/z 406.2 (M+H)⁺.

### EXAMPLE 61

### 4-(1-Oxopyrid-4-yl)-N-(3,4,5-trimethoxyphenyl)-2-pyrimidineamine

In a manner analogous to the preparation of the compound of Example 1, from 3,4,5-trimethoxyphenylguanidine nitrate (2.25g, 7.81 mmol), 3-dimethylamino-1-(1-oxo-pyrid-4-yl)-2-propen-1-one (880mg, 7.81mmol) and sodium hydroxide (344mg, 8.6mmol) to give the title compound (1.2g) as a green solid m.p. 220°. MS m/z 355 (M+H)⁺.
The 3-dimethylamino-1-(1-oxopyrid-4-yl)-2-propen-1-one used as starting material was prepared by heating a solution of 4-acetylpyridine-N-oxide (2.5g, 18.2mmol) in dimethylformamide diethylacetal (30ml) at reflux for 0.5h. On cooling the resulting solid was collected and washed with diethyl ether to give the desired product (3.18g) as an orange solid m.p. 181°.
The 4-acetylpyridine-N-oxide was prepared by treating a solution of 4-acetylpyridine (3.0g, 24.8mmol) in CH₂Cl₂ with 3-chloro-peroxybenzoic acid [Aldrich 57-86%] (8,4g) at room temperature for 12 h. The reaction was filtered, the filtrate concentrated under reduced pressure and the resulting residue subjected to column chromatography [silica 10% methanol-ethyl acetate] to give the desired product (3.2g) as a white solid m.p. 101°.

### EXAMPLE 62

### 4-(4-(Hydroxymethyl)phenyl)-N-(3,4,5-trimethoxyphenyl)-2-pyrimidmeamine

To a suspension of LiAlH₄ (560mg, 14.64mmol) in dry THF (50ml) at 0° was added dropwise to a solution of the compound of Example 28 in THF (100ml) and the reaction heated at reflux for 18h. On cooling an aqueous saturated solution of ammonium chloride (70ml) was added and the organic solvent removed under reduced pressure. The aqueous solution was extracted with CH₂Cl₂ (300ml), and this was dried (MgSO₄) and evaporated under reduced pressure. The residue was subjected to column chromatography [silica-ethyl acetate] to give title compound (212mg) as a pale yellow solid m.p. 171-172°. MS m/z 368 (M+H)⁺.

### EXAMPLE 63

### 4-(2-(3-Hydroxypropylamino)pyridin-5-yl)-N-(3,4,5-trimethoxyphenyl)-2-pyrimidineamine

The compound of Example 35 (200mg, 0.54mmol) and 3-amino-1-propanol (1.0ml, 13.05mmol) were heated at 100° for 3.5h. On cooling the reaction was concentrated under reduced pressure, water (15ml) added and the resulting precipitate collected. After washing with water, drying under vacuum and recrystallisaiton from ethanol the title compound (105mg) was obtained as a yellow solid m.p. 168-160°. δH(d⁶ DMSO) 9.40 (1H, s), 8.83 (1H, s), 8.38 (1H, d, J 5.3Hz), 8.12 (1H, br dm, J 7.0Hz), 7.26 (2H, s), 7.22 (1H, d, J 5.2Hz), 7.16 (1H, br t, J 3.0Hz), 6.54 (1H, d, J 8.8Hz), 4.53 (1H, t, J 5.0Hz), 3.76 (6H, s), 3.60 (3H, s), 3.47 (2H, q, J 5.4Hz), 3.32 (2H, m) and 1.68 (2H, m). MS m/z 412 (M+H)⁺.

The compounds of Examples 64-87 were prepared in a similar manner to the compound of Example 63 using the compound of Example 35 and the amine shown:

### EXAMPLE 64

### 4-(2-(2-Aminoethylamino)pyridin-5-yl)-N-(3,4,5-trimethoxyphenyl)-2-pyrimidineamine

from the compound of Example 35 (200mg, 0.54mmol) and ethylenediamine (1ml, 14.96mmol) to give the title compound (105mg) as a yellow solid m.p. 117-118° δH(d⁶ DMSO) 9.35 (1H, s), 8.83 (1H, d, J 2.2Hz), 8.38 (1H, d, J 5.3Hz), 8.13 (1H, dd, J 8.9, 2.4Hz), 7.27 (2H, s), 7.22 (1H, d, J 5.3Hz), 7.11 (1H, br t, J 5.0Hz), 3.77 (6H, s), 3.62(3H, s), 3.29 (2H, q, J 6.3Hz), 2.71 (2H, t, J 6.3Hz) and 1.60 (2H, br s). MS m/z 397 (M+H)⁺.

### EXAMPLE 65

### 4-(2-(2-Hydroxyethylamino)pyridin-5-yl)-N-(3,4,5-trimethoxyphenyl)-2-pyrimidineamine

from the compound of Example 35 (300mg, 0.81mmol) and ethanolamine (1.0ml, 16.56mmol) to give the title compound (190mg) as an off-white solid m.p. 123°. δH(d⁶ DMSO) 9.35 (1H, s), 8.82 (1H, d, J 2.2Hz), 8.38 (1H, d, J 5.3Hz), 8.12 (1H, dd, J 8.8, 2.4Hz), 7.26 (2H, s), 7.22 (1H, d, J 6,3Hz), 7.11 (1H, br t, J 5.0Hz), 6.59 (1H, d, J 8.8Hz), 4.73 (1 H, br s), 3.77 (6H, s), 3.62 (3H, s), 3.57-3.52 (2H, m) and 3.45-3.38 (2H, m). MS m/z 398 (M+H)⁺.

### EXAMPLE 66

### 4-(2-(3-Aminopropylamino)pyridin-5-yl)-N-(3,4,5-trimethoxyphenyl)-2-pyrimidineamine

from the compound of Example 35(250mg, 0.68mmol) and 1,3-diaminopropane (1.0ml, 11.89mmol) to give the title compound (120mg) as a yellow solid m.p. 152-153°. δH(d⁶ DMSO) 9.34 (1H, s), 8.83 (1H, d, J 2.2Hz), 8.37 (1H, d, J 5.3Hz), 8.12 (1H, dd J 8.8, 2.4Hz), 7.26 (2H, s), 7.21 (1H, d, J 5.3Hz), 7.14 (1H, bt, J 5.5Hz), 6.53 (1H, d, J 8.8Hz), 3.77 (6H, s), 3.62 (3H, s), 3.34 (2H, q, J 7.0Hz), 2.61 (2H, t, J 6.7 Hz), 2.40 (2H, br s) and 1.65-1.56 (2H, m). MS m/z 411 (M+H)⁺.

### EXAMPLE 67

### 4-(2-(2-Dimethylaminoethylamino)pyridin-5-yl)-N-(3,4,5-trimethoxyphenyl)-2-pyrimidineamine

from the compound of Example 35(250mg, 0.68mmol) and N,N-dimethylethylenediamine (0.75mol, 14.17mmol) to give the title compound (85mg) as an off-white solid m.p. 110-111°. MS m/z 425.5 (M+H)⁺.

### EXAMPLE 68

### 4-(2-(4-Hydroxybutylamino)pyridin-5-yl)-N-(3,4,5-trimethoxyphenyl)-2-pyrimidineamine

from the compound of Example 35(250mg, 0.68mmol) and 4-amino-1-butanol (0.750mg, 8.43mmol) to give the title compound (255mg) as a yellow solid m.p. 170-171°. MS m/z 4216 (M+H)⁺.

### EXAMPLE 69

### 4-(2-(2-(Pyrrolidin-1-yl)ethylamino)pyridin-5-yl)-N-(3,4,5-trimethoxyphenyl)-2-pyrimidineamine

from the compound of Example 35 (250mg, 0.68mmol) and N-(2-aminoethyl)pyrrolidine (0.75ml, 7.31mmol) to give the title compound (191mg) as a yellow solid m.p. 165-166°. MS m/z 451 (M+H)⁺.

### EXAMPLE 70

### 4-(2-(2-Methylamino)ethyl(methyl)amino)pyridin-5-yl)-N-(3,4,5-trimethoxyphenyl)-2-pyrimidineamine

from the compound of Example 35 (300mg, 0.81mmol) and N,N'-dimethylethylenediamine (0.61ml, 5.68mmol) to give the title compound (60mg) as a buff solid m.p. 115-116°. MS m/z 425 (M+H)⁺.

### EXAMPLE 71

### 4-(2-(3-Isopropylaminopropylamino)pyridin-5-yl)-N-(3,4,5-trimethoxyphenyl)-2-pyrimidineamine

from the compound of Example 35 (300mg, 0.81mmol) and N-isopropyl-1,3-propanediamine (0.51ml, 4.0mmol) to give the title compound (208mg) as a pale yellow solid m.p. 124-125°. MS m/z 453 (M+H)⁺.

### EXAMPLE 72

### 4-(2-(1-Benzylpiperid-4-ylamino)pyridin-5-yl)-N-(3,4,5-trimethoxyphenyl)-2-pyrimidineamine

from the compound of Example 35 (500mg, 1.35mmol) and 4-amino-1-benzylpiperidine (514mg, 2.7mmol) to give the title compound (370mg) as a yellow solid m.p. 113-114°. MS m/z 527 (M+H)⁺.

### EXAMPLE 73

### 4-(2-(3-(Morpholino)propylamino)pyridin-5-yl)-N-(3,4,5-trimethoxyphenyl)-2-pyrimidineamine

from the compound of Example 35(300mg, 0.81mmol) and 3-(aminopropyl)morpholine (0.59ml, 4mmol) to give the title compound (300mg) as a white solid m. p. 165-166°. MS m/z 481 (M+H)⁺.

### EXAMPLE 74

### 4-(2-(4-Methoxyphenylamino)pyridin-5-yl)-N-(3,4,5-trimethoxyphenyl)-2-pyrimidineamine

from the compound of Example 35 (300mg, 0.81mmol) and para-anisidine (200mg, 1.62mmol) to give the title compound (170mg) as a yellow solid m.p. 209-210°. MS m/z 459 (M+H)⁺.

### EXAMPLE 75

### 4-(2-(3-Methylaminopropyl(methyl)amino)pyridin-5-yl)-N-(3,4,5-trimethoxyphenyl)-2-pyrimidineamine

In a manner analogous to Example 63 from the compound of Example 35 (500mg) and N,N'-dimethyl-1,3-propanediamine (1.62ml, 13.0mmol) to give the title compound (31 mg) as a yellow solid. m.p. 103°. MS m/z 439 (M+H)⁺.

### EXAMPLE 76

### 4-(2-(2-Hydroxycyclohexylamino)pyridin-5-yl)-N-(3,4,5-trimethoxyphenyl)-2-pyrimidineamine

from the compound of Example 35 (300mg, 0.81mmol), trans-2-aminocyclohexanol hydrochloride (614mg, 4.05mmol) and triethylamine (0.56ml, 4.05mmol) to give the title compound (147mg) as a yellow solid m.p. 147-148°. MS m/z 452 (M+H)⁺.

### EXAMPLE 77

### 4-(2-(3-Dimethylamino-2,2-dimethylpropylamino)pyridin-5-yl)-N-(3,4,5-trimethoxyphenyl)-2-pyrimidineamine

from the compound of Example 35 (300mg, 0.81mmol) and N,N,2,2-tetramethyl-1,3-propanediamine (527mg, 4.05mmol) to give the title compound (147mg) as a white solid m.p. 125°. MS m/z 467 (M+H)⁺.

### EXAMPLE 78

### 4-(2-(3-Dimethylaminopropylamino)pyridin-5-yl)-N-(3,4,5-trimethoxyphenyl)-2-pyrimidine

from the compound of Example 35 (300mg, 0.81mmol) and 3-dimethylaminopropylamine (248mg, 2.43mmol) to give the title compound (201 mg) as a buff solid m.p. 170°. MS m/z 439 (M+H)⁺.

### EXAMPLE 79

### 4-(2-(2-Diethylaminoethyl(methyl)amino)pyridin-5-yl)-N-(3,4,5-trimethoxyphenyl)-2-pyrimidine

from the compound of Example 35 (300mg, 0.81mmol) and N,N-diethyl-N'-methylethylenediamine (316mg, 2.43mmol) to give the title compound (372mg) as a buff solid m.p. 111°. MS m/z 467 (M+H)⁺.

### EXAMPLE 80

### 4-(2-(3-Diethylaminopropylamino)pyridin-5-yl)-N-(3,4,5-trimethoxyphenyl)-2-pyrimidineamine

from the compound of Example 35 (300mg, 0.81 mmol) and N,N-diethylaminopropylamine (282mg, 2.43mmol) to give the title compound (146mg) as a yellow solid m.p. 67°. MS m/z 453 (M+H)⁺.

### EXAMPLE 81

### 4-(2-(2-(1-Methylpyrrolidin-2-yl)ethylamino)pyridin-5-yl)-N-(3,4,5-trimethoxyphenyl)-2-pyrimidineamine

from the compound of Example 35 (300mg, 0.81mmol) and 2-(2-aminoethyl)-1-methylpyrrolidine (512mg, 4.0mmol) to give the title compound (190mg) as a yellow solid m.p. 176-177°. MS M/z 465 (M+H)⁺.

### EXAMPLE 82

### 4-(2-(1-(Ethylpyrrolidin-2-yl)methylamino)pyridin-5-yl)-N-(3,4,5-trimethoxy-phenyl)-2-pyrimidineamine

from the compound of Example 35 (300mg), 0.81mmol) and 2-(aminomethyl)-1-ethylpyrrolidine (5.3mg, 4.0mmol) to give the title compound (240mg) as a yellow solid m.p. 81-82°. MS m/z 465 (M+H)⁺.

### EXAMPLE 83

### 4-(2-(2-Dimethylaminoethyl(ethyl)amino)pyridin-5-yl)-N-(3,4,5-trimethoxyphenyl)-2-pyrimidineamine.

from the compound of Example 35(300mg, 0.81 mmol) and N,N-dimethyl-N'-ethylethylenediamine (282mg, 2.43mmol) to give the title compound (193mg) as a yellow solid, m.p. 90°. MS m/z 453 (M+H)⁺.

### EXAMPLE 84

### 4-(2-(4-Aminobutylamino)pyridin-5-yl)-N-(3,4,5-trimethoxyphenyl)-2-pyrimidineamine

from the compound of Example 35 (300mg, 0.81 mmol) and 1,4-butane-diamine (705mg, 8.0mmol) as a yellow solid m.p. 209-201°. δH(d⁶ DMSO) 9.35 (1H, s), 8.82 (1H, d, J 2.3Hz), 8.37 (1H, d, J 5.3Hz), 8.11 (1H, dd, J 8.9, 2.3Hz), 7.26 (2H, s), 7.21 (1H, d, J 5.3Hz), 7.15 (1H, br t, NH), 6.53 (1H, d, J 8.9Hz), 3.77 (6H, s), 3.61 (3H, s), 3.28 (2H, q, J 6.0Hz), 2.58 (2H, t, J 6.9Hz), 1.58-1.53 (2H, m) and 1.97-1.39 (2H, m). MS m/z 425 (M+H)⁺.

### EXAMPLE 85

### 4-(2-(2-Diethylaminoethylamino)pyridin-5-yl)-N-(3,4,5-trimethoxyphenyl)-2-pyrimidineamine

from the compound of Example 35 (300mg, 0.81 mmol) and N,N-diethylethylenediamine (282mg, 2.43mmol) to give the title compound (50mg) as a yellow solid m.p. 67°. MS m/z 453 (M+H)⁺.

### EXAMPLE 86

### 4-(2-(3-(4-Methylpiperazin-1-yl)propylamino)pyridin-5-yl)-N-(3,4,5-trimethoxyphenyl)-2-pyrimidineamine

from the compound of Example 35 (350mg, 0.94mmol) and 1-(3-aminopropyl)-4-methylpiperazine (629mg, 4.0mmol) to give the title compound (238mg) as a white solid m.p. 183-184°. MS m/z 494 (M+H)⁺.

### Example 87

### 4-(2-(2-Dimethylaminoethyl(methyl)amino)pyridin-5-yl)-N-(3,4,5-trimethoxyphenyl)pyrimidine-2-amine

from the compound of Example 35 (30mg, 0.81mmol) and N,N,N'-trimethylethylenediamine (400mg, 4.0mmol) to give the title compound (310g) as a yellow solid m.p. 97-98°, MS m/z 439 (M+H)⁺.

### EXAMPLE 88

### 4-(2-(Piperid-4-ylamino)pyridin-5-yl)-N-(3,4,5-trimethoxyphenyl)-2-pyrimidineamine acetate

To a solution of the compound of Example 72 (90mg, 0.15mmol) as the acetate salt in ethanol (10ml) was added cyclohexadiene (0.095ml, 1.0mmol) and 10% palladium on charcoal (100mg) and the reaction heated at reflux for 1h. On cooling the reaction was filtered through a pad of Celite® and the filtrate concentrated under reduced pressure to give the title compound (20mg) as a yellow solid m.p.87-88°. MS m/z 437 (M+H)⁺.

### EXAMPLE 89

### N4-Phenyl-N2-(3,4,5-trimethoxyphenyl)-2,4-pyrimidinediamine

A solution of N-phenyl-2-chloro-4-pyrimidineamine (1.83g, 8.91mmol) and 3,4,5-trimethoxyaniline (1.83g, 10.0mmol) in acetone (10ml) and water (15ml) containing concentrated hydrochloric acid (0.2ml) was heated at reflux for 10h. On cooling the acetone was removed under reduced pressure, and the reaction taken to pH10 with 4N NaOH and extracted with ethyl acetate (2x75ml). The combined organic layers were dried (MgSO₄), concentrated under reduced pressure and the residue crystallised from ethyl acetate to give the title compound (2.41g) as a colourless solid m.p. 191°. δH (CDCl₃) 8.04 (1H, d, J 5.8Hz), 7.37-7.34 (4H, m), 7.16-7.10 (1H, m), 7.05 (1H, br s), 6.85 (2H, s), 6.68 (1H, br s), 6.16 (1H, d, J 5.8Hz), 3.82 (3H, s) and 3.81 (6H, s). MS m/z 353 (M+H)⁺. The pyrimidineamine used as starting material was prepared by heating a solution of 2,4-dichloropyrimidine (4.0g, 26.8mmol), aniline (2.46ml, 27.0mmol) and triethylamine (4.3ml, 30.0mmol) in ethanol (50ml) at reflux for 2h. On cooling a white precipitate was collectd which was washed with cold ethanol (100ml) and recrystallised from ethyl acetate to give the desired product (6.01g) as a colourless solid m.p. 186°. MS/mz 206 (M+H)⁺.

The compounds of Examples 90-110 were prepared in a similar manner to the compound of Example 89 using the starting material shown. In each case the pyrimidineamine starting material was prepared from the available compounds shown in a similar manner to the pyrimidineamine starting material of Example 89:

### EXAMPLE 90

### N4-(4-(2-Hydroxyethoxy)phenyl)-N2-(3,4,5-trimethoxyphenyl)2,4-pyrimidineamine

from 2-chloro-N-(4-(2-hydroxyethoxy)phenyl)-4-pyrimidineamine (187mg, 0.70mmol) and 3,4,5-trimethoxyaniline (146mg, 0.8mmol) to give the title compound (18mg) as a colourless solid m.p. 212°. δH (d⁶ DMSO) 9.09 (1H, br s), 8.89 (1H, br s), 7.94 (1H, d, J 5.8Hz), 7.53 (2H, d, J 8.6Hz), 7.10 (2H, s), 6.86 (2H, d, J 8.6Hz), 6.10 (1H, d, J 5.8Hz), 4.83 (1H, t, J 5.6Hz),. 3.95 (2H, t, J 4.9Hz), 3.71-3.68 (2H, m), 3.65 (6H, s) and 3.60 (3H,s). MS m/z 413 (M+H)⁺.
The pyrimidineamine starting material was prepared from 4-(2-hydroxyethoxy)aniline (481mg, 3.14mmol) 2,4-dichloropyrimidine (468mg, 3.14mmol) and triethylamine (0.46ml, 3.25mmol) to give the desired product as a yellow solid m.p. 169°. MS m/z 266(M+H)⁺.

### EXAMPLE 91

### N4-(3,4-Methylenedioxyphenyl)-N2-(3,4,5-trimethoxyphenyl)-2,4-pyrimidinediamine

from 2-chloro-N-(3,4-methylenedioxyphenyl)-4-pyrimidineamine (200mg, 0.85mmol) and 3,4,5-trimethoxyaniline (156mg, 0.85mmol) to give the title compound (187mg) as a buff solid m.p. 199°. MS m/z 397 (M+H)⁺.
The pyrimidineamine starting material was prepared from 3,4-methylenedioxyaniline (219mg, 1.60mmol) 2,4-dichloropyrimidine (250mg, 1.60mmol) and triethylamine (0.25ml, 1.80mmol) to give the desired product (215mg) as a buff solid m.p. 148°. MS m/z 250 (M+H)⁺.

### EXAMPLE 92

### N4-(3-Methoxyphenyl)-N2-(3,4,5-trimethoxyphenyl)-2,4-pyrimidinediamine

from 2-chloro-N4-(3-methoxyphenyl)-4-pyrimidineamine (195mg, 0.83mmol) and 3,4,5-trimethoxyaniline (152mg, 0.83mmol) to give the title compound (189mg) as a white solid m.p. 78°. MS m/z 383 (M+H)⁺.
The pyrimidineamine starting material was prepared from meta-anisidine (0.18mg, 1.6mmol), 2,4-dichloropyrimidine (250mg, 1.6mmol) and triethylamine (0.25mg, 1.8mmol) to give the desired product (200mg) as a white solid m.p. 107°. MS m/z 236 (M+H)⁺.

### EXAMPLE 93

### N4-(3-(2-Hydroxyethyl)phenyl)-N2-(3,4,5-trimethoxyphenyl)-2,4-pyrimidinediamine

from 2-chloro-N-(4-(2-hydroxyethyl)phenyl)-4-pyrimidineamine (250mg, 1.0mmol) and 3,4,5-trimethoxyaniline (184mg, 1.0mmol) to give the title compound (257mg) as a white solid m.p. 72°. MS m/z 397 (M+H)⁺.
The pyrimidineamine starting material was prepared from 2-(4-aminophenyl)ethanol (219mg, 1.6mmol) 2,4-dichloropyrimidine (250mg, 1.6mmol) and triethylamine (0.25ml, 1.8mmol) to give the desired product (289mg) as an orange solid m.p. 163°.

### EXAMPLE 94

### N4-(4-(Diethylamino)phenyl)-N2-(3,4,5-trimethoxyphenyl)-2,4-pyrimidinediamine

from 2-chloro-N-(4-(diethylamino)phenyl)-4-pyrimidineamine (250mg, 0.9mmol) and 3,4,5-trimethoxyaniline (166mg, 0.9mmol) to give the title compound (80mg) as a green solid m.p. 87°, MS m/z 424.5 (M+H)⁺.
The pyrimidineamine starting material was prepared from N,N-diethyl-1,4-phenyldiamine (263mg, 1.6mmol), 2,4-dichloropyrimidine (250mg, 1.6mmol) and triethylamine (0.25ml, 1.8mmol) to give the desired prdocut (410mg) as a green solid m.p. 212°. MS m/z 277 (M+H).⁺

### EXAMPLE 95

### N4-(4-(3-Hydroxypropoxy)phenyl)-N2-(3,4,5-trimethoxyphenyl)-2,4-pyrimidinediamine

from 2-chloro-N-(4-(3-hydroxypropoxy)phenyl)-4-pyrimidineamine (250mg, 0.89mmol) and trimethoxyaniline (164mg, 0.89mmol) to give the title compound (61 mg) as a white solid m.p. 69°. MS m/z 427 (M+H)⁺.
The pyrimidineamine starting material was prepared (from 4-(3-hydroxypropoxy)aniline (267mg, 1.60mmol), 2,4-dichloropyrimidine (250mg, 1.60mmol) and triethylamine (0.25ml, 1.80mmol) to give the desired product (383mg) as a pale orange solid m.p. 181°. MS m/z 280 (M+H)⁺.

### EXAMPLE 96

### N4-(3,4-Dimethoxyphenyl)-N2-(3,4,5-trimethoxyphenyl)-2,4-pyrimidinediamine

from 2-chloro-N-(3,4-dimethoxyphenyl)-4-pyrimidineamine (250mg) and 3,4,5-trimethoxyaniline (189mg, 1.0mmol) to give the title compound (160mg) as a light pink solid m.p. 86°. MS m/z 413 (M+H)⁺.
The pyrimidineamine starting material was prepared from aminoveratrole (257mg, 1.60mmol), 2,4-dichloropyrimidine (2.50mg, 1.60mmol) and triethylamine (0.25ml, 1.80mmol) to give the desired product (357mg) as a purple solid m.p. 104° MS m/z 266 (M+H)⁺.

### EXAMPLE 97

### N4-(4-(4-Methylpiperazin-1-yl)phenyl)-N2-(3,4,5-trimethoxyphenyl)-2,4-pyrimidinediamine

from 2-chloro-N-(4-(4-methylpiperazin-1-yl)phenyl)-4-pyrimidineamine (110mg, 0.40mmol) and 3,4,5-trimethoxyaniline (66mg, 0.40mmol) to give the title compound (80mg) as a white solid m.p. 104°. MS m/z 451 (M+H)⁺.
The pyrimidineamine starting material was prepared from 4-(4-methylpiperazin-1-yl)aniline (511mg, 2.67mmol), 2,4-dichloropyrimidine (398mg, 2.67 mmol) and triethylamine (10.4ml, 2.94mmol) to give the desired product (110mg) as a white solid m.p. 122°.

### EXAMPLE 98

### N4-(3,5-Dimethoxyphenyl)-N2-(3,4,5-trimethoxyphenyl)-2,4-pyrimidinediamine

from 2-chloro-N-(3,5-dimethoxyphenyl)-4-pyrimidineamine (150mg, 0.56mmol) and 3,4,5-trimethoxyaniline (104mg, 0.56mmol) to give the title compound (162mg) as a white solid m.p. 180°. MS m/z 413 (M+H)⁺.
The pyrimidineamine starting material was prepared from 3,5-dimethoxyaniline (257mg, 1.60mmol), 2,4-dichloropyrimidine (250mg, 1.60mmol) and triethylamine (0.25ml, 1.80mmol) to give the desired product (237mg) as a white solid m.p. 225°. MS m/z 266 (M+H)⁺.

### EXAMPLE 99

### N4-(4-Morpholinophenyl)-N2-(3,4,5-trimethoxyphenyl)-2,4-pyrimidinediamine

from 2-chloro-N-(4-morpholinophenyl)-4-pyrimidineamine (300mg, 1.03mmol) and 3,4,5-trimethoxyaniline (189mg, 1.03mmol) to give the title compound (230mg) as a white solid m.p. 214°. MS m/z 438 (M+H)⁺.
The pyrimidineamine starting material was prepared from 4-morpholinoaniline (285mg, 1.60mmol), 2,4-dichloropyrimidine (205mg, 1.60mmol) and triethylamine (0.25ml, 1.80mmol) to give the desired product (322mg) as a white solid m.p. 221°. MS m/z 291 (M+H)⁺.

### EXAMPLE 100

### N4-(3-Trifluoromethoxyphenyl)-N2-(3,4,5-trimethoxyphenyl)-2,4-pyrimidinediamine

from 2-chloro-N-(3-trifluoromethoxyphenyl)-4-pyrimidineamine (230mg, 0.79mmol) and 3,4,5-trimethoxyaniline (145mg, 0.79mmol) to give the title compound (220mg) as a white solid m.p. 154-156°. MS m/z 437 (M+H)⁺.
The pyrimidineamine starting material was prepared from 3-trifluoromethoxyaniline (354mg, 2.0mmol) 2,4-dichloropyrimidine (218mg, 2.0mmol) and triethylamine (0.3ml, 2.20mmol) to give the desired product (240mg) as a white solid m.p. 161-163°. MS m/z 290 (M+H)⁺.

### EXAMPLE 101

### N4-(2-Methylbenzimidazol-5-yl]-N2-(3,4,5-trimethoxyphenylamino)-2,4-pyrimidinediamine

from 2-chloro-N-(2-methylbenzimidazol-5-yl)-4-pyrimidineamine (250mg, 0.96mmol) and 3,4,5-trimethoxyaniline (176mg, 0.96mmol) to give the title compound (36mg) as a white solid m.p. 179°. MS m/z 407 (M+H)⁺.
The pyrimidineamine starting material was prepared from 5-amino-2-methylbenzimidazole (2147mg, 1.68mmol), 2,4-dichloropyrimidine (250mg, 1.68mmol) and triethylamine (0.25ml, 1.80mmol) to give the desired product (344mg) as a pale pink solid m.p. >300°. MS m/z 260 (M+H)⁺.

### EXAMPLE 102

### N4-(4-(Dimethylamino)phenyl)-N2-(3,4,5-trimethoxyphenylamino)-2,4-pyrimidinediamine

from 2-chloro-N-(4-(dimethylamino)phenyl)-4-pyrimidineamine (250mg, 1.0mmol) and 2,3,4-trimethoxyaniline (184mg, 1.0mmol) to give the title compound (127mg) as a grey solid m.p. 215°. MS m/z 396 (M+H)⁺.
The pyrimidineamine starting material was prepared from N,N-dimethyl-1,4-phenylenediamine (228mg, 1.68mmol) 2,4-dichloroprimidine (250mg, 1.68mol) and triethylamine (0.25ml, 1.80mmol) to give the desired product (402mg) as a white solid m.p. 212°.

### EXAMPLE 103

### N4-(4-(Ethoxycarbonylmethyl)phenyl)-N2-(3,4,5-trimethoxyphenyl)-2,4-pyrimidinediamine

from 2-chloro-N-(4-ethoxycarbonylmethyl)phenyl)-4-pyrimidineamine (1.74g, 5.97mmol) and 3,4,5-trimethoxyphenylaniline (1,09g, 5.97mmol) to give the title compound (1.01g) as a white solid m.p. 143-144°. δH (CDCl₃) 8.04 (1H, d, J 5.8Hz), 7.32 (2H, d, J 8.6Hz), 7.26 (2H, d, J 8.6Hz), 7.01 (1H, br s), 6.86 (2H, s), 6.64 (1H, br s), 6.15 (1H, d, J 5.8Hz), 4.17 (2H, q, J 7.1Hz), 3.82 (3H, s), 3.81 (6H, s), 3.60 (2H, s) and 1.27 (3H, t, J 7.1Hz). MS m/z 439 (M+H)⁺
The pyrimidineamine starting material was prepared from ethyl 4-aminophenylacetate (1.79g, 10.0mmol) 2,4-dichloropyrimidine (1.49g, 10.0mmol) and triethylamine (2.8ml, 20mmol) to give the desired product (2.88g) as a white solid, m.p. 151-152°.

### EXAMPLE 104

### N4-(4-Benzyloxyphenyl)-N2-(3,4,5-trimethoxyphenyl)-2,4-pyrimidinediamine

from N-(4-benzyloxyphenyl)-2-chloro-4-pyrimidineamine (25.0g, 80.0mmol) and 3,4,5-trimethoxyaniline (14.7g, 80.0mmol) to give the title compound (18.6g) as a white solid m.p. 161-164°. MS m/z 459 (M+H)⁺.
The pyrimidineamine starting material was prepared from 4-benzyloxyaniline (20.0g, 0.1mol), 2,4-dichloropyrimidine (15.0g, 0.1mol) and triethylamine (15.0ml, 0.11mol) to give the desired product (30.0g) as a cream solid m.p. 198°. MS m/z 312 (M+H)⁺.

### EXAMPLE 105

### N4-(3-Ethoxyphenyl)-N2-(3,4,5-triemthoxyphenyl)-2,4-pyrimidinediamine

from 2-chloro-N-(3-ethoxyphenyl)-4-pyrimidineamine (125mg, 0.5mmol) and 3,4,5-trimethoxyaniline (92mg, 0.5mmol) to give the title compound (157mg) as a white solid m.p. 79°. MS m/z 397 (M+H)⁺.
The pyrimidineamine starting material was prepared from metaphenetidine (0.15ml, 1.68mmol), 2,4-dichloropyrimidine (250mg, 1.68mmol) and triethylamine (0.25ml, 1.80mmol) to give the desired product (125mg) as a cream solid m.p. 97°.

### EXAMPLE 106

### N4-(4-Benzyloxycarbonylmethylphenyl)-N2-(3,4,5-trimethoxyphenyl)-2.4-pyrimidinediamine

from N-(4-(benzyloxy-carbonylmethyl)phenyl)-2-chloro-4-pyrimidineamine (3.14g, 8.90mmol) and 3,4,5-trimethoxyaniline (1.63g, 8.90mmol) to give the title compound (3,47g) as a white solid m.p. 141-143°. δH (CDCl₃) 8.04 (1H, d, J 5.8Hz), 7.36-7.24 (9H, m), 7.15 (1H, br s), 6.86 (2H, s), 6.74 (1H, br s), 6.15 (1H, d, J 5.8Hz), 5.15 (2H, s), 3.82 (3H, s), 3.81 (6H, s) and 3.66 (2H, s). MS m/z 501 (M+H)⁺.
The pyrimidineamine starting material was prepared from benzyl 4-aminophenylacetate (4.82g, 20.0mmol), 2,4-dichloropyrimidine (2.98g, 20.0mmol) and triethylamine (5.60ml, 40.0mmol) to give the desired product (3.24g) as a white solid m.p. 149°. MS m/z 354 (M+H).⁺

### EXAMPLE 107

### N4-(4-Methoxyphenyl)-N2-(3,4,5-trimethoxyphenyl)-2,4-pyrimidinediamine

from 2-chloro-N-(4-methoxyphenyl)-4-pyrimidineamine (315mg, 1.30mmol) and 3,4,5-trimethoxyaniline (270mg, 1.50mmol) to give the title compound (470mg) as a white solid m.p.210°. MS m/z 383 (M+H)⁺.
The pyrimidineamine starting material was prepared from para-anisidine (616mg, 5.0mmol), 2,4-dichloropyrimidine (745mg, 5.0mmol) and triethylamine (0.77ml, 5.5mmol) to give the desired compound (450mg) as a white solid m.p. 250°.

### EXAMPLE 108

### N4-(3-Benzyloxyphenyl)-N2-(3,4,5-trimethoxyphenyl)-2,4-pyrimidineamine

from N-(3-benzyloxyphenyl)-2-chloro-4-pyrimidineamine (2.0g, 6.4mmol) and 3,4,5-trimethoxyaniline (1.18g, 6.4mmol) to give the title compound (2.60g) as a white solid m.p. 186°. MS m/z 459 (M+H)⁺.
The pyrimidineamine starting material was prepared from 3-benzytoxyaniline (5.0g, 25.0mmol), 2,4-dichloropyrimidine (3.7g, 25.0mmol) and triethylamine (3.8ml, 27.5mmol) to give the desired compound (4.95g) as a white solid m.p. 119°. MS m/z 312 (M+H)⁺.

### EXAMPLE 109

### N4-(1-Phenylsulphonylindol-5-yl)-N2-(3,4,5-trimethoxyphenyl)-2,4-pyrimidinediamine

from 2-chloro-N-(1-phenylsulphonylindol-5-yl)-4-pyrimidineamine (1.09g, 2.70mmol) and 3,4,5-trimethoxyaniline (496mg, 2.70mmol) to give the title compound (950mg) as a white solid m.p. 127°. MSm/z 532 (M+H)⁺.
The pyrimidineamine starting material was prepared from 5-amino-1-phenylsulphonylindole (1.0g, 3.9mmol), 2,4-dichloropyrimidine (577mg, 3.9mmol) and triethylamine (0.61ml) to give the desired product (1.20g) as an orange solid m.p. 158°. MS m/z 385 (M+H)⁺.

### EXAMPLE 110

### N4-Cyclohexyl-N2-(3,4,5-trimethoxyphenyl)-2,4-pyrimidinediamine

from 2-chloro-N-cyclohexylamino-4-pyrimidineamine (200mg, 0.9mmol) and 3,4,5-trimethoxyaniline (173mg, 0.9mmol) to give the title compound (173mg) as a white solid m.p. 160-161°. δH (CDCl₃) 7.91 (1H, d, J 5.9Hz), 6.90 (1H,s ), 6.87 (2H, s), 5,81 (1H, d, J 5.9Hz), 4.63 (1H, br s), 3.86 (6H, s), 3.81 (3H, s), 2.00 (2H, m), 1.70 (4H, m) and 1.25 (4H, m). MS m/z 359 (M+H)⁺
The pyrimidineamine starting material was prepared from cyclohexylamine (0.4ml, 3.4mmol), 2,4-dichloropyrimidine (500mg, 3.4mmol) and triethylamine (0.5ml, 3.7mmol) to give the desired product (270mg) as a white solid m.p. 128°. MS m/z 212 (M+H)⁺.

### EXAMPLE 111

### N4-(1-Benzylpiperid-4-yl)-N2-(3,4,5-trimethoxyphenyl)-2,4-pyrimidinediamine

from N-(1-benzylpiperid-4-yl)-2-chloro-4-pyrimidineamine (390mg, 1.3mmol) and 3,4,5-trimethoxyaniline (236mg, 1.3mmol) to give the title compound (126mg) as a white solid m.p. 147°. δH (CDCl₃) 7.90 (1H, d, J 5.8Hz), 7.28 (5H, m), 6.94 (1H, s), 6.86 (2H, s), 5.81 (1H, d, J 5.8Hz), 4.62 (1H, br s), 3.85 (6H, s), 3.81 (3H, s), 3.53 (2H, s), 2.83 (2H, m), 2.16 (2H, m), 2.01 (2H, m) and 1.52 (2H, m).
The pyrimidineamine starting material was prepared from 4-amino-1-benzylpiperidine (0.7ml, 3.4mmol), 2,4-dichloropyrimidine (500mg, 3.4mmol) and triethylamine (0.5ml, 3.7mmol) to give the desired product (650mg) as a white solid m.p. 136°. MS m/z 303 (M+H)⁺.

### EXAMPLE 112

### N4-Benzyl-N2-(3,4,5-trimethoxyphenyl)-2,4-pyrimidinediamine

from N-benzyl-2-chloro-4-pyrimidineamine (1.5g, 6.83mmol) and 3,4,5-trimethoxyaniline (1.31g, 7.17mmol) to give the title compound (2.30g) as a white solid m.p. 167-168°. MS m/z 367 (M+H)⁺.
The pyrimidineanine starting material was prepared from benzylamine (3.67ml, 33.56mmol), 2,4-dichloropyrimidine (5.0g, 33.56mmol) and triethylamine (5.14ml, 36.9mmol) to give the desired product (4.21g) as a white solid. m.p. 135-136°. MS m/z 220 (M+H)⁺.

### EXAMPLE 113

### N2-(3,4-Dimethoxy-5-(methylthio)phenyl)-N4-(3-methoxyphenyl)-2,4-pyrimidinediamine

from 2-chloro-N-(3-methoxy-phenyl)-4-pyrimidineamine (0.59g, 2.51mmol) [see Example 92] and 3,4-dimethoxy-5-(methylthio)aniline (0.50g, 2.51mmol) to give the title compound (0.91g) as a white solid m.p. 68-70°. δH (CDCl₃) 8.02 (1H, d, J 5.8Hz), 7.40 (1H, br, s), 7.26 (1H, m), 7.08 (1H, d, J 2.2Hz), 6.90 (4H, m), 6.68 (1H, dd, J 8.2, 2.2Hz), 6.20 (1H, d, J 5.8Hz), 3.82 (3H, s), 3.79 (3H, s), 3.77 (3H, s) and 2.35 (3H, s), MS m/z 399 (M+H)⁺.
The aniline starting material was prepared by treating a solution of 1,2-dimethoxy-3-(methylsulphinyl)-5-nitrobenzene (1.64g, 6.69mmol) in methanol (20ml) and concentrated hydrochloric acid (20ml) was treated with anhydrous tin (II) chloride (7.15g, 37.7mmol) and the resulting mixture refluxed for 1.25h. On cooling to room temperature the mixture was poured into excess 1M NaOH solution and extracted with CH₂Cl₂. The organic extract was dried (MgSO₄) and evaporated to afford the desired product (1.33g) as an off-white solid m.p. 106-107°. MS m/z 199 (M+H)⁺.

### EXAMPLE 114

### N2-(3,4-Dimethoxy-5-methylphenyl)-N4-(3-methoxyphenyl)-2,4-pyrimidinediamine

from 2-chloro-N-(3-methoxyphenyl)-4-pyrimidineamine (1.93g) [see Example 92] and 3,4-dimethoxy-5-methylaniline (1.50g) to give the title compound (2.53g) as a pale pink solid m.p. 66-68°. δH (CDCl₃) 8.02 (1H, d, J 5.8Hz), 7.39 (1 H, br s), 7.23 (1 H, t, J 8.1Hz), 7.08 (1 H, m), 7.05 (1H, br s), 6.97-6.91 (2H, m), 6.86 (1H, d, J 2.0Hz), 6.69-6.65 (1H, m), 6.18 (1H, d, J 5.8Hz), 3.77 (3H, s), 3.75 (6H, s) and 2.22 (3H, s). MS m/z 367 (M+H)⁺.
The aniline used as starting material was prepared according to the methods of I Sanchez*et al*: Tetrahedron 41, 2355 (1985).

### EXAMPLE 115

### N2-(3,4-Dimethoxyphenyl)-N4-(3-methoxyphenyl)-2,4-pyrimidinediamine

from 2-chloro-N-(3-methoxyphenyl)-4-pyrimidineamine (0.77g, 3.27mmol) [see Example 92] and 3,4-dimethoxyaniline (0.50g, 3.27mmol) to give the title compound (0.89g) as a beige solid m.p. 135-138°. δH (CDCl₃) 8.03 (1H, d, J 5.8Hz), 7.22 (2H, m), 7.13 (1H, br s), 7.02 (1H, dd, J 8.6, 2.3Hz), 6.97 (1H, m), 6.92 (1H, m), 6.82 (1H, d, J 87.6Hz), 6.77 (1H, br s), 6.68 (1H, dd, J 8.2, 2.3Hz), 6.18 (1H, d, J 5.8Hz), 3.86 (3H, s), 3.83 (3H, s) and 3.77 (3H, s). MS m/z 353 (M+H)⁺.

### EXAMPLE 116

### 5-Bromo-N2.N4-Bis(trimethoxyphenyl)-2,4-pyrimidinediamine

from 5-bromo-2-chloro-N-(3,4,5-trimethoxyphenyl)-4-pyrimidineamine (936mg, 2.5mmol) and 3,4,5-trimethoxyaniline (459mg, 2.5mmol) to give the title compound (160mg) as a white solid m.p. 186-191°. δH (CDCl₃) 8.16 (1H, s), 7.45 (1H, br s), 6.98 (1H, br s), 6.77 (2H, s), 6.74 (2H, s), 3.83 (3H, s), 3.80 (3H, s), 3.71 (6H, s), 3.66 (6H, s), MS m/z 523 [(M+H)⁺, ⁸¹Br,100%] 521 [(M+H)⁺, ⁷⁹Br, 100%].
The pyrimidineamine starting material was prepared from 5-bromo-2,4-dichloropyrimidine (1.14g, 5.0mmol), 3,4,5-trimethoxyaniline (916mg, 5.0mmol) and triethylamine (0.77ml, 5.5mmol) following the method used for the pyrimidineamine starting material in Example 89. This gave the desired compound as a white solid (1.71g) m.p. 185-186°. MS m/z 378 [(M+H)⁺, 22%], 376 [(M+H)⁺, 100%], 374 [(M+H)⁺, 78%].

### EXAMPLE 117

### N4-(4-Hydroxyphenyl)-N2-(3,4,5-trimethoxyphenyl)-2,4-pyrimidinediamine

In a manner analogous to the preparation of the compound of Example 49 from the compound of Example 104 (3.0g, 6.55mmol), ammonium formate (1.20g, 19.60mmol) and 10% palladium on carbon (300mg) to give the title compound (2.40g) as a white solid m.p. 112-115°. δH (CDCl₃) 7/96 )1H. d, J 5.9Hz), 7.16 (2H, d, J 8.7Hz), 7.06 (1H, s), 6.83 (1H, s), 6.80 (3H, m), 6.68 (1H, s), 6.01 (1H, d, J 5.9Hz), 3.79 (3H, s) and 3.78 (6H, s). MS m/z 369 (M+H)⁺.

### EXAMPLE 118

### N4-(4-(3-Aminopropoxy)phenyl)-N2-(3,4,5-trimethoxyphenyl)-2,4-pyrimidinediamine dihydrochloride

In a manner analogous to the preparation of the compound of Example 48 from N4-(4-(3-phthalimidopropoxy)phenyl)-N2-(3,4,5-trimethoxyphenyl)-2,4-pyrimidinediamine (400mg, 0.72mmol) and hydrazine monohydrate (0.1ml, 2.16mmol) to give the title compound (108mg) as a buff solid m.p. 258°. δH (d⁶ DMSO) 11.04 (1H, s), 10.54 (1H, s), 8,08 (3H, s), 7.91 (1H, d, J 7.3Hz), 7.55 (2H, m), 6.86 (2H, d, J 8.7Hz), 6.78 (2H, s), 6.49 (1H, s), 4.03 (2H, m), 3.65 (9H, s), 2.94 (2H, m) and 2.02 (2H, m). MS m/z 426 (M+H)⁺.

The pyrimidinediemine starting material was prepared according to the method of Example 45 from the compound of Example 117 and 3-bromopropylphthalimide

### EXAMPLE 119

### N4-(4-(Carboxymethyl)phenyl)-N2-(3,4,5-trimethoxyphenyl)-2,4-pyrimidinediamine

The compound of Example 103 (250mg, 0.57mmol) in a solution of ethanol (10ml) containing 6N aqueous NaOH (2.0ml) was heated a reflux for 2h. On cooling the ethanol was removed under reduced pressure and the basic solution brought to pH 5 with 2M hydrochloric acid. The resulting solution was concentrated under reduced pressure, and the residue taken up in hot ethanol (20ml). This solution was filtered and the filtrate evaporated under reduced pressure to give a residue which was crystalised from ethanol to give the title compound (89mg) as a white solid m.p. 144-150°. MS m/z 411 (M+H)⁺.

### EXAMPLE 120

### N4-(4-(tert-Butoxycarbonylmethoxy)phenyl)-N2-(3,4,5-trimethoxyphenyl)-2,4-pyrimidinediamine

In a manner analogous to the preparation of the compound of Example 50, from the compound of Example 120 (500mg, 1.40mmol), *tert*-butyl bromoacetate (0.2ml, 1.40mmol) and NaH (60% dispersion in oil) (60ml, 1.50mmol) to give the title compound (380mg) as a green solid m.p.139°. MS m/z 483 (M+H)⁺.

### EXAMPLE 121

### N4-(4-(2-Hydroxyethyl)phenyl)-N2-(3,4,5-trimethoxyphenyl)-2,4-pyrimidinediamine

In a manner analogous to the preparation of the compound of Example 62 (267mg, 0.5mmol) from the compound of Example 106 and LiAlH₄ (40mg, 1.1mmol) to give the title compound (108mg) as a light pink solid m.p. 1720. MS m/z 397 (M+H)⁺.

### EXAMPLE 122

### N4-(4-(Carboxymethoxy)phenyl)-N2-(3,4,5-trimethoxyphenyl)-2,4-pyrimidinediamine

A solution of the compound of Example 123 (300mg, 0.62mmol) in CH₂Cl₂ (20ml) was treated with trifluoroacetic acid (3.0ml) and stirred at room temperature for 5h. After this time the reaction was concentrated under reduced pressure to give a brown oil, which was subjected to column chromatography [silica CH₂Cl₂/methanol/acetic acid/water 86.5:10:2:1.5] to give the title compound (123mg) as a white solid m.p. 159° after recrystallisation from methanol. MS m/z 427 (M+H)⁺.

### EXAMPLE 123

### 4-Phenoxy-N2-(3,4,5-trimethoxyphenyl)-2-pyrimidineamine

2-Methylsulphonyl-4-phenoxypyrimidine (0.76g, 3.04mmol) and 3,4,5-trimethoxyaniline (0.56g, 4.0mmol) were heated as a melt at 140° for 2h. On cooling the residue was partitioned between ethyl acetate (50ml) and 2M hydrochloric acid (50ml), and the organic layer was washed with water (50ml), dried (MgSO₄) and concentrated under reduced pressure. The residue was subjected to column chromatography [silica 20% ethyl acetate-hexane] to give the title compound (18mg) as a white solid m.p. 149° after recrystallisation from ethyl acetate. δH (CDCl₃) 8.26 (1H, d, J 5.6Hz), 7.39 (2H, m), 7.26 (1H,m), 7.18-7.15 (2H, m), 6.73 (2H, s), 6.31 (1 H, d, J 5.6Hz), 3.77 (3H, s) and 3.64 (6H, s). MS m/z 354 (M+H)⁺.
The pyrimidine used as starting material was prepared by the addition of 50% 3-chloroperoxybenzoic acid (15.19g, 88.0mmol) to a solution of 4-phenoxy-2-thiomethylpyrimidine (5.0g, 22.9mmol) in CH₂Cl₂ (100ml) at 0°. The resulting mixture was allowed to rise to room temperature over 12h, and was then washed with 2M aqueous NaOH (2x100ml), saturated aqueous sodium sulphite (2x100ml), dried (MgSO₄) and concentrated under reduced pressure. The residue was subjected to column chromatography to give the desired product (0.81g) as a colourless oil. δH (CDCl₃) 8.49 (1H, d, J 5.5Hz), 7.45 (2H, t, J 8.0Hz), 7.33-7.26 (1H, m), 7.19-7.16 (2H, m), 6.85 (1H, d, J 5.5Hz) and 3.23 (3H, s).
The 4-phenoxy-2-thiomethylpyrimidine used as starting material was prepared by heating a solution of 4-fluoro-2-thiomethylpyridine [N. Plé *et al.*, J. Het. Chem. 31, 1311-1315 (1994)] (9.98g, 69.3mmol) and phenol (6.58g, 70.0mmol) in dry DMF (100ml) in the presence of caesium carbonate (22.8g, 70.0mmol) at 40° for 6h. The reaction was concentrated *in vacuo* and the residue was partitioned between ethyl acetate (100ml) and water (100ml). The organic layer was dried (MgSO₄), concentrated under reduced pressure and the residue subjected to column chromatography [silica 40% ethyl acetate-hexane] to give the desired product (7.01g) as a colourless oil. δH (CDCl₃) 8.33 (1H, dd, J 4.7, 0.9Hz), 7.40 (2H, dd, 8.0, 1.1Hz), 7.26 (1H, m), 7.23 (2H, m), 6.46 (1H, dd, J 4.7, 0.9Hz) and 2.37 (3H, s). MS m/z 219 (M+H)⁺.

### EXAMPLE 124

### N.N'-Bis(3,4,5-Trimethoxyphenyl)-2,4-pyrimidinediamine

A solution of 2,4-dichloropyrimidine (1.0g, 6.7mmol), 3,4,5-trimethoxyaniline (2.5g, 13.4mmol) and triethylamnine (1.8ml, 12.6mmol) was heated at reflux for 5h. On cooling the resulting precipitate was collected and recrystallised from ethyl acetate to give the title compound (450mg) as a purple solid m.p. 180°. MS m/z 443 (M+H)⁺.

### EXAMPLE 125

### 4-Benzoyl-N-(3,4,5-trimethoxyphenyl)-2-pyrimidineamine

To a solution of N-methoxy-N-methyl-2-(3,4,5-trimethoxyphenylamino)-pyrimidine-4-carboxamide (0.82g, 2.30mmol) in THF (40ml) cooled to -78° under a nitrogen atmosphere was added phenylmagnesium bromide [Aldrich 1M/THF] (6.90ml, 6.90mmol) dropwise. On completion of addition the reaction was left to warm to room temperature over 3h and aqueous NH₄Cl solution (10ml) was then added and the mixture concentrated under reduced pressure. The residue was partitioned between water (20ml) and ethyl acetate (40ml), the organic layer dried (MgSO₄) and evaporated. The residue was subjected to column chromatography [silica 50% hexane-ethyl acetate] and after recrystallisation from ethyl acetate-hexane the title compound (123mg) was obtained as a yellow solid m.p 135°. δH (CDCl₃) 8.65 (1H, d, J 4.8Hz), 8.05 (2H, dd, J 5.1, 2.0Hz), 7.61 (1H, dd, J 5.3, 2.0Hz), 7.46 (2H, m), 7.24 (1H, d, J 4.8Hz), 3.79 (3H, s) and 3.63 (6H, s). MS m/z 366 (M+H)⁺.
The pyrimidine-4-carboxamide used as starting material was prepared by treating a solution of 4-carboxy-N-(3,4,5-trimethoxyphenyl)-2-pyrimidineamine hydrochloride (2.0g, 5.86mmol) and triethylamine (2.0ml, 14.0mmol) in CH₂Cl₂ (40ml), cooled to -20° under a nitrogen atmosphere, with isobutylchloroformate (1.1ml, 7.0mmol). The resulting mixture was stirred for 20min. before N,O-dimethylhydroxylamine hydrochloride (683mg, 7.0mmol) and triethylamine (1.0ml, 7.0mmol) were added and the reaction allowed to warm to room temperature over 3h. After washing with 2M hydrochloric acid (1x50ml) and 2M NaOH solution (1x50ml), the reaction was dried (MgSO₄) and concentrated under reduced pressure. The residue was subjected to column chromatography [silica ethyl acetate] to give the desired product (1.02g), as a yellow oil. δH (CDCl₃) 8.51 (1H, d, J 4.9Hz), 7.21 (1H, br s), 6.92 (3H, br s), 3.86 (6H, s), 3.82 (3H, s), 3.69 (3H, br s) and 3.36 (3H, s). MS m/z 349 (M+H)⁺.
The 4-carboxy-N-(3,4,5-trimethoxyphenyl)-2-pyrimidineamine hydrochloride was prepared by heating a solution of 4-cyano-N-(3,4,5-trimethoxyphenyl)-N-pyrimidineamine (2.88g, 10.49mmol) in aqueous 2N NaOH (60ml) and ethanol (10ml) at reflux for 3h. On cooling the reaction mixtrure was adjusted to pH 3 with 2M hydrochloric acid, and the resulting precipitate collected and dried to give the desired product (2.7g) as an orange solid m.p. 241°. MS m/z 306 (M+H)⁺.
The 4-cyano-N-(3,4,5-trimethoxyphenyl)-2-pyrimidineamine was prepared by heating a solution of 2-chloro-4-cyanopyrimidine [G. Davies *et al. J.* Het. Chem. 1, 130-133, (1963)] (11.0g, 78.8mmol), 3,4,5-trimethoxy aniline (14.4g, 79.4mmol) and triethylamine (12.0ml) in ethanol at reflux for 4h. On cooling the resulting precipitate was collected and dried to give the desired product (13.70 g) as a yellow solid m.p. 165°. MS m/z 287 (M+H)⁺.

### EXAMPLE 126

### N-Phenyl-2-(3,4,5-trimethoxyphenylamino)pyrimidine-4-carboxamide

In a manner analogous to the preparation of the pyrimidine-4-carbox amide intermediate of Example 125, from 4-carboxy-N-(3,4,5-trimethoxyphenyl)-2-pyrimidineamine hydrochloride (1.0g, 2.92mmol), isobutylchloroformate (0.4ml, 3.07mmol), aniline (0.28ml, 3.07mmol) and triethylamine (1.22ml, 8.76mmol) to give the title compound (655mg) as a yellow solid m.p. 158-159°. δH (CDCl₃) 9.66 (1H, br s), 8.67 (1H, d, J 4.9Hz), 7.71-7.67 (2H, m), 7.58 (1H, d, J 4.9Hz), 7.40-7.34 (3H, m), 7.19-7.14 (1H, m), 6.88 (2H, s), 3.87 (6H, s) and 3.86 (3H, s). MS m/z 381 (M+H)⁺.

### EXAMPLE 127

### 4-Phenylsulphonamido-N2-(3,4,5-trimethoxyphenyl)-2-pyrimidineamine

A solution of 2-chloro-4-phenylsulphonamidopyrimidine (250mg, 0.93mmol) and 3,4,5-trimethoxyaniline (187mg, 1.02mmol) in ethoxyethanol (5ml) was heated at 140° for 12h. After this time the solvent was evaporated under reduced pressure and the residue partitioned between ethyl acetate (10ml) and water (10ml). The organic layer was washed with water (2x25ml), dried (MgSO₄) and evaporated and the resulting solid subjected to column chromatography [silica-ethyl acetate] to give the title compound (187mg) as a buff solid, m.p. 222-223°. δH (CDCl₃) 8.11 (1H, d, J 5.9Hz), 7.92 (3H, m), 7.57 (1H, m), 7.50 (3H, m), 6.80 (2H, s), 6.66 (1H, d, J 5.9Hz), 3.81 (6H, s) and 3.21 (3H, s). MS m/z 417 (M+H)⁺.
The 2-chloro-4-phenylsulphonamidopyrimidine was prepared by heating 2,4-dichloropyrimidine (1.42g, 9.5mmol), benzenesulphonamide (4.5g, 29.0mmol) and potassium, carbonate (3.3g, 24.0mmol) in DMA (25ml) for 0.75h). The reaction was cooled to 5°, water (20ml) added, and adjusted to pH2.5 with 2M HCl. The resulting precipitate was collected to give the desired product (1.8g) as a light yellow solid m.p. 164°. MS m/z 270 (M+H)⁺.

### EXAMPLE 128

### N4-(tert-Butoxycarbonyl)-N2-(3,4,5-trimethoxyphenyl)-2,4-pyrimidinediamine

To a solution of diphenylphosphorylazide (0.92ml, 4.3mmol) in *tert*-butyl alcohol (10ml), was added triethylamine (1,2ml, 8.5mmol) and 4-carboxy-N-(3,4,5-trimethoxyphenyl)-2-pyrimidineamine (1.0g, 2.9mmol), [see Example 118] and the mixture heated at reflux for 2h. After this time the solvent was evaporated and the residue partitioned between ethyl acetate (100ml) and water (100ml). The organic layer was washed with water (100ml), dried (MgSO₄) and concentrated under reduced pressure. Column chromatography [silica 2% methanol CH₂Cl₂] gave the title compound (850mg) as a light yellow solid m.p. 124°. δH (CDCl₃) 8.27 (1 H, d, J 5.8Hz), 7.45 (1H, br s), 7.32 (1H, d, J 5.8Hz), 7.20 (1H, br s), 6.81 (2H, s), 3.85 (6H, s), 3.81 (3H, s) and 1.53 (9H, s). MS m/z 377 (M+H)⁺.

### EXAMPLE 129

### 4-Phenylcarboxamido-N-(3,4,5-trimethoxyphenyl)-2-pyrimidineamine

In a manner analogous to the preparation of the pyrimidine-4-caboxamide intermediate of Example 125, from benzoic acid (66mg, 0.54mmol), isobutylchloroformate (0.078ml, 0.6mmol) N2-(3,4,5-trimethoxyphenyl)-2,4-pyrimidinediamine (150mg, 0.54mmol) to give the title compound (30mg) as a white solid m.p. 88-89°. δH (CDCl₃) 8.39 (1H, d, J 5.6Hz), 8.32 (1H, br s), 7.89-7.85 (2H, m), 7.74 (1H, d, J 5.6Hz), 7.64-6.50 (3H,m), 6.99 (1H, br s), 6.85 (2H, s), 3.88 (6H, s) and 3.84 (3H, s). MS m/z 381 (M+H)⁺.
The N2-(3,4,5-trimethoxyphenyl)-2,4-pyrimidinediamine used as starting material was prepared by treating a solution of the compound of Example 131 (600mg, 1.6mmol) in methanol (10ml) with 2M hydrochloric acid (10ml), which was then heated at reflux for 2h. On cooling the methanol was evaporated and the aqueous solution adjusted to pH 12 with 2N aqueous NaOH. The basic solution was extracted with CH₂Cl₂ (3x30ml), then the combined organic extracts were washed with saturated brine (1x20ml), dried (MgSO₄) and concentrated under reduced pressure to give the desired product (340mg) as a light yelllow solid m.p. 89°. MS m/z 277 (M+H)⁺.

The compound of Examples 130-133 were prepared in a similar manner to the compound of Example 1 from the starting materials shown:

### EXAMPLE 130

### 5-Methoxy-4-phenyl-N-(3,4,5-trimethoxyphenyl)-2-pyrimidineamine

from 3,4,5-trimethoxyphenylguanidine nitrate (1.43g, 5.0mmol), 3-dimethylamino-2-methoxy-1-phenyl-2-propen-1-one (1.03g, 5.0mmol) and NaOH (220mg) to give the title compound (200mg) as straw coloured crystals m.p. 168-170°. δH (d⁶ DMSO) 9.36 (1H, br s), 8.47 (1H, s), 8.09 (2H, m), 7.48 (3H, s), 7.26 (2H, s), 3.88 (3H, s), 3.75 (6H, s) and 3.59 (3H, s). MS m/z 368 (M+H)⁺.
The 3-dimethylamino-2-methoxy-1-phenyl-2-propen-1-one was prepared in a similar manner to the analogous starting material of Example 61, from 2-methoxyacetophenone (4.51g, 30.0mmol) to give the desired product (5.30g) as an orange oil. MS m/z 206 (M+H)⁺.

### EXAMPLE 131

### 4-Phenyl-5-thiomethyl-N-(3,4,5-trimethoxyphenyl)-2-pyrimidineamine

from 3,4,5-trimethoxyphenylguanidine nitrate (1.43g, 5.0mmol), 3-dimethylamino-1-phenyl-2-thiomethyl-2-propen-1-one (1.11g, 5.0mmol) and NaOH (220mg, 5.5mmol) to give the title compound (79.8mg) as light yellow crystals m.p. 143°. δH (CDCl₃) 8.52 (1H, s), 7.88-7.85 (2H, m), 7.49 (3H, m), 7.16 (1H, br s), 7.02 (2H, s), 3.86 (6H, s), 3.82 (3H, s) and 2.26 (3H, s). MS m/z 384 (M+H)⁺.
The 3-dimethylamino-1-phenyl-2-thiomethyl-2-propen-1-one was prepared in a similar manner to the analogous starting material of Example 61, from 2-thiomethylacetophenone (4.28g, 25.7mmol) to give the desired product (3.41g) as an orange oil. δH (CDCl₃) 7.49-7.47 (2H, m), 7.46 (1H, s), 7.41-7.34 (3H, s), 3.27 (6H, s) and 2.12 (3H, s) MS m/z 222 (M+H)⁺.

### EXAMPLE 132

### 5-Nitro-4-phenyl-N-(3.4.5-trimethoxyphenyl)-2-pyrimidineamine

from 3,4,5-trimethoxyphenylguanidine nitrate (1.14g, 4.0mmol), 3-dimethylamino-2-nitro-1-phenyl-2-propen-1-one (0.88g, 4.0mmol) and NaOH (176mg, 4.4mmol) to give the title compound (1.33g) as an orange powder m.p. 188-189°. δH (d⁶ DMSO) 10.60 (1H, br s), 9.16 (1H, s), 7.61 (2H, m), 7.56-7.46 (3H, m), 7.25 (2H, br s), 3.74 (6H, s) and 3.63 (3H, s). MS m/z 383 (M+H)⁺.
The 3-dimethylamino-2-nitro-1-phenyl-2-propen-1-one was prepared in a similar manner to the analogous starting material of Example 61 from benzoylnitromethane (4.12g, 25.0mmol) to give the desired product (1.27g) as yellow crystals m.p. 103-105°. MS m/z 243 [(M+Na)⁺, 70%] 221 [(M+H)⁺, 100%].

### EXAMPLE 133

### 5-Ethoxycarbonyl-4-phenyl-N-(3,4,5-trimethoxyphenyl)-2-pyrimidineamine

from 3,4,5-trimethoxyphenylguanidine nitrate (2.86g, 10.0mmol), 3-dimethylamino-2-ethoxycarbonyl-1-phenyl-2-propen-1-one (2.47g, 10.0mmol) and NaOH (440mg, 11.0mmol) to give the title compound (3.10g) as straw coloured crystals m.p. 160-161°. δH (d⁶ DMSO) 10.12 (1H, br s), 8.87 (1H, s), 7.59 (2H, m), 7.50-7.45 (3H, m), 7.28 (2H, s), 4.11 (2H, q. J 7.1 Hz), 3.74 (6H, s), 3.61 (3H, s) and 1.06 (3H, t, J 7.1Hz).
The 3-dimethylamino-2-ethoxycarbonyl-1-phenyl-2-propen-1-one was prepared in a similar manner to the analogous starting material of Example 61, from ethylbenzoylacetate (5.77g, 30.0mmol) to give the desired product (6.01g) as a yellow solid m.p. 64-65°. MS m/z 248 (M+H)⁺.

### EXAMPLE 134

### 5-Amino-4-phenyl-N-(3,4,5-trimethoxyphenyl)-2-pyrimidineamine

In a manner analogous to the preparation of the compound of Example 38, from the compound of Example 132 (500mg, 1.32mmol) to give the title compound (260mg) as a yellow solid m.p. 149-150°. δH (CDCl₃) 8.09 (1H, s), 7.87-7.84 (2H, m), 7.51-7.47 (3H, m), 7.00 (2H, s), 6.87 (1H, br s), 3.86 (6H, s), 3.81 (3H, s) and 3.52 (2H, br s). MS m/z 353 (M+H)⁺.

### EXAMPLE 135

### 4-Phenylsulphanyl-N-(3,4,5-trimethoxyphenyl)-2-pyrimidineamine

In a manner analogous to the preparation of the compound of Example 89, from 2-chloro-4-phenyl-sulphanylpyrimidine (1.41g, 6.35mmol) and 3,4,5-trimethoxyphenylaniline (1.16g, 6.35mmol) to give the title compound (1.43g) as an off-white solid m.p. 110°. δH (CDCl₃) 8.04 (1 H, d, J 5.4Hz), 7.59 (2H,m), 7.47 (3H, m), 7.22 (1H, br s), 6.88 (2H, s), 6.18 (1H, d, J 5.4Hz), 3.83 (6H, s) and 3.81 (3H, s). MS m/z 370 (M+H)⁺.
The 2-chloro-4-phenylsulphanylpyrimidine was prepared by treating a solution of thiophenol (4.7ml, 45.4mmol) in THF (30ml) at 0°, with a 1.0M solution of sodium bis(trimethylsilyl)amide in THF (45.4ml) and the mixture stirred for 0.5h. After a thick white precipitate had formed, 2,4-dichloropyrimidine (6.83g, 45.4mmol) and DMA (70ml) were added and the mixture heated at 120° for 2h. The reaction was then concentrated under reduced pressure and the residue partitioned between wat er and ethyl acetate. The aqueous layer was further extracted with ethyl acetate and the combined organic layers were dried (MgSO₄) and evaporated. The residue was subjected to column chromatography (silica 20% ethyl acetate-hexane) to give the desired product (2.66g) as a white solid. δH 8.17 (1H, d, J 5.5Hz), 7.59 (2H, m), 7.53 (3H, m) and 6.62 (1H, d, J 5.5Hz). MS m/z 223 (M+H)⁺.

### EXAMPLE 136

### N-(4-(2-(3,4,5-Trimethoxyphenylamino)pyrimidin-4-yl)-2-aminoacetamide

A solution of N-(4-(2-(3,4,5-trimethoxyphenylamino)pyrimidin-4-yl)phenyl)-2-N-(9-fluorenylmethoxycarbonyl)aminoacetamide (210mg, 0.33mmol) and piperidine (0.5ml, 5mmol) in DMF (2.5ml) was stirred at room temperature for 0.5h. The sovlent was evaporated and the residue triturated with hot ethyl acetate to give the title compound (25mg) as an off-white solid m.p. 213°. δH (d⁶ DMSO) 9.47 (1H, s), 8.49 (1H, d, J 5.4Hz), 8.11 (2H, d, J 8.3Hz), 7.75 (2H, d, J 8.2Hz), 7.29 (3H,m), 3.80 (6H, s) and 3.61 (3H, s). MS m/z 410 (M+H)⁺.
The starting material for the above process was obtained by treating a solution of the compound of Example 39 (250mg, 0.71 mmol) in CH₂Cl₂ (10ml) with N-FMOCglycyl chloride (337mg, 1.07mmol) and 5% aqueous Na₂CO₃ solution (8ml). After the mixture had been stirred for 1h at room temperature, the aqueous phase was discarded and the organic layer dried (MgSO₄) and evaporated. The residue was subjected to column chromatography [silica 10% methanol-CH₂Cl₂] to give the desired product (235mg) as an off-white solid. m.p. 102° (decomp.). δH (d⁶ DMSO) 10.23 (1H, s), 9.48 (1H, s), 8.48 (1H, d, J 5.1Hz), 8.15 (2H, d, J 8.3Hz), 7.88 (2H, d, J 7.3Hz), 7.73 (4H, m), 7.64 (1H, m), 7.43-7.30 (7H, m), 4.34-4.20 (3H, m), 3.79 (8H, m) and 3.62 (3H, s).

### EXAMPLE 137

### 6-Methyl-4-phenoxy-N-(3,4,5-trimethoxyphenyl)pyrimidine-2-amine

4-Chloro-6-methyl-N-(3,4,5-trimethoxyphenyl)pyrimidine-2-amine (0.30g, 0.97mmol) was added to a solution of sodium phenoxide (0.97mmol) [prepared from phenol (0.091g, 0.97mmol) and NaH (60% in mineral oil; 0.039g, 0.97mmol)], and heated to 80° for 17h with stirring. Water was added and the mixture was extracted with CH₂CH₂ (2x30ml), the organic phases were dried (MgSO₄) and evaporated to a yellow oil. The residue was subjected to chromatography (silica, 2% methanol/CH₂CH₂ ) to give the title compound (D.046g). A portion was recrystallised from methanol to give an off white crystalline solid m.p. 175°. δ_{H} (CDCl₃) 2.38 (3H, s), 3.64 (6H, s), 3.77 (3H, s), 6.19 (1H, s), 6.78 (2H, s), 6.95 (1H, s), 7.15 (2H, d, J 7.5Hz), 7.23 (1 H, m) and 7.39 (2H, t, J 7.5Hz).

The amine starting material for this reaction was prepared from the following:

### 4-Chloro-6-methyl-N-(3,4,5-trimethoxyphenyl)pyrimidine-2-amine (b)

6-Methyl-2-(3,4,5-trimethoxyphenylamino)-4(3H)pyrimidinone (1.0g, 3.63mmol) was dissolved in phosphoryl chloride and heated to 100° for 2 h. The mixture was cooled to room temperature and evaporated *in vacuo* to a viscous oil. The oil was dissolved in CH₂CH₂ (50ml) and washed twice with saturated aqueous NaHCO₃, the organic phases dried (MgSO₄) and concentrated *in vacuo.* The residue was subjected to column chromatography (silica, 2% methanol/CH₂CH₂) to afford the title compound as an off white solid (1.03g). A small amount was recrystallised from ethyl acetate to give fine off-white needles. m.p. 195-196°. δ_{H} (CDCl₃) 2.40 (3H, s), 3.82 (3H, s), 3.88 (6H, s), 6.64 (1H, s), 6.95 (2H, s), and 7.07 (1 H, br s).

The pyrimidinone starting material for this reaction was prepared as follows:

### 6-Methyl-2-(3,4,5-trimethoxyphenylamino)-4-(3H)pyrimidinone

To a solution of sodium methoxide (0.96g, 1.79mmol) in methanol was added ethyl-3-oxo-butanoate (0.23g, 1.79mmol) and 3,4,5-trimethoxyphenylguanidinium nitrate (0.5g, 1.79mmol). The mixture was stirred at reflux for 17h. The reaction mixture was cooled to room temperature and evaporated *in vacuo* to give a dark grey solid. Recrystallisation from ethanol gave the title compound (0.25g, 49%) as a light grey solid, m.p. 228-230° (dec). δ_{H} (d⁶DMSO) 2.12 (3H, s), 3.61 (3H, s), 3.74 (6H, s), 5.69 (1H, s), 7.02 (2H, s), 8.70 (1H, br s) and 10.47 (1H, br s).

### BIOLOGICAL ACTIVITY

The following assays were used to demonstrate the activity and selectivity of compounds according to the invention:

### p56^{Ick} kinase assay

The tyrosine kinase activity of p56^{Ick} was determined using a RR-src peptide (RRLIEDNEYTARG) and [γ-³³P]ATP as substrates. Quantitation of the ³³P-phosphorylated peptide formed by the action of p56^{Ick} was achieved using an adaption of the method of Geissler *et al* (J. Biol. Chem. (1990) 265, 22255-22261).

All assays were performed in 20mM HEPES pH 7.5 containing 10mM MgCl₂, 10mM MnCl₂, 0.05% Brij, 1µM ATP (0.5µCi[γ-³³P]ATP) and 0.8mg/ml RR-src. Inhibitors in dimethylsulphoxide (DMSO) were added such that the final concentration of DMSO did not exceed 1%, and enzyme [human p56^{lck}] such that the consumption of ATP was less than 10%. After incubation at 30°C for 15min, the reaction was terminated by the addition of one-third volume of stop reagent (0.25mM EDTA and 33mM ATP in dH₂O). A 15µl aliquot was removed, spotted onto a P-30 filtermat (Wallac, Milton Keynes, UK), and washed sequentially with 1% acetic acid and dH₂O to remove ATP. The bound ³³P-RR-src was quantitated by scintillation counting of the filtermat in a Betaplate scintillation counter (Wallac, Milton Keynes, UK) after addition of Meltilex scintillant (Wallac, Milton Keynes, UK). The dpm obtained, being directly proportional to the amount of ³³P-RR-src produced by p56^{lck}, were used to determine the IC₅₀ for each compound.

### p59^{fyn} kinase assay

Compounds of the invention were assayed for p59^{fyn} inhibitory activity in a similar manner to the p56^{lck} assay, using human p59^{fyn}.

### EGFr kinase assay

The tyrosine kinase activity of the EGF receptor (EGFr) was determined using a similar methodology to the p56^{lck} kinase assay, except that the RR-src peptide was replaced by a peptide substrate for EGFr obtained from Amersham International plc (Little Chalfont, UK) and used at the manufacturers recommended concentration. IC₅₀ values for each test inhibitor were determined as described previously in the p56^{lck} assay.

### ZAP-70 kinase assay

The tyrosine kinase activity of ZAP-70 was determined using a capture assay based on that employed above for p56^{lck}. The RR-src peptide was replaced with polyGlu-Tyr (Sigma; Poole, UK) at a final concentration of 17 µg/ml. After addition of the stopped reaction to the filtermat, trichloroacetic acid 10% (w/v) was employed as the wash reagent instead of acetic acid and a final wash in absolute ethanol was also performed before scintillation counting. IC₅₀ values for each test inhibitor were determined as described above in the p56^{lck} assay.

### Csk kinase assay

Compounds of the invention were assayed for csk kinase inhibitory activity in a similar manner to the ZAP-70 assay using human csk kinase.

### Protein kinase C assay

Inhibitor activity against protein kinase C (PKC) was determined using PKC obtained from Sigma Chemical Company (Poole, UK) and a commercially available assay system (Amersham International plc, Little Chalfont, UK). Briefly, PKC catalyses the transfer of the γ-phosphate (³²P) of ATP to the threonine group on a peptide specific for PKC. Phosphorylated peptide is bound to phosphocellulose paper, subsequently quantified by scintillation counting and IC₅₀ values determined as before.

In the above assays, compounds according to the invention, including the compounds of the Examples inhibit the protein kinase p56^{Ick}, p59^{fyn}, ZAP-70 or protein kinase C at concentrations at which they have little or no effect on the remaining kinases, including EGFr kinase and Csk kinase. Thus, for example the compounds of Example 96 and 103 have IC₅₀ values in the p56^{Ick} assay of 215nM and 40nM respectively. The compounds are also active against p59^{fyn} but have IC₅₀ values at least 10x greater against the remaining kinases described above. In another example the compounds of Example 64 and 66 have IC₅₀ values in the ZAP-70 assay of 124nM and 68nM respectively. The compounds are also active in the protein kinase C assay, but have IC₅₀ values at least 10x greater against the remaining kinases. In a further example, the compounds of Examples 48 and 81 have IC₅₀ values in the protein kinase C assay of 22nM and 92nM respectively, but have IC₅₀ values at least 10x greater against the remaining kinases.

## Claims

1. A compound of formula (1): 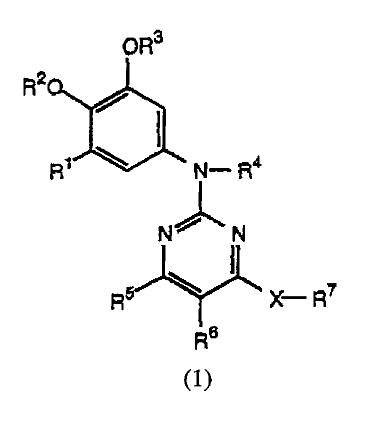 wherein
R¹ is -OCH₃, -OCH₂F, -OCH₂Cl, -OCHF₂, -OCHCl₂, -OCF₃ or -OCCl₃;
R² and R³, which may be the same or different, is each a straight or branched chain C₁₋₆ alkyl, C₂₋₆ alkenyl or C₂₋₆ alkynyl group, optionally substituted with one, two or three groups selected from halogen atom, hydroxyl, C₁₋₆ alkoxy, thiol, C₁₋₆ alkylthio, amino, C₁₋₆ alkylamino, or C₁₋₆ dialkylamino;
R⁴ is a hydrogen atom or a straight or branched chain C₁₋₆ alkyl group;
R⁵ is a hydrogen atom or a straight or branched chain alkyl, alkenyl or alkynyl group, optionally substituted with one, two or three groups selected from halogen atom, hydroxyl, C₁₋₆ alkoxy, thiol, C₁₋₆ alkylthio, amino, C₁₋₆ alkylamino, or C₁₋₆ dialkylamino;
R⁶ is a hydrogen or halogen atom or an amino (-NH₂), -NHR⁹ or -NR⁹R¹⁰, nitro, carboxyl (-CO₂H), -CO₂Alk1 or a group -X¹-R^{6a};
R^{6a} is a straight or branched chain C₁₋₆ alkyl, C₂₋₆ alkenyl or C₂₋₆ alkynyl group, optionally substituted with one, two or three groups selected from halogen atom, hydroxyl, C₁₋₆ alkoxy, thiol, C₁₋₆ alkylthio, amino, C₁₋₆ alkylamino, or C₁₋₆ dialkylamino;
R⁷ is an aliphatic, cycloaliphatic, heteroaliphatic, heterocycloaliphatic, aromatic or heteroaromatic group;
R⁹ and R¹⁰ are the same or different and each is a group -R⁸ or -COR⁸;
R⁸ is a straight or branched C₁₋₆ alkyl, C₂₋₆ alkenyl, or C₂₋₆ alkynyl group, optionally substituted with a phenyl group and/or one, two or three groups selected from halogen atom, hydroxyl, C₁₋₆ alkoxy, thiol, C₁₋₆ alkylthio, amino, C₁₋₆ alkylamino, or C₁₋₆ dialkylamino;
Alk¹ is a straight or branched C₁₋₈ alkyl group; C₆₋₁₂aryllC₁₋₈alkyl group; C₆₋₁₂ aryl; C ₆₋₁₂ aryloxyC₁₋₈alkyl; C₁₋₈alkanoyloxyC₁₋₈alkyl group; or C₆₋₁₂aroyloxyC₁₋₈alkyl; each of which may be optionally substituted with a R¹³ group;
Alk is a straight or branched C₁₋₆ alkylene, C₂₋₆ alkenylene or C₂₋₆ alkynylene chain, optionally interrupted by one, two or three -O- or -S- atoms or S-(O)-, -S(O)₂- or -N(R¹¹)-groups;
R¹¹ is a hydrogen atom or C₁₋₆ alkyl, where each R¹¹ group is the same or different;
R¹³ is a halogen atom, or an amino (-NH₂), -NHR¹⁴ [where R¹⁴ is an -AlkCR¹³)ₘ, heterocycloalkyl, -Alk-heterocycloalkyl, aryl or heteroaryl group], -N(R¹⁴)₂ [where each R¹⁴ group is the same or different], nitro, cyano, hydroxyl (-OH), -OR¹⁴, formyl, carboxyl (-CO₂H), -CO₂Alk¹, thiol (-SH), -SR¹⁴, -COR¹⁴, -CSR¹⁴, -SO₃H, -SO₂R¹⁴, -SO₂NH₂, -SO₂NHR¹⁴, SO₂N[R¹⁴]₂, -CONH₂, -CSNH₂, -CONHR¹⁴, -CSNHR¹⁴, -CON[R¹⁴]₂, -CSN[R¹⁴]₂, -NHSO₂H, -NHSO₂R¹⁴, -N[SO₂R¹⁴]₂, -NHSO₂NH₂, -NHSO₂NHR¹⁴, -NHSO₂N[R¹⁴]₂, -NHCOR¹⁴, -NHCONH₂, -NHCONHR¹⁴, -NHCON[R¹⁴]₂, -NHCSR¹⁴, -NHCSNH₂, -NHCSNHR¹⁴, -NHCSN[R¹⁴]₂, -NHC(O)OR¹⁴, or cycloalkyl, aryl or heteroaryl group;
R¹² is selected from R¹³, -Alk(R¹³)m, heterocycloalkyl or -Alk-heterocycloalkyl group, provided that when R¹² is a heterocycloalkyl group, X is either a linker atom or group, or X is a bond and R⁷ is other than a pyridyl group;
-X-, -X¹- and -X²- are independently selected from a direct bond, -O-, -S-, -C(O)-, -C(S)-, -S(O)-, -S(O)₂-, -N(R¹¹)-, -CON(R¹¹)-, -OC(O)N(R¹¹)-, -CSN(R¹¹)-, -N(R¹¹)CO-, -N(R¹¹)C(O)O-, -N(R¹¹)CS-, -SON(R¹¹), -SO₂N(R¹¹), -N(R¹¹)SO₂-, -N(R¹¹)CON(R¹¹)-, -N(R¹¹)CSN(R¹¹)-, -N(R¹¹)SON(R¹¹)- or -N(R¹¹)SO₂N(R¹¹) group;
heterocycloalkyl is a heteroC₃₋₆cycloalkyl group containing one or two oxygen, sulphur or nitrogen atoms, optionally substituted with one or two C₁₋₆ alkyl, hydroxyl (-OH), hydroxyC₁₋₆alkyl or C₁₋₆ alkoxy;
cycloalkyl is a C₅₋₇ cycloalkyl group optionally substituted with one or two C₁₋₆ alkyl, hydroxyl (-OH), hydroxyC₁₋₆alkyl or C₁₋₆ alkoxy;
aryl or aromatic group is a monocyclic or bicyclic fused ring C₆₋₁₂ aromatic group optionally substituted with one, two or three R¹² groups;
heteroaryl or heteroaromatic group is a C₁₋₉ heteroaromatic group containing one, two, three or four heteroatoms selected from oxygen, sulphur or nitrogen, optionally substituted with one, two or three R¹² groups;
aliphatic group is a C₁₋₆ alkyl, C₂₋₆ alkenyl, or C₂₋₆ alkynyl group, optionally substituted with one, two or three R¹² groups;
cycloaliphatic group is a C₃₋₁₀ cycloalkyl, C₃₋₁₀ cycloalkenyl or C₃₋₁₀ cycloalkynyl group, optionally substituted with one, two or three R¹² groups;
heteroaliphatic group is a C₁₋₆ alkyl, C₂₋₆ alkenyl, or C₂₋₆ alkynyl group, additionally containing one, two, three or four -X²- groups, and optionally substituted with one, two or three R¹² groups;
heterocycloaliphatic group is a C₃₋₁₀ cycloalkyl, C₃₋₁₀ cycloalkenyl or C₃₋₁₀ cycloalkynyl group, additionally containing one, two, three or four -X²- groups, and optionally substituted with one, two or three R¹² groups;
m is zero or an integer 1, 2 or 3;
provided that when R¹ is -OCH₃, R², R³, R⁵ and R⁷ are all -CH₃, and R⁴ and R⁶ are both H, then -X- is not a direct bond;
and the salts, solvates, hydrates and N-oxides thereof.

2. A compound according to Claim 1 wherein R⁴ is a hydrogen atom.

3. A compound according to Claim 1 or Claim 2 wherein R⁵ and R⁶ is each a hydrogen atom.

4. A compound according to any one of the preceding Claims wherein R² and R³ is each a methyl or ethyl group optionally substituted with one, two or three groups selected from halogen atom, hydroxyl, C₁₋₆ alkoxy, thiol, C₁₋₆ alkylthio, amino, C₁₋₆ alkylamino, or C₁₋₆ dialkylamino.

5. A compound according to Claim 4 wherein R² and R³ is each a methyl group.

6. A compound according to any one of the preceding Claims wherein X is a direct bond, a sulphur atom or a -NH- group.

7. A compound according to any one of Claims 1 to 6 wherein R⁷ is an aromatic or heteroaromatic group as defined in Claim 1.

8. A compound according to Claim 7 wherein R⁷ is a phenyl, 2-pyridyl, 3-pyridyl or 4-pyridyl group, optionally substituted with one, two or three R¹² groups.

9. A compound according to Claim 1 which is
N,N'-Bis(3,4,5-Trimethoxyphenyl)-2,4-pyrimidinediamine;
4-(2-(2-Dimethylaminoethylamino)pyridin-5-yl)-N-(3,4,5-trimethoxyphenyl)-2-pyrimidineamine;
4-(2-(4-Hydroxybutylamino)pyridin-5-yl)-N-(3,4,5-trimethoxyphenyl)-2-pyrimidineamine;
4-(3-(3-Aminopropoxy)phenyl)-N-(3,4,5-trimethoxyphenyl)-2-pyrimidineamine;
N4-(3,4-Dimethoxyphenyl)-N2-(3,4,5-trimethoxyphenyl)-2,4-pyrimidinediamine;
4-(4-(2-Hydroxyethoxy)phenyl)-N-(3,4,5-trimethoxypheny])-2-pyrimidineamine;
4-(2-(3-(Morpholino)propylamino)pyridin-5-yl)-N-(3,4,5-trimethoxyphenyl)-2-pyrimidineamine;
4-(2-(2-(1-Methylpyrrolidin-2-yl)ethylamino)pyridin-5-yl)-N-(3,4,5-trimethoxyphenyl)-2-pyrimidineamine;
N4-(4-(Ethoxycarbonylmethyl)phenyl)-N2-(3,4,5-trimethoxyphenyl)-2,4-pyrimidinediamine;
N4-(4-Hydroxyphenyl)-N2-(3,4,5-trimethoxyphenyI)-2,4-pyrimidinediamine; and
N4-(4-(3-Aminopropoxy)phenyl)-N2-(3,4,5-trimethoxyphenyl)-2,4-pyrimidinediamine;
and the salts, solvates, hydrates and N-oxides thereof.

10. A pharmaceutical composition comprising a compound of formula 1 as defined in any one of the preceding Claims.

## Patentansprüche

1. Verbindung der Formel (1): 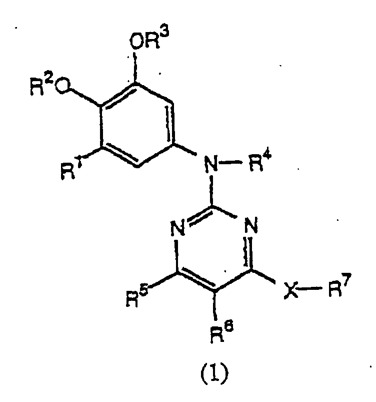 wobei R¹ -OCH₃, -OCH₂F, -OCH₂Cl, -OCHF₂, -OCHCl₂, -OCF₃ oder -OCCl₃ ist,
wobei R² und R³, die gleich oder unterschiedlich sein können, jeweils eine geradkettige oder verzweigtkettige C₁₋₆-Alkyl-, C₂₋₆-Alkenyl- oder C₂₋₆-Alkinylgruppe sind, die gegebenenfalls mit einer, zwei oder drei Gruppen substituiert sind, die aus einem Halogenatom, einer Hydroxyl-, einer C₁₋₆-Alkoxy-, einer Thiol-, einer C₁₋₆-Alkylthio-, einer Amino-, einer C₁₋₆-Alkylamino- oder einer C₁₋₆-Dialkylaminogruppe ausgewählt sind,
wobei R⁴ ein Wasserstoffatom oder eine geradkettige oder verzweigtkettige C₁₋₆-Alkylgruppe ist,
wobei R⁵ ein Wasserstoffatom oder eine geradkettige oder verzweigtkettige Alkyl-, Alkenyl- oder Alkinylgruppe ist, die gegebenenfalls mit einer, zwei oder drei Gruppen substituiert ist, die aus einem Halogenatom, einer Hydroxyl-, einer C₁₋₆-Alkoxy-, einer Thiol-, einer C₁₋₆-Alkylthio-, einer Amino-, einer C₁₋₆-Alkylamino- oder einer C₁₋₆-Dialkylaminogruppe ausgewählt ist,
wobei R⁶ ein Wasserstoff- oder ein Halogenatom oder eine Aminogruppe (-NH₂), -NHR⁹ oder -NR⁹R¹⁰, eine Nitro-, eine Carboxylgruppe (-CO₂H), -CO₂Alk¹ oder eine Gruppe -X¹-R^{6a} ist,
wobei R^{6a} eine geradkettige oder verzweigtkettige C₁₋₆-Alkyl-, C₂₋₆-Alkenyloder C₂₋₆-Alkinylgruppe ist, die gegebenenfalls mit einer, zwei oder drei Gruppen substituiert ist, die aus einem Halogenatom, einer Hydroxyl-, einer C₁₋₆-Alkoxy-, einer Thiol-, einer C₁₋₆-Alkylthio-, einer Amino-, einer C₁₋₆-Alkylamino- oder einer C₁₋₆-Dialkylaminogruppe substituiert ist,
wobei R⁷ eine aliphatische, eine cycloaliphatische, eine heteroaliphatische, eine heterocycloaliphatische, eine aromatische oder eine heteroaromatische Gruppe ist,
wobei R⁹ und R¹⁰ gleich oder unterschiedlich sind und jeweils eine Gruppe -R⁸ oder -COR⁸ sind,
wobei R⁸ eine geradkettige oder verzweigtkettige C₁₋₆-Alkyl-, C₂₋₆-Alkenyloder C₂₋₆-Alkinylgruppe ist, die gegebenenfalls mit einer Phenylgruppe und/oder einer, zwei oder drei Gruppen substituiert ist, die aus einem Halogenatom, einer Hydroxyl-, einer C₁₋₆-Alkoxy-, einer Thiol-, einer C₁₋₆-Alkylthio-, einer Amino-, einer C₁₋₆-Alkylamino- oder einer C₁₋₆-Dialkylaminogruppe ausgewählt ist,
wobei Alk¹ eine geradkettige oder verzweigtkettige C₁₋₈-Alkyl-, C₆₋₁₂-ArylC₁₋₈-Alkyl-, C₆₋₁₂-Aryl-, C₆₋₁₂-AryloxyC₁₋₈-Alkyl-, C₁₋₈-AlkanoyloxyC₁₋₈-Alkyl- oder C₆₋₁₂-AryloxyC₁₋₈Alkylgruppe ist, wobei jede davon gegebenenfalls mit einer Gruppe R¹³ substituiert sein kann,
wobei Alk eine geradkettige oder verzweigtkettige C₁₋₆-Alkylen-, C₂₋₆-Alkenylen- oder C₂₋₆-Alkinylenkette ist, die gegebenenfalls durch eine, zwei oder drei -O- oder -S-Atome oder -S(O)-, -S(O)₂- oder -N(R¹¹)-Gruppen unterbrochen ist,
wobei R¹¹ ein Wasserstoffatom oder eine C₁₋₆-Alkylgruppe ist, wobei jede Gruppe R¹¹ gleich oder unterschiedlich ist,
wobei R¹³ ein Halogenatom oder eine Aminogruppe (-NH₂), -NHR¹⁴ [wobei R¹⁴ -Alk(R¹³)ₘ, eine Heterocycloalkyl-, eine -Alk-Heterocycloalkyl-, eine Aryl- oder eine Heteroarylgruppe ist], -N(R¹⁴)₂ [wobei jede Gruppe R¹⁴ gleich oder unterschiedlich ist], eine Nitro-, eine Cyano-, eine Hydroxylgruppe (-OH), -OR¹⁴, eine Formyl-, eine Carboxylgruppe (-CO₂H), -CO₂Alk¹, eine Thiolgruppe (-SH), -SR¹⁴, -COR¹⁴, -CSR¹⁴, -SO₃H. -SO₂R¹⁴, -SO₂NH₂, -SO₂NHR¹⁴, -SO₂N[R¹⁴]₂, -CONH₂, -CSNH₂, -CONHR¹⁴, -CSNHR¹⁴, -CON[R¹⁴]₂, -CSN[R¹⁴]₂, -NHSO₂H, -NHSO₂R¹⁴, -N[SO₂R¹⁴]₂, -NHSO₂NH₂, -NHSO₂NHR¹⁴, -NHSO₂N[R¹⁴]₂, -NHCOR¹⁴, -NHCONH₂, -NHCONHR¹⁴, -NHCON[R¹⁴]₂, -NHCSR¹⁴, -NHCSNH₂, -NHCSNHR¹⁴, -NHCSN[R¹⁴]₂, -NHC(O)OR¹⁴ oder eine Cycloalkyl-, eine Aryl- oder eine Heteroarylgruppe ist.
wobei R¹² aus R¹³, -Alk(R¹³)ₘ, einer Heterocycloalkyl- oder einer -Alk-Heterocycloalkylgruppe ausgewählt ist, mit der Maßgabe, daß, wenn R¹² eine Heterocycloalkylgruppe ist, X entweder ein Verknüpfungsatom oder eine Verknüpfungsgruppe ist oder X eine Bindung ist und R⁷ von einer Pyridylgruppe verschieden ist,
wobei -X-, -X¹- und -X²- unabhängig voneinander aus einer direkten Bindung, einer -O-, einer -S-, einer -C(O)-, einer -C(S)-, einer -S(O)-, einer -S(O)₂-, einer -N(R¹¹)-, einer -CON(R¹¹)-, einer -OC(O)N(R¹¹)-, einer -CSN(R¹¹)-, einer -N(R¹¹)CO-, einer -N(R¹¹)C(O)O-, einer -N(R¹¹)CS-, einer -SON(R¹¹)-, einer -SO₂N(R¹¹)-, einer -N(R¹¹)SO₂-, einer -N(R¹¹)CON(R¹¹)-, einer -N(R¹¹)CSN(R¹¹)-, einer -N(R¹¹)SON(R¹¹)- oder einer -N(R¹¹)SO₂N(R¹¹)-Gruppe ausgewählt sind,
wobei Heterocycloalkyl eine HeteroatomC₃₋₆-Cycloalkylgruppe ist, die ein oder zwei Sauerstoff-, Schwefel- oder Stickstoffatome enthält, die gegebenenfalls mit einer oder zwei C₁₋₆-Alkyl-, Hydroxyl- (-OH), HydroxyC₁₋₆-Alkyl- oder C₁₋₆-Alkoxygruppen substituiert ist,
wobei Cycloalkyl eine C₅₋₇-Cycloalkylgruppe ist, die gegebenenfalls mit einer oder zwei C₁₋₆-Alkyl-, Hydroxyl- (-OH), HydroxyC₁₋₆-Alkyl- oder C₁₋₆-Alkoxygruppen substituiert ist,
wobei Aryl oder eine aromatische Gruppe eine monocyclische oder bicyclische aromatische C₆₋₁₂-Gruppe mit einem kondensierten Ring ist, die gegebenenfalls mit einer, zwei oder drei Gruppen R¹² substituiert ist,
wobei Heteroaryl oder eine heteroaromatische Gruppe eine heteroaromatische C₁₋₉-Gruppe ist, die ein, zwei, drei oder vier Heteroatome enthält, die aus Sauerstoff-, Schwefel- oder Stickstoffatomen ausgewählt sind, die gegebenenfalls mit einer, zwei oder drei Gruppen R¹² substituiert sind,
wobei eine aliphatische Gruppe eine C₁₋₆-Alkyl-, eine C₂₋₆-Alkenyl- oder eine C₂₋₆-Alkinylgruppe ist, die gegebenenfalls mit einer, zwei oder drei Gruppen R¹² substituiert ist,
wobei eine cycloaliphatische Gruppe eine C₃₋₁₀-Cycloalkyl-, eine C₃₋₁₀-Cycloalkenyl- oder eine C₃₋₁₀-Cycloalkinylgruppe ist, die gegebenenfalls mit einer, zwei oder drei Gruppen R¹² substituiert ist,
wobei eine heteroaliphatische Gruppe eine C₁₋₆-Alkyl-, eine C₂₋₆-Alkenyloder eine C₂₋₆-Alkinylgruppe ist, die zusätzlich eine, zwei, drei oder vier Gruppen -X²- enthält und gegebenenfalls mit einer, zwei oder drei Gruppen R¹² substituiert ist,
wobei eine heterocycloaliphatische Gruppe eine C₃₋₁₀-Cycloalkyl-, eine C₃₋₁₀-Cycloalkenyl- oder eine C₃₋₁₀-Cycloalkinylgruppe ist, die zusätzlich eine, zwei, drei oder vier Gruppen -X²- enthält und gegebenenfalls mit einer, zwei oder drei Gruppen R¹² substituiert ist,
wobei m Null oder eine ganze Zahl von 1, 2 oder 3 ist,
mit der Maßgabe, daß, wenn R¹ -OCH₃ ist, R², R³, R⁵ und R⁷ alle -CH₃ sind, und wenn R⁴ und R⁶ beide H sind, dann -X- keine direkte Bindung ist,
und Salze, Solvate, Hydrate und N-Oxide davon.

2. Verbindung nach Anspruch 1, wobei R⁴ ein Wasserstoffatom ist.

3. Verbindung nach Anspruch 1 oder 2, wobei R⁵ und R⁶ jeweils ein Wasserstoffatom sind.

4. Verbindung nach einem der vorhergehenden Ansprüche, wobei R² und R³ jeweils eine Methyl- oder eine Ethylgruppe sind, die gegebenenfalls mit einer, zwei oder drei Gruppen substituiert sind, die aus einem Halogenatom, einer Hydroxyl-, einer C₁₋₆-Alkoxy-, einer Thiol-, einer C₁₋₆-Alkylthio-, einer Amino-, einer C₁₋₆-Alkylamino- oder einer C₁₋₆-Dialkylaminogruppe ausgewählt sind.

5. Verbindung nach Anspruch 4, wobei R² und R³ jeweils eine Methylgruppe sind.

6. Verbindung nach einem der vorhergehenden Ansprüche, wobei X eine direkte Bindung, ein Schwefelatom oder eine -NH-Gruppe ist.

7. Verbindung nach einem der Ansprüche 1 bis 6, wobei R⁷ eine wie in Anspruch 1 definierte aromatische oder heteroaromatische Gruppe ist.

8. Verbindung nach Anspruch 7, wobei R⁷ eine Phenyl-, eine 2-Pyridyl-, eine 3-Pyridyl- oder eine 4-Pyridylgruppe ist, die gegebenenfalls mit einer, zwei oder drei Gruppen R¹² substituiert ist.

9. Verbindung nach Anspruch 1, die
N,N'-Bis(3,4,5-trimethoxyphenyl)-2,4-pyrimidindiamin,
4-(2-(2-Dimethylaminoethylamino)pyridin-5-yl)-N-(3,4,5-trimethoxyphenyl)-2-pyrimidinamin,
4-(2-(4-Hydroxybutylamino)pyridin-5-yl)-N-(3,4,5-trimethoxyphenyl)-2-pyrimidinamin,
4-(3-(3-Aminopropoxy)phenyl)-N-(3,4,5-trimethoxyphenyl)-2-pyrimidinamin,
N4-(3,4-Dimethoxyphenyl)-N2-(3,4,5-trimethoxyphenyl)-2,4-pyrimidindiamin,
4-(4-(2-Hydroxyethoxy)phenyl)-N-(3,4,5-trimethoxyphenyl)-2-pyrimidinamin,
4-(2-(3-(Morpholino)propylamino)pyridin-5-yl)-N-(3,4,5-trimethoxyphenyl)-2-pyrimidinamin,
4-(2-(2-(1-Methylpyrrolidin-2-yl)ethylamino)pyridin-5-yl)-N-(3,4,5-trimethoxyphenyl)-2-pyrimidinamin,
N4-(4-(Ethoxycarbonylmethyl)phenyl)-N2-(3,4,5-trimethoxyphenyl)-2,4-pyrimidindiamin,
N4-(4-Hydroxyphenyl)-N2-(3,4,5-trimethoxyphenyl)-2,4-pyrimidindiamin
und
N4-(4-(3-Aminopropoxy)phenyl)-N2-(3,4,5-trimethoxyphenyl)-2,4-pyrimidindiamin ist
und Salze, Solvate, Hydrate und N-Oxide davon.

10. Pharmazeutische Zusammensetzung, umfassend eine wie in einem der vorhergehenden Ansprüche definierte Verbindung der Formel 1.

## Revendications

1. Composé de formule (1) : 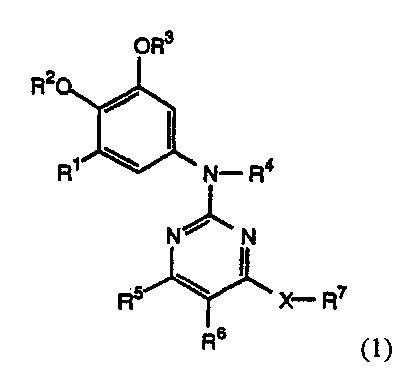 dans laquelle :
R¹ est -OCH₃, -OCH₂F, -OCH₂Cl, -OCHF₂, -OCHCl₂, -OCF₃ ou -OCCl₃;
R² et R³, qui peuvent être identique ou différents, sont chacun un groupe alkyle en C₁₋₆, alcényle en C₂₋₆ ou alcynyle en C₂₋₆ à chaîne droite ou ramifiée, éventuellement substitué par un, deux ou trois groupes choisis parmi un atome d'halogène ou un groupe hydroxyle, alcoxy en C₁₋₆, thiol, alkylthio en C₁₋₆, amino, alkylamino en C₁₋₆ ou dialkylamino en C₁₋₆;
R⁴ est un atome d'hydrogène ou un groupe alkyle en C₁₋₆ à chaîne droite ou ramifiée;
R⁵ est un atome d'hydrogène ou un groupe alkyle, alcényle ou alcynyle à chaîne droite ou ramifiée, éventuellement substitué par un, deux ou trois groupes choisis parmi un atome d'halogène ou un groupe hydroxyle, alcoxy en C₁₋₆, thiol, alkylthio en C₁₋₆, amino, alkylamino en C₁₋₆ ou dialkylamino en C₁₋₆;
R⁶ est un atome d'hydrogène ou d'halogène, ou un groupe amino (-NH₂), -NHR⁹ ou -NR⁹R¹⁰, nitro, carboxyle (-CO₂H), -CO₂Alkl ou -X¹-R^{6a};
R^{6a} est un groupe alkyle en C₁₋₆, alcényle en C₂₋₆ ou alcynyle en C₂₋₆ à chaîne droite ou ramifiée, éventuellement substitué par un, deux ou trois groupes choisis parmi un atome d'halogène ou un groupe hydroxyle, alcoxy en C₁₋₆, thiol, alkylthio en C₁₋₆, amino, alkylamino en C₁₋₆ ou dialkylamino en C₁₋₆;
R⁷ est un groupe aliphatique, cycloaliphatique, hétéroaliphatique, hétérocycloaliphatique, aromatique ou hétéroaromatique;
R⁹ et R¹⁰ sont identiques ou différents et chacun d'entre eux est un groupe -R⁸ ou -COR⁸;
R⁸ est un groupe alkyle en C₁₋₆, alcényle en C₂₋₆ ou alcynyle en C₂₋₆ à chaîne droite ou ramifiée, éventuellement substitué par un groupe phényle et/ou par un, deux ou trois groupes choisis parmi un atome d'halogène ou un groupe hydroxyle, alcoxy en C₁₋₆, thiol, alkylthio en C₁₋₆, amino, alkylamino en C₁₋₆ ou dialkylamino en C₁₋₆;
Alk¹ est un groupe alkyle en C₁₋₈ à chaîne droite ou ramifiée, un groupe aryl(C₆₋₁₂)alkyle(C₁₋₈); aryle en C₆₋₁₂; aryloxy(C₆₋₁₂)alkyle(C₁₋₈); alcanoyloxy(C₁₋₈)alkyle(C₁₋₈); ou aroyloxy(C₆₋₁₂)alkyle(C₁₋₈); dont chacun peut être éventuellement substitué par un groupe R¹³;
Alk est une chaîne alkylène en C₁₋₆, alcénylène en C₂₋₆ ou alcynylène en C₂₋₆, droite ou ramifiée, éventuellement interrompue par un, deux ou trois atomes -O- ou -S- ou groupes S-(O)-, -S(O)₂- ou -N(R¹¹)-;
R" est un atome d'hydrogène ou un groupe alkyle en C₁₋₆, où chaque groupe R¹¹ est identique ou différent;
R¹³ est un atome d'halogène ou un groupe amino (-NH₂), -NHR¹⁴ [où R¹⁴ est un groupe -Alk(R¹³)ₘ, hétérocycloalkyle, -Alk-hétérocycloalkyle, aryle ou hétéroaryle], -N(R¹⁴)₂ [où chaque groupe R¹⁴ est identique ou différent], nitro, cyano, hydroxyle (-OH), -OR¹⁴, formyle, carboxyle (-CO₂H), -CO₂Alk¹, thiol (-SH), -SR¹⁴, -COR¹⁴, CSR¹⁴, -SO₃H, -SO₂R¹⁴, -SO₂NH₂-, -SO₂NHR¹⁴, SO₂N[R¹⁴]₂, -CONH₂, -CSNH₂, -CONHR¹⁴, -CSNHR¹⁴, -CON[R¹⁴]₂, -CSN[R¹⁴]₂, -NHSO₂H, -NHSO₂R¹⁴, -N[SO₂R¹⁴]₂, -NHSO₂NH₂, -NHSO₂NHR¹⁴, -NHSO₂N[R¹⁴]₂, -NHCOR¹⁴, -NHCONH₂, -NHCONHR¹⁴, -NHCON[R¹⁴]₂, -NHCSR¹⁴-, NHCSNH₂, -NHCSNHR¹⁴, -NHCSN[R¹⁴]₂, -NHC(O)OR¹⁴ ou cycloalkyle, aryle ou hétéroaryle;
R¹² est choisi parmi les groupes R¹³, -Alk(R¹³)m, hétérocycloalkyle ou -Alk-hétérocycloalkyle, à condition que, lorsque R¹² est un groupe hétérocycloalkyle, X soit un atome ou un groupe de liaison, ou que X soit une liaison et R⁷ un groupe autre qu'un groupe pyridyle;
-X-, -X¹ et X² sont choisis indépendamment parmi une liaison directe ou un groupe -O-, -S-, -C(O)-, -C(S)-, -S(O)-, -S(O)₂-, -N(R¹¹), -CON(R¹¹)-, -OC(O)N(R¹¹)-, -CSN(R¹¹)-, -N(R¹¹)CO-, -N(R¹¹)C(O)O-, -N(R¹¹)CS-, -SON(R¹¹), -SO₂N(R¹¹), -N(R¹¹)SO₂-, -N(R¹¹)CON(R¹¹)-, -N(R¹¹)CSN(R¹¹)-, -N(R¹¹)SON(R¹¹)- ou -N(R¹¹)SO₂N(R¹¹);
le groupe hétérocycloalkyle est un groupe hétérocycloalkyle(C₃₋₆) contenant un ou deux atomes d'oxygène, de soufre ou d'azote, éventuellement substitué par un ou deux groupes alkyle en C₁₋₆, hydroxyle (-OH), hydroxyalkyle(C₁₋₆) ou alcoxy en C₁₋₆;
le groupe cycloalkyle est un groupe cycloalkyle en C₅₋₇ éventuellement substitué par un ou deux groupes alkyle en C₁₋₆, hydroxyle (-OH), hydroxyalkyle(C₁₋₆) ou alcoxy en C₁₋₆;
le groupe aryle ou aromatique est un groupe aromatique monocyclique ou bicyclique en C₆₋₁₂ à cycle condensé, éventuellement substitué par un, deux ou trois groupes R¹²;
le groupe hétéroaryle ou hétéroaromatique est un groupe hétéroaromatique en C₁₋₉ contenant un, deux, trois ou quatre hétéroatomes choisis parmi l'oxygène, le soufre ou l'azote, éventuellement substitué par un, deux ou trois groupes R¹²;
le groupe aliphatique est un groupe alkyle en C₁₋₆, alcényle en C₂₋₆ ou alcynyle en C₂₋₆, éventuellement substitué par un, deux ou trois groupes R¹²;
le groupe cycloaliphatique est un groupe cycloalkyle en C₃₋₁₀, cycloalcényle en C₃₋₁₀, cycloalcynyle en C₃₋₁₀, éventuellement substitué par un, deux ou trois groupes R¹²;
le groupe hétéroaliphatique est un groupe alkyle en C₁₋₆, alcényle en C₂₋₆ ou alcynyle en C₂₋₆, contenant en outre un, deux ou trois groupes -X²-, et éventuellement substitué par un, deux ou trois groupes R¹²;
le groupe hétérocycloaliphatique est un groupe cycloalkyle en C₃₋₁₀, cycloalcényle en C₃₋₁₀ ou cycloalcynyle en C₃₋₁₀, contenant en outre un, deux, trois ou quatre groupes -X²-, et éventuellement substitué par un, deux ou trois groupes R¹²;
m est zéro ou un nombre entier 1, 2 ou 3;
à condition que, lorsque R¹ est -OCH₃, que R², R³, R⁵ et R⁷ sont tous -CH₃ et que R⁴ et R⁶ sont tous deux H, -X- ne soit pas une liaison directe; et
ses sels, solvates, hydrates et N-oxydes.

2. Composé selon la revendication 1, dans lequel R⁴ est un atome d'hydrogène.

3. Composé selon la revendication 1 ou 2, dans lequel R⁵ et R⁶ sont chacun un atome d'hydrogène.

4. Composé selon l'une quelconque des revendications précédentes, dans lequel R² et R³ sont chacun un groupe méthyle ou éthyle éventuellement substitué par un, deux ou trois groupes choisis parmi un atome d'halogène ou un groupe hydroxyle, alcoxy en C₁₋₆, thiol, alkythio en C₁₋₆, amino, alkylamino en C₁₋₆ ou dialkylamino en C₁₋₆.

5. Composé selon la revendication 4, dans lequel R² et R³ sont chacun un groupe méthyle.

6. Composé selon l'une quelconque des revendications précédentes, dans lequel X est une liaison directe, un atome de soufre ou un groupe -NH-.

7. Composé selon l'une quelconque des revendications 1 à 6, dans lequel R⁷ est un groupe aromatique ou hétéroaromatique tel que défini dans la revendication 1.

8. Composé selon la revendication 7, dans lequel R⁷ est un groupe phényle, 2-pyridyle, 3-pyridyle ou 4-pyridyle, éventuellement substitué par un, deux ou trois groupes R¹².

9. Composé selon la revendication 1 qui est l'un des suivants :
N,N'-bis(3,4,5-triméthoxyphényl)-2,4-pyrimidinediamine;
4-(2-(2-diméthylaminoéthylamino)pyridin-5-yl)-N-(3,4,5-triméthoxyphényl)-2-pyrimidineamine;
4-(2-(4-hydroxybutylamino)pyridin-5-yl)-N-(3,4,5-triméthoxyphényl)-2-pyrimidinemaine;
4-(3-(3-aminopropoxy)phényl)-N-(3,4,5-triméthoxyphényl)-2-pyrimidineamine;
N4-(3,4-diméthoxyphényl)-N2-(3,4,5-triméthoxyphényl)-2,4-pyrimidinediamine;
4-(4-(2-hydroxyéthoxy)phényl)-N-(3,4,5-triméthoxyphényl))-2-pyrimidinediamine;
4-(2-(3-morpholino)propylamino)pyridin-5-yl)-N-(3,4,5-triméthoxyphényl)-2-pyrimidineamine;
4-(2-(2-(1-méthylpyrrolidin-2-yl)éthylamino)pyridin-5-yl)-N-(3,4,5-triméthoxyphényl)-2-pyrimidineamine;
N4-(4-(éthoxycarbonylméthyl)phényl)-N2-(3,4,5-triméthoxyphényl)-2,4-pyrimidinediamine;
N4-(4-hydroxyphényl)-N2-(3,4,5-triméthoxyphényl)-2,4-pyrimidinediamine; et
N4-(4-(3-aminopropoxy)phényl)-N2-(3,4,5-triméthoxyphényl)-2,4-pyrimidinediamine; et
ses sels, solvates, hydrates et N-oxydes.

10. Composition pharmaceutique comprenant un composé de formule 1 tel que défini dans l'une quelconque des revendications précédentes.
